# EUROPEAN PATENT APPLICATION

(11) **EP 4 768 486 A2**
(43) Date of publication of application: **01.07.2026**
(21) Application number: 26174129.2
(22) Date of filing: 15.04.2024
(51) Int. Cl.: C07D 491/048

(54) **TRIARYLAMINE DERIVATIVES FOR USE IN ORGANIC ELECTROLUMINESCENT DEVICES**

(30) Priority: 26.09.2023 CN 202311253001
(62) Divisional of application: 24170165.5
(71) Applicant: Changchun Hyperions Technology Co., Ltd., Changchun, Jilin 130000 (CN)
(72) Inventor: DONG, Xiuqin, Changchun City, Jilin Province, 130000 (CN); ZHOU, Wenting, Changchun City, Jilin Province, 130000 (CN); LIU, Xiqing, Changchun City, Jilin Province, 130000 (CN)
(74) Representative: Ricker, Mathias

(57) **Abstract**

Provided are a triarylamine compound and an organic electroluminescent device thereof, which specifically relate to the field of organic electroluminescent materials. The triarylamine compound has high hole mobility, good thermal stability and great film formability, and when applied to the hole transport layer of an organic electroluminescent device, can reduce the energy potential barrier in a hole injection process, improve hole injection efficiency so that the hole transport rate is balanced with electron transport, increase the recombination probability of excitons in a light-emitting layer, and achieve the maximum recombination of carriers, thereby improving the luminescence efficiency of the device and extending the lifetime of the device. Therefore, the compound has great application and commercial values in the application of OLED devices and possesses a good industrialization prospect.

## Description

### TECHNICAL FIELD

The present disclosure relates to the field of organic electroluminescent materials and specifically, to a triarylamine compound and an organic electroluminescent device thereof.

### BACKGROUND

The reason why the organic electroluminescent device, also known as organic light-emitting diode (OLED), has become the most developed new display technology at present is that the OLED has properties of being full-solid-state, active light emission, high contrast, low power consumption, being free of viewing angle limitation, fast response speed, wide operating range, being easy to achieve flexible display and 3D display, and the like, and thus the OLED has been widely used in various fields such as display and lighting, has attracted extensive attention at home and abroad, and has gradually become the focus of research in related industries.

In the organic electroluminescent device, under the driving of an applied voltage, electrons and holes are injected into an organic layer from a cathode and an anode, respectively, migrated in the organic layer and recombined to form excitons, and the excitons achieve molecular luminescence by radiative decay. The basic structure of the OLED can be a single-layer structure, a double-layer structure, a three-layer structure or a multi-layer structure. However, with the extensive application of the OLED, the OLED of a single-layer structure, a double-layer structure, and a three-layer structure cannot meet the requirements of the market. Therefore, to better improve the efficiency of the device, the multi-layer structure is mostly adopted in the OLED, and such a structure may specifically include functional layers such as a hole injection layer, a hole transport layer, an electron blocking layer, a light-emitting layer, a hole blocking layer, an electron transport layer, an electron injection layer and a capping layer.

The main functions of a hole transport material are to improve the balance between injection and transport of holes in a device to effectively block electrons in the light-emitting layer, achieve the maximum recombination of carriers, improve luminescence efficiency, and reduce the energy potential barrier in the hole injection process to improve hole injection efficiency, thereby improving the efficiency and lifetime of the device. However, most current hole transport materials have problems such as low hole mobility, ill film formability, and poor thermal stability. Since the hole transport material is indispensable among the OLED materials, to improve the luminescence efficiency and lifetime of the OLED device, the hole transport material should have high hole mobility, good thermal stability, and great film formability.

Although the research on the hole transport material is becoming increasingly mature, many problems still exist in the application of the hole transport material to the device, such as low luminescence efficiency and a short lifetime of the device. Therefore, to comprehensively improve the performance of the organic electroluminescent device, a hole transport material with excellent performance needs to be designed to improve the luminescence efficiency of the device and extend the lifetime of the device.

### SUMMARY

In terms of the preceding problems, the present disclosure provides a triarylamine compound and an organic electroluminescent device thereof. The compound, when applied to an organic electroluminescent device, can effectively improve the luminescence efficiency of the organic electroluminescent device and extend the lifetime of the organic electroluminescent device. Specifically, the schemes of the present disclosure are as follows:

The present disclosure provides a triarylamine compound. The triarylamine compound is represented by a structure shown in Formula 1: 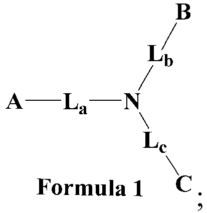 wherein A is selected from Formula 2, B is selected from Formula 3, and C is selected from Formula 3: 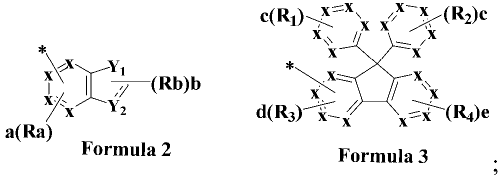
Y₁ is selected from O or S; Y₂ is selected from CH, O or S;
"--" is selected from none or a single bond; when Y₂ is selected from CH, "--" is selected from a single bond; when Y₂ is selected from O or S, "--" is selected from none;
Rₐ, R_{b}, R₁, R₂, R₃ and R₄ are independently selected from any one of hydrogen, deuterium, tritium, cyano, halogen, nitro, substituted or unsubstituted C1 to C15 alkyl, a substituted or unsubstituted C3 to C15 alicyclic group, substituted or unsubstituted silyl, substituted or unsubstituted C6 to C30 aryl, substituted or unsubstituted fused cyclyl formed by fusing a C3 to C15 alicyclic ring to a C6 to C30 aromatic ring, substituted or unsubstituted C2 to C30 heteroaryl, substituted or unsubstituted fused cyclyl formed by fusing a C3 to C15 alicyclic ring to a C2 to C30 heteroaromatic ring, and combinations thereof; or two adjacent R₁, two adjacent R₂, two adjacent R₃ or two adjacent R₄ can be linked to each other to form a substituted or unsubstituted ring;
x is identically or differently selected from N or CH; x at a linkage is selected from C;
a is selected from 0, 1, 2 or 3; b is selected from 0, 1 or 2; c is selected from 0, 1, 2, 3, 4 or 5; d is selected from 0, 1, 2 or 3; e is selected from 0, 1, 2, 3 or 4; when two or more Rₐ, R_{b}, R₁, R₂, R₃ and R₄ are present, the two or more Rₐ, R_{b}, R₁, R₂, R₃ and R₄ are identical to or different from each other;
Lₐ is selected from any one of a single bond, substituted or unsubstituted phenylene, substituted or unsubstituted biphenylylene, substituted or unsubstituted terphenylene, substituted or unsubstituted naphthylene, substituted or unsubstituted phenanthrylene, substituted or unsubstituted triphenylenylene, substituted or unsubstituted fluorenylene, substituted or unsubstituted dibenzofurylene, substituted or unsubstituted dibenzothienylene, substituted or unsubstituted carbazolylene, substituted or unsubstituted benzocyclopropylene, substituted or unsubstituted benzocyclobutylene, substituted or unsubstituted benzocyclopentylene, substituted or unsubstituted benzocyclohexylene, substituted or unsubstituted benzocycloheptylene, substituted or unsubstituted benzocyclobutenylene, substituted or unsubstituted benzocyclopentenylene, substituted or unsubstituted benzocyclohexynylene, substituted or unsubstituted benzocycloheptenylene, substituted or unsubstituted pyridylene, substituted or unsubstituted pyrimidinylene, substituted or unsubstituted pyridazinylene, substituted or unsubstituted pyrazinylene, substituted or unsubstituted quinolylene, substituted or unsubstituted isoquinolylene, substituted or unsubstituted quinazolinylene, substituted or unsubstituted quinoxalinylene, and combinations thereof;
L_{b} and L_{c} are independently selected from any one of a single bond, substituted or unsubstituted C6 to C30 arylene, substituted or unsubstituted C2 to C30 heteroarylene, substituted or unsubstituted fused cyclylene formed by fusing a C3 to C15 alicyclic ring to a C6 to C30 aromatic ring, substituted or unsubstituted fused cyclylene formed by fusing a C3 to C15 alicyclic ring to a C2 to C30 heteroaromatic ring, and combinations thereof.

The present disclosure further provides an organic electroluminescent device. The organic electroluminescent device includes an anode, a cathode and an organic layer, wherein the organic layer is disposed between the anode and the cathode or is disposed on an outer side of at least one of the anode and the cathode, and the organic layer includes any one or a combination of at least two of the triarylamine compound described in the present disclosure.

The beneficial effects are as follows:

The present disclosure provides a triarylamine compound and an organic electroluminescent device thereof. The triarylamine compound provided by the present disclosure has high hole mobility, good thermal stability and good film formability, and when applied to the hole transport layer of an organic electroluminescent device, can reduce the energy potential barrier in a hole injection process, improve hole injection efficiency so that the hole transport rate is balanced with electron transport, increase the recombination probability of excitons in a light-emitting layer, and achieve the maximum recombination of carriers, thereby improving the luminescence efficiency of the device and extending the lifetime of the device.

### DETAILED DESCRIPTION

The present disclosure will be described below clearly and completely in conjunction with schemes in the specific embodiments of the present disclosure. Apparently, the embodiments described herein are part, not all, of the embodiments of the present disclosure. Based on the embodiments of the present disclosure, all other embodiments obtained by those skilled in the art without creative work are within the scope of the present disclosure.

In the compounds of the present disclosure, any atom not specifically designated as a particular isotope includes any stable isotope of that atom and includes atoms at both its natural and non-natural isotopic abundance.

Examples of halogen in the present disclosure may include fluorine, chlorine, bromine and iodine.

In the specification, " *-" refers to a moiety linked to another substituent. " *-" may be linked to any optional position of a linked group/segment.

In the specification, when a substituent or a bond on which a linkage site is located runs through two or more rings, it indicates that the substituent or the bond may be linked to any one of the two or more rings and may be specifically linked to any one of the corresponding optional sites of the ring. For example, 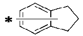 may represent 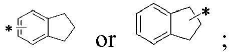 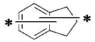 may represent 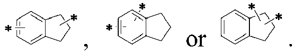 The rest can be represented in the same manner.

In the specification, when the position of a substituent or a linkage site on a ring is not fixed, it indicates that the substituent or the linkage site may be linked to any one of the corresponding optional sites of the ring. For example, 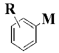 may represent 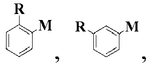 or 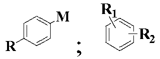 may represent 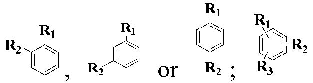 may represent 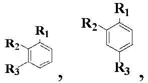 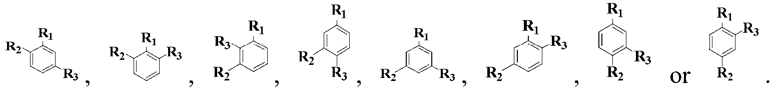 The rest can be represented in the same manner.

In the present disclosure, the expression that "two adjacent groups are linked to form a ring" means that adjacent groups are bound to each other and optionally aromatized to form a substituted or unsubstituted aromatic ring, heteroaromatic ring, aliphatic ring or aliphatic heterocyclic ring. The aliphatic ring and the aliphatic heterocyclic ring may be saturated rings or may be unsaturated rings. The linked ring may be a three-membered ring, a four-membered ring, a five-membered ring, a six-membered ring, a seven-membered ring, a spirocyclic ring or a fuse ring. Examples of the linked ring may include, but are not limited to, benzene, naphthalene, indene, cyclopentene, cyclopentane, cyclopentanobenzene, cyclohexene, cyclohexane, cyclohexanobenzene, pyridine, quinoline, isoquinoline, benzofuran, benzothiophene, dibenzofuran, dibenzothiophene, phenanthrene or pyrene.

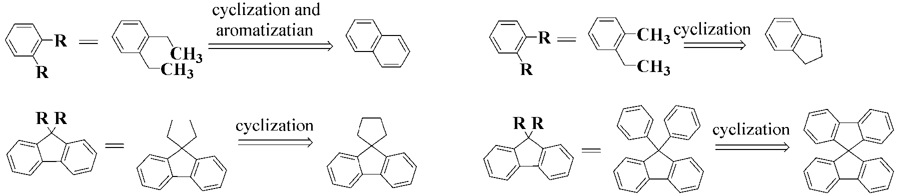

The alkyl in the present disclosure refers to the generic term of monovalent groups obtained by removing one hydrogen atom from an alkane molecule. Alkyl may be linear alkyl or branched alkyl and preferably has 1 to 15 carbon atoms, more preferably 1 to 12 carbon atoms, and particularly preferably 1 to 6 carbon atoms. Alkyl may be substituted or unsubstituted. The linear alkyl includes, but is not limited to, methyl, ethyl, *n*-propyl, *n*-butyl, *n*-pentyl, *n*-hexyl, *n*-heptyl, *n*-octyl, *n*-nonyl, *n-*decyl, undecyl, dodecyl, and the like; and the branched alkyl includes, but is not limited to, isopropyl, isobutyl, *s*-butyl, *tert*-butyl, isomeric groups of *n*-pentyl, isomeric groups of *n*-hexyl, isomeric groups of *n*-heptyl, isomeric groups of *n*-octyl, isomeric groups of *n*-nonyl, isomeric groups of *n*-decyl, and the like.

The alicyclic group as used in the present disclosure refers to a monovalent group obtained by removing one hydrogen atom from an alicyclic hydrocarbon molecule and may be cycloalkyl or cycloalkenyl. The alicyclic group preferably has 3 to 15 carbon atoms, more preferably 3 to 12 carbon atoms, and most preferably 3 to 7 carbon atoms. Examples of the alicyclic group may include, but are not limited to, cyclopropyl, cyclobutyl, cyclopentyl, cyclohexyl, cycloheptyl, adamantyl, norbornyl, cyclopropenyl, cyclobutenyl, cyclopentenyl, cyclohexenyl, cycloheptenyl, and the like.

The "substituted or unsubstituted silyl" as used in the present disclosure refers to -Si(Rₘ)₃ groups, wherein each Rₘ is identically or differently selected from any one of the following groups: hydrogen, deuterium, tritium, cyano, halogen, nitro, substituted or unsubstituted C1 to C15 alkyl, substituted or unsubstituted C1 to C15 alkenyl, substituted or unsubstituted C3 to C15 cycloalkyl, substituted or unsubstituted C6 to C30 aryl, substituted or unsubstituted C2 to C30 heteroaryl, substituted or unsubstituted fused cyclyl formed by fusing a C3 to C15 alicyclic ring to a C6 to C30 aromatic ring, substituted or unsubstituted fused cyclyl formed by fusing a C3 to C15 alicyclic ring to a C2 to C30 heteroaromatic ring, and combinations thereof. Preferably, each Rₘ is identically or differently selected from, but not limited to, one of the following groups: hydrogen, deuterium, tritium, cyano, halogen, nitro, substituted or unsubstituted methyl, substituted or unsubstituted ethyl, substituted or unsubstituted propyl, substituted or unsubstituted butyl, substituted or unsubstituted pentyl, substituted or unsubstituted hexyl, substituted or unsubstituted heptyl, substituted or unsubstituted octyl, substituted or unsubstituted cyclopropyl, substituted or unsubstituted cyclobutyl, substituted or unsubstituted cyclopentyl, substituted or unsubstituted cyclohexyl, substituted or unsubstituted cycloheptyl, substituted or unsubstituted adamantyl, substituted or unsubstituted norbornyl, substituted or unsubstituted phenyl, substituted or unsubstituted biphenyl, substituted or unsubstituted naphthyl, and the like.

Aryl as used in the present disclosure refers to the generic term of monovalent groups obtained by removing one hydrogen atom from the aromatic nucleus carbon of an aromatic compound molecule. Aryl may be monocyclic aryl, polycyclic aryl or fused ring aryl and preferably has 6 to 30 carbon atoms, more preferably 6 to 20 carbon atoms, and particularly preferably 6 to 14 carbon atoms. Aryl may be substituted or unsubstituted. The monocyclic aryl refers to aryl having only one aromatic ring in its molecule, for example, but not limited to, phenyl. The polycyclic aryl refers to aryl having two or more independent aromatic rings in its molecule, for example, but not limited to, biphenyl, terphenyl, and the like. The fused ring aryl refers to aryl having two or more aromatic rings in its molecule that are fused to each other through two adjacent carbon atoms, for example, but not limited to, naphthyl, anthryl, phenanthryl, pyrenyl, perylenyl, triphenylenyl, fluoranthenyl, and fluorenyl.

Heteroaryl as used in the present disclosure refers to the generic term of monovalent groups obtained by substituting one or more aromatic nucleus carbon atoms in aryl with heteroatoms, wherein the heteroatoms include, but are not limited to, oxygen, sulphur, nitrogen, silicon or phosphorus atoms. Heteroaryl preferably has 2 to 30 carbon atoms, more preferably 2 to 20 carbon atoms, and particularly preferably 3 to 15 carbon atoms. Heteroaryl may be substituted or unsubstituted. The linkage site of heteroaryl may be located on a ring carbon atom or on a ring heteroatom. Heteroaryl may be monocyclic heteroaryl, polycyclic heteroaryl or fused ring heteroaryl. The monocyclic heteroaryl includes, but is not limited to, pyridyl, pyrimidinyl, pyrazinyl, pyridazinyl, triazinyl, furyl, thienyl, pyrrolyl, oxazolyl, thiazolyl, and imidazolyl. The polycyclic heteroaryl includes, but is not limited to, bipyridyl, bipyrimidinyl, phenylpyridyl, phenylpyrimidinyl. The fused ring heteroaryl includes, but is not limited to, quinolyl, isoquinolyl, indolyl, benzothienyl, benzofuryl, benzoxazolyl, benzimidazolyl, benzothiazolyl, dibenzofuryl, benzodibenzofuryl, dibenzothienyl, benzodibenzothienyl, carbazolyl, phenylcarbazolyl, acridinyl, phenoxazinyl, phenothiazinyl, phenoxathiinyl, spirofluorenexanthyl, and spirofluorenethioxanthyl.

The fused cyclyl formed by fusing an alicyclic ring to an aromatic ring as used in the present disclosure refers to a monovalent group formed by fusing an aromatic ring to an alicyclic ring and removing one hydrogen atom, wherein the aromatic ring preferably has 6 to 30 carbon atoms, more preferably 6 to 20 carbon atoms, and most preferably 6 to 14 carbon atoms, and the alicyclic ring preferably has 3 to 15 carbon atoms, more preferably 3 to 12 carbon atoms, and most preferably 3 to 7 carbon atoms. Examples of the fused cyclyl formed by fusing an alicyclic ring to an aromatic ring include, but are not limited to, benzocyclopropyl, benzocyclobutyl, benzocyclopentyl, benzocyclohexyl, benzocycloheptyl, benzocyclobutenyl, benzcyclopentenyl, benzocyclohexenyl, benzocycloheptenyl, naphthocyclopropyl, naphthocyclobutyl, naphthocyclopentyl, naphthocyclohexyl, naphthocyclopentenyl, and naphthocyclohexenyl.

The fused cyclyl formed by fusing an alicyclic ring to a heteroaromatic ring as used in the present disclosure refers to the generic term of monovalent groups formed by fusing an alicyclic ring to a heteroaromatic ring and removing one hydrogen atom, wherein the alicyclic ring preferably has 3 to 15 carbon atoms, more preferably 3 to 12 carbon atoms, and particularly preferably 3 to 7 carbon atoms, and the heteroaromatic ring preferably has 2 to 30 carbon atoms, more preferably 2 to 20 carbon atoms, and particularly preferably 3 to 15 carbon atoms. Examples of fused cyclyl formed by fusing an alicyclic ring to a heteroaromatic ring include, but are not limited to, pyridocyclobutyl, pyridocyclopentyl, pyridocyclohexyl, pyridocycloheptyl, pyridocyclopentenyl, pyridocyclohexenyl, and the like.

Arylene as used in the present disclosure refers to the generic term of divalent groups formed by removing two hydrogen atoms from an aromatic nucleus carbon. The description stated above about aryl may be applied to arylene except that arylene is a divalent group.

Heteroarylene as used in the present disclosure refers to the generic term of divalent groups formed by removing two hydrogen atoms from the nuclear carbon of a heteroaromatic ring composed of carbon and heteroatoms. The description stated above about heteroaryl may be applied to heteroarylene except that heteroarylene is a divalent group.

The fused cyclylene formed by fusing an alicyclic ring to an aromatic ring as used in the present disclosure refers to the generic term of divalent groups formed by fusing an alicyclic ring to an aromatic ring and removing two hydrogen atoms. The description stated above about the fused cyclyl formed by fusing an alicyclic ring to an aromatic ring may be applied to the fused cyclylene formed by fusing an alicyclic ring to an aromatic ring except that the fused cyclylene formed by fusing an alicyclic ring to an aromatic ring is a divalent group.

The fused cyclylene formed by fusing an alicyclic ring to a heteroaromatic ring as used in the present disclosure refers to the generic term of divalent groups formed by fusing an alicyclic ring to a heteroaromatic ring and removing two hydrogen atoms. The description stated above about the fused cyclyl formed by fusing an alicyclic ring to a heteroaromatic ring may be applied to the fused cyclylene formed by fusing an alicyclic ring to a heteroaromatic ring except that the fused cyclylene formed by fusing an alicyclic ring to a heteroaromatic ring is a divalent group.

"Substituted" in "substituted or unsubstituted" as used in the present disclosure means that at least one hydrogen atom on the group is substituted with a substituent. When a plurality of hydrogens are substituted with a plurality of substituents, the plurality of substituents may be identical to or different from each other. The position of the hydrogen substituted with the substituent can be any position. The substituents used for "substituted" in "substituted or unsubstituted" include, but are not limited to, the following groups: deuterium, tritium, cyano, nitro, hydroxyl, a halogen atom, substituted or unsubstituted C1 to C15 alkyl, substituted or unsubstituted C2 to C15 alkenyl, a substituted or unsubstituted C3 to C15 alicyclic group, substituted or unsubstituted C6 to C30 aryl, substituted or unsubstituted C2 to C30 heteroaryl, substituted or unsubstituted fused cyclyl formed by fusing a C3 to C15 alicyclic ring to a C6 to C30 aromatic ring, substituted or unsubstituted fused cyclyl formed by fusing a C3 to C15 alicyclic ring to a C2 to C30 heteroaromatic ring, substituted or unsubstituted C1 to C15 alkoxy, substituted or unsubstituted C1 to C15 alkylthio, substituted or unsubstituted C6 to C30 aryloxy, and substituted or unsubstituted C6 to C30 arylamine. The substituent is preferably selected from the following groups: deuterium, tritium, cyano, fluorine, chlorine, bromine, iodine, nitro, methyl, ethyl, propyl, butyl, pentyl, hexyl, heptyl, octyl, cyclopropyl, cyclobutyl, cyclopentyl, cyclohexyl, cyclobutenyl, cyclopentenyl, cyclohexenyl, adamantyl, norbornyl, trifluoromethyl, phenyl, biphenyl, terphenyl, naphthyl, phenanthryl, triphenylenyl, anthryl, pyrenyl, chrysenyl, fluoranthenyl, benzocyclopropyl, benzocyclobutyl, benzocyclopentyl, benzocyclohexyl, benzocycloheptyl, benzocyclobutenyl, benzcyclopentenyl, benzocyclohexenyl, 9,9-dimethylfluorenyl, 9,9-diphenylfluorenyl, 9-methyl-9-phenylfluorenyl, 9,9'-spirobifluorenyl, pyridyl, pyrimidinyl, pyrazinyl, pyridazinyl, triazinyl, quinolyl, isoquinolyl, quinazolinyl, quinoxalinyl, benzoquinolyl, benzisoquinolyl, phenanthrolinyl, oxazolyl, benzoxazolyl, thiazolyl, benzothiazolyl, imidazolyl, benzimidazolyl, benzofuryl, dibenzofuryl, benzothienyl, dibenzothienyl, indolyl, carbazolyl, and the like. In addition, each of the preceding substituents may be substituted or unsubstituted, and two adjacent substituents may be linked to form a ring.

The present disclosure provides a triarylamine compound. The triarylamine compound is represented by a structure shown in Formula 1: 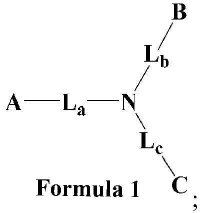 wherein A is selected from Formula 2, B is selected from Formula 3, and C is selected from Formula 3: 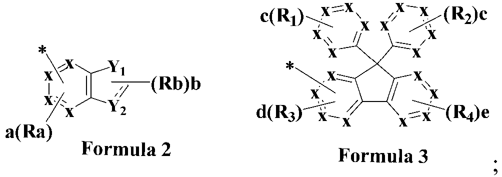
Y₁ is selected from O or S; Y₂ is selected from CH, O or S;
"--" is selected from none or a single bond; when Y₂ is selected from CH, "--" is selected from a single bond; when Y₂ is selected from O or S, "--" is selected from none;
Rₐ, R_{b}, R₁, R₂, R₃ and R₄ are independently selected from any one of hydrogen, deuterium, tritium, cyano, halogen, nitro, substituted or unsubstituted C1 to C15 alkyl, a substituted or unsubstituted C3 to C15 alicyclic group, substituted or unsubstituted silyl, substituted or unsubstituted C6 to C30 aryl, substituted or unsubstituted fused cyclyl formed by fusing a C3 to C15 alicyclic ring to a C6 to C30 aromatic ring, substituted or unsubstituted C2 to C30 heteroaryl, substituted or unsubstituted fused cyclyl formed by fusing a C3 to C15 alicyclic ring to a C2 to C30 heteroaromatic ring, and combinations thereof; or two adjacent R₁, two adjacent R₂, two adjacent R₃ or two adjacent R₄ can be linked to each other to form a substituted or unsubstituted ring;
x is identically or differently selected from N or CH; x at a linkage is selected from C;
a is selected from 0, 1, 2 or 3; b is selected from 0, 1 or 2; c is selected from 0, 1, 2, 3, 4 or 5; d is selected from 0, 1, 2 or 3; e is selected from 0, 1, 2, 3 or 4; when two or more Rₐ, R_{b}, R₁, R₂, R₃ and R₄ are present, the two or more Rₐ, R_{b}, R₁, R₂, R₃ and R₄ are identical to or different from each other;
Lₐ is selected from any one of a single bond, substituted or unsubstituted phenylene, substituted or unsubstituted biphenylylene, substituted or unsubstituted terphenylene, substituted or unsubstituted naphthylene, substituted or unsubstituted phenanthrylene, substituted or unsubstituted triphenylenylene, substituted or unsubstituted fluorenylene, substituted or unsubstituted dibenzofurylene, substituted or unsubstituted dibenzothienylene, substituted or unsubstituted carbazolylene, substituted or unsubstituted benzocyclopropylene, substituted or unsubstituted benzocyclobutylene, substituted or unsubstituted benzocyclopentylene, substituted or unsubstituted benzocyclohexylene, substituted or unsubstituted benzocycloheptylene, substituted or unsubstituted benzocyclobutenylene, substituted or unsubstituted benzocyclopentenylene, substituted or unsubstituted benzocyclohexynylene, substituted or unsubstituted benzocycloheptenylene, substituted or unsubstituted pyridylene, substituted or unsubstituted pyrimidinylene, substituted or unsubstituted pyridazinylene, substituted or unsubstituted pyrazinylene, substituted or unsubstituted quinolylene, substituted or unsubstituted isoquinolylene, substituted or unsubstituted quinazolinylene, substituted or unsubstituted quinoxalinylene, and combinations thereof;
L_{b} and L_{c} are independently selected from any one of a single bond, substituted or unsubstituted C6 to C30 arylene, substituted or unsubstituted C2 to C30 heteroarylene, substituted or unsubstituted fused cyclylene formed by fusing a C3 to C15 alicyclic ring to a C6 to C30 aromatic ring, substituted or unsubstituted fused cyclylene formed by fusing a C3 to C15 alicyclic ring to a C2 to C30 heteroaromatic ring, and combinations thereof.

Preferably, Formula 2 is selected from any one of the following groups: 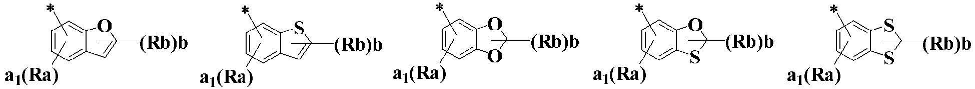 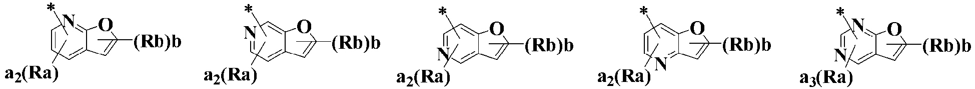 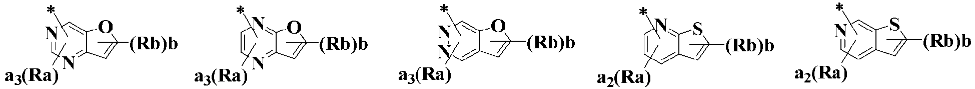 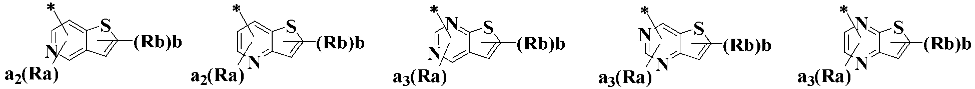 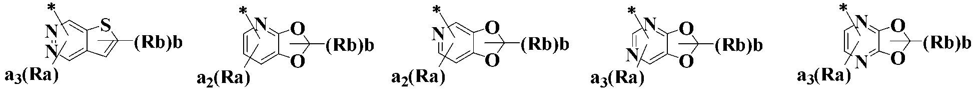 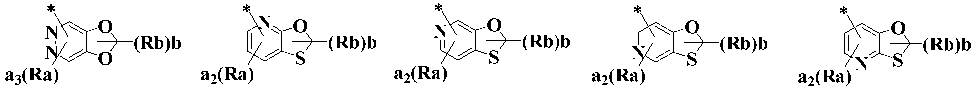 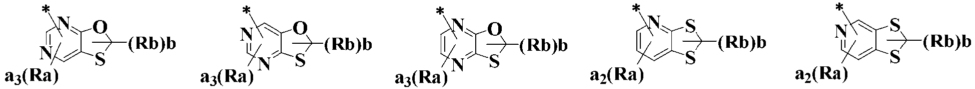 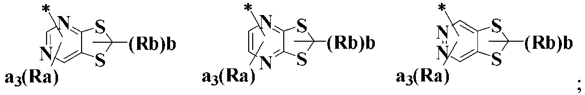 wherein a₁ is selected from 0, 1, 2 or 3; a₂ is selected from 0, 1 or 2; a₃ is selected from 0 or 1; b is selected from 0, 1 or 2; when two or more Rₐ and R_{b} are present, the two or more Rₐ and R_{b} are identical to or different from each other.

Preferably, Rₐ and R_{b} are independently selected from any one of hydrogen, deuterium, tritium, cyano, halogen, nitro, substituted or unsubstituted following groups: methyl, ethyl, *n*-propyl, isopropyl, *n*-butyl, isobutyl, *tert-*butyl*,* cyclopropyl, cyclobutyl, cyclopentyl, cyclohexyl, adamantyl, norbornyl, phenyl, biphenyl, terphenyl, naphthyl, anthryl, phenanthryl, triphenylenyl, benzocyclobutyl, benzocyclopentyl, benzocyclohexyl, benzocyclopentenyl, benzocyclohexenyl, fluorenyl, benzofuryl, dibenzofuryl, benzothienyl, dibenzothienyl, benzoxazolyl, benzothiazolyl, benzimidazolyl, indolyl, carbazolyl, pyridyl, pyrimidinyl, pyrazinyl, pyridazinyl, quinolyl, isoquinolyl, quinazolinyl, quinoxalinyl, trimethylsilyl, triethylsilyl, tripropylsilyl, tri-tert-butylsilyl, triphenylsilyl and combinations thereof.

The substituent in "substituted or unsubstituted" in Rₐ and R_{b} is selected from one or more of deuterium, tritium, halogen, cyano, methyl, ethyl, *n*-propyl, isopropyl, *n*-butyl, isobutyl, *tert-butyl,* cyclopropyl, cyclobutyl, cyclopentyl, cyclohexyl, adamantyl, norbornyl, phenyl, biphenyl or naphthyl, and when two or more substituents are present, the two or more substituents are present are identical to or different from each other.

Preferably, R₁, R₂, R₃ and R₄ are independently selected from any one of hydrogen, deuterium, tritium, cyano, halogen, nitro, substituted or unsubstituted following groups: methyl, ethyl, n-propyl, isopropyl, *n*-butyl, isobutyl, *tert-*butyl*,* cyclopropyl, cyclobutyl, cyclopentyl, cyclohexyl, adamantyl, norbornyl, phenyl, biphenyl, terphenyl, naphthyl, anthryl, phenanthryl, triphenylenyl, benzocyclobutyl, benzocyclopentyl, benzocyclohexyl, benzocyclopentenyl, benzocyclohexenyl, fluorenyl, benzofuryl, dibenzofuryl, benzothienyl, dibenzothienyl, benzoxazolyl, benzothiazolyl, benzimidazolyl, indolyl, carbazolyl, pyridyl, pyrimidinyl, pyrazinyl, pyridazinyl, quinolyl, isoquinolyl, quinazolinyl, quinoxalinyl, trimethylsilyl, triethylsilyl, tripropylsilyl, tri-*tert*-butylsilyl, triphenylsilyl and combinations thereof; or two adjacent R₁, two adjacent R₂, two adjacent R₃ or two adjacent R₄ can be linked to each other to form a substituted or unsubstituted ring;
the substituent in "substituted or unsubstituted" in R₁, R₂, R₃ and R₄ is selected from one or more of deuterium, tritium, halogen, cyano, methyl, ethyl, *n*-propyl, isopropyl, *n*-butyl, isobutyl, *tert-*butyl*,* cyclopropyl, cyclobutyl, cyclopentyl, cyclohexyl, adamantyl, norbornyl, phenyl, biphenyl or naphthyl, and when two or more substituents are present, the two or more substituents are present are identical to or different from each other.

More preferably, Formula 3 is selected from any one of the following groups:

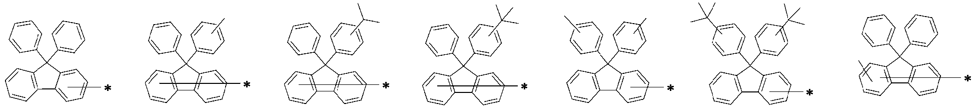

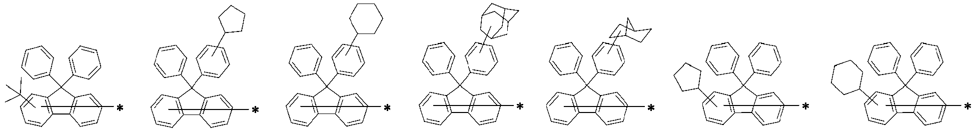

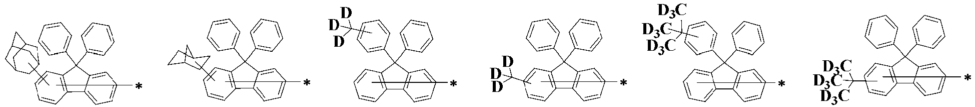

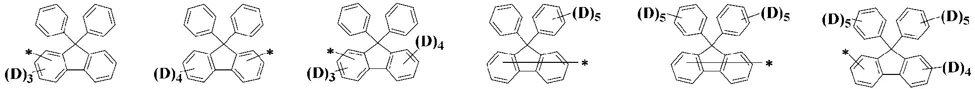

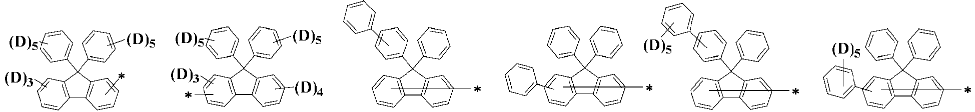

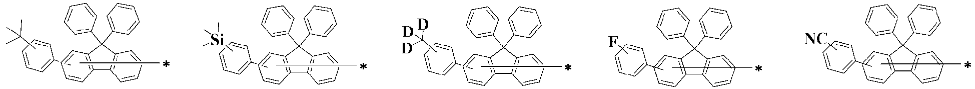

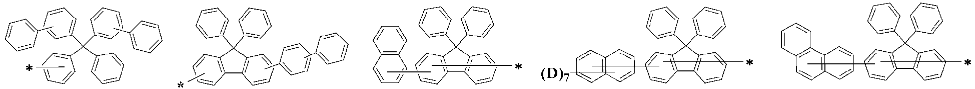

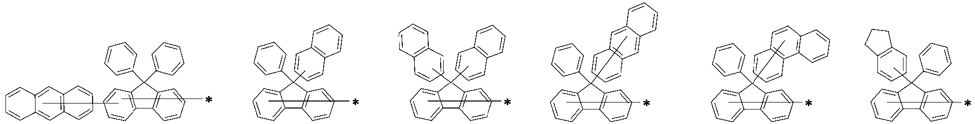

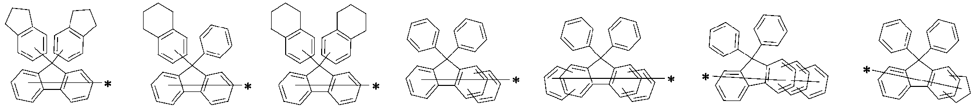

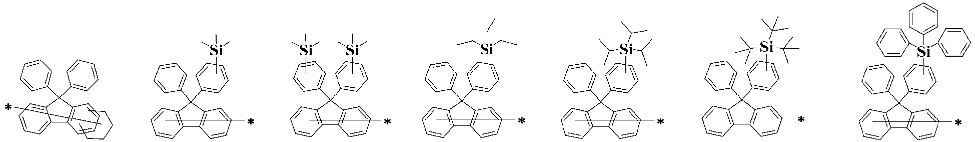

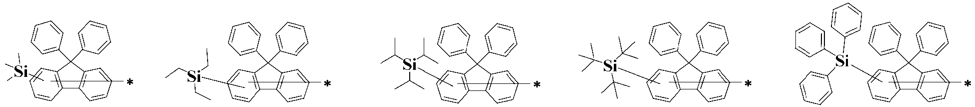

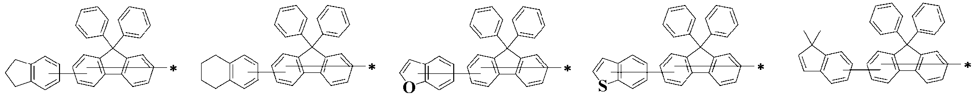

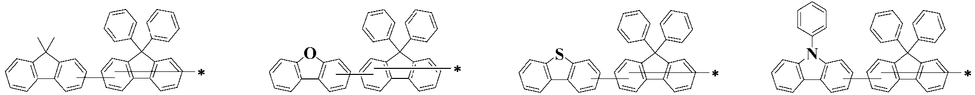

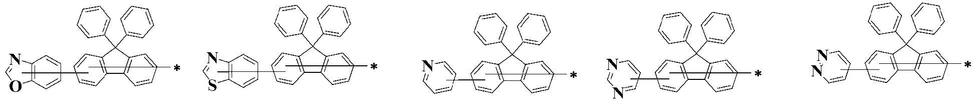

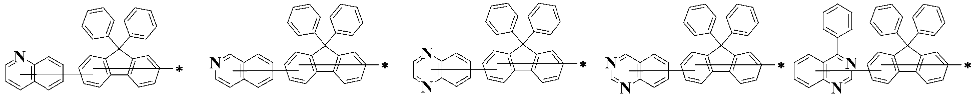

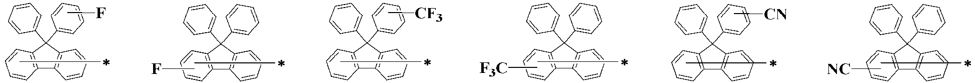

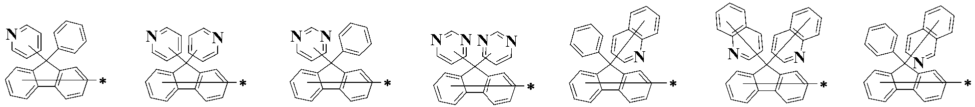

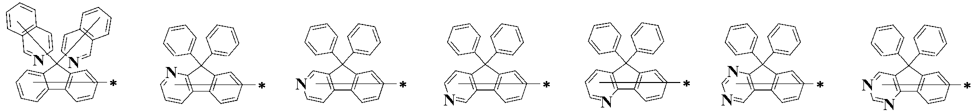

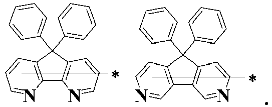

Preferably, Lₐ is selected from a single bond or any one of the following groups: 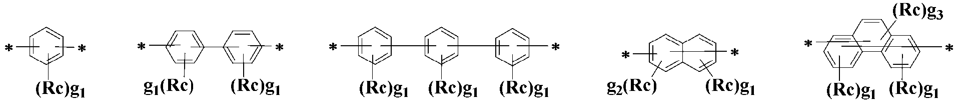 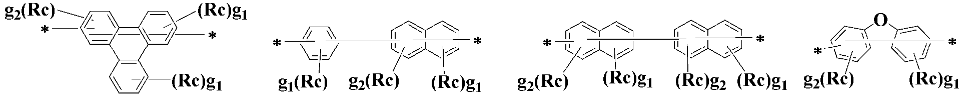 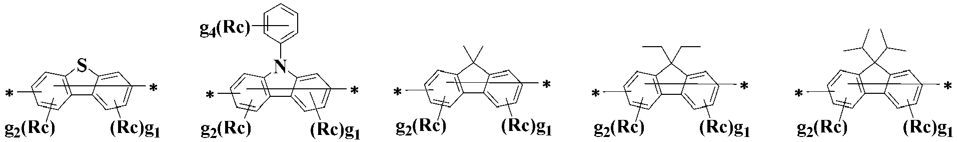 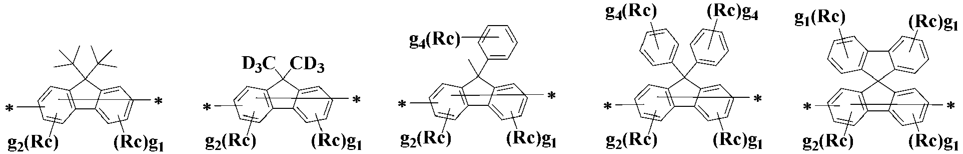 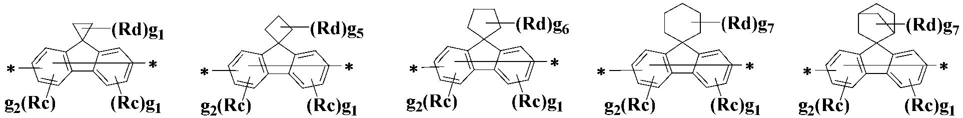 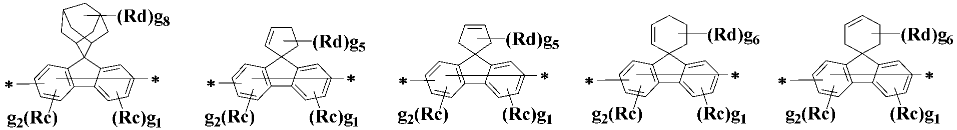 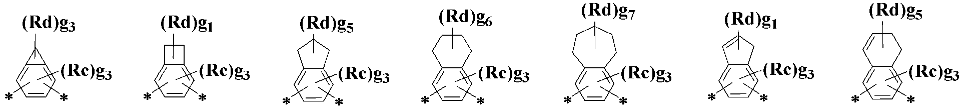 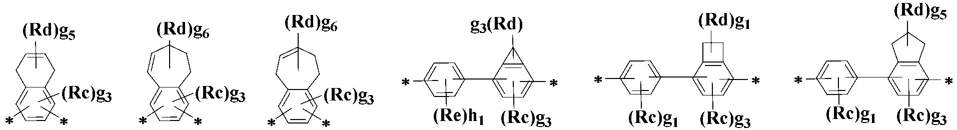 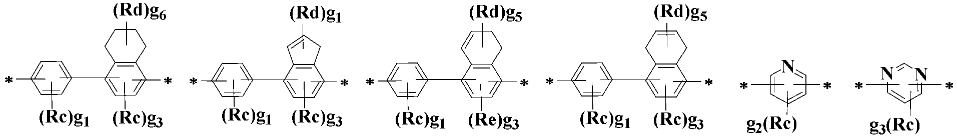 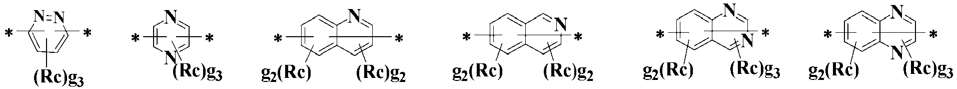 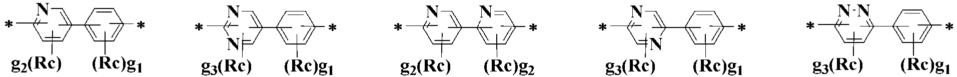 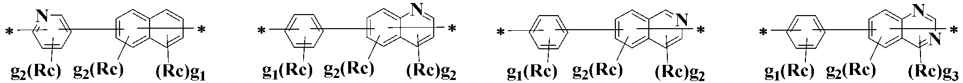 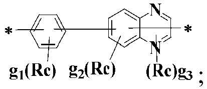
wherein R_{c} is identically or differently selected from any one of hydrogen, deuterium, tritium, cyano, halogen, trifluoromethyl, substituted or unsubstituted following groups: methyl, ethyl, *n*-propyl, isopropyl, *n*-butyl, isobutyl, *tert-*butyl, cyclopropyl, cyclobutyl, cyclopentyl, cyclohexyl, adamantyl, norbornyl, phenyl, biphenyl, terphenyl, naphthyl, anthryl, phenanthryl, triphenylenyl, fluorenyl, benzofuryl, dibenzofuryl, benzothienyl, dibenzothienyl, benzoxazolyl, benzothiazolyl, benzimidazolyl, indolyl, carbazolyl, pyridyl, pyrimidinyl, pyrazinyl, pyridazinyl, quinolyl, isoquinolyl, quinazolinyl, quinoxalinyl, trimethylsilyl, triethylsilyl, tripropylsilyl, tri-*tert*-butylsilyl, triphenylsilyl and combinations thereof;
R_{d} is identically or differently selected from any one of hydrogen, deuterium, tritium, cyano, halogen, trifluoromethyl, substituted or unsubstituted following groups: methyl, ethyl, *n*-propyl, isopropyl, *n*-butyl, isobutyl, *tert-*butyl, cyclopropyl, cyclobutyl, cyclopentyl, cyclohexyl, adamantyl, norbornyl, phenyl, biphenyl, terphenyl, naphthyl, anthryl, phenanthryl, triphenylenyl, fluorenyl, benzofuryl, dibenzofuryl, benzothienyl, dibenzothienyl, carbazolyl, trimethylsilyl, triethylsilyl, tripropylsilyl, tri-*tert*-butylsilyl, triphenylsilyl and combinations thereof;
g₁ is selected from 0, 1, 2, 3 or 4; g₂ is selected from 0, 1, 2 or 3; g₃ is selected from 0, 1 or 2; g₄ is selected from 0, 1, 2, 3, 4 or 5; g₅ is selected from 0, 1, 2, 3, 4, 5 or 6; g₆ is independently selected from 0, 1, 2, 3, 4, 5, 6, 7 or 8; g₇ is selected from 0, 1, 2, 3, 4, 5, 6, 7, 8, 9 or 10; g₈ is selected from 0, 1, 2, 3, 4, 5, 6, 7, 8, 9, 10, 11, 12, 13 or 14; when two or more R_{c} and R_{d} are present, the two or more R_{c} and R_{d} are identical to or different from each other.

The substituent in "substituted or unsubstituted" in R_{c} and R_{d} is selected from one or more of deuterium, tritium, halogen, cyano, methyl, ethyl, *n*-propyl, isopropyl, *n*-butyl, isobutyl, *tert-*butyl*,* cyclopropyl, cyclobutyl, cyclopentyl, cyclohexyl, adamantyl, norbornyl, phenyl, biphenyl or naphthyl, and when two or more substituents are present, the two or more substituents are present are identical to or different from each other.

More preferably, Lₐ is selected from a single bond or any one of the following groups:

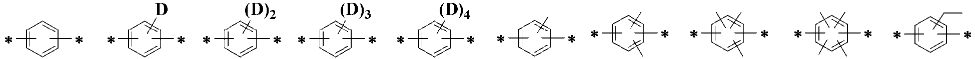

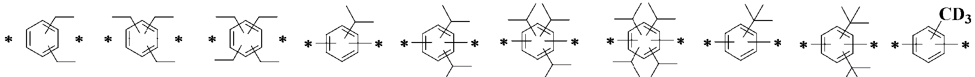

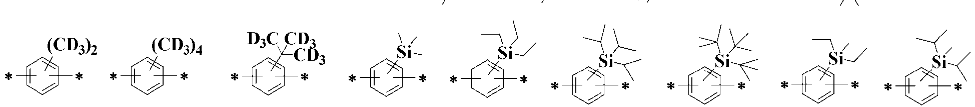

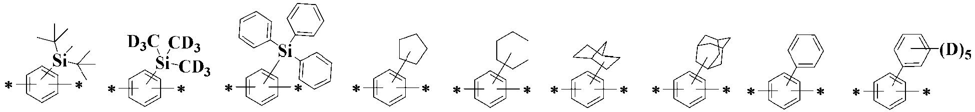

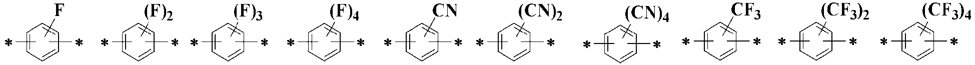

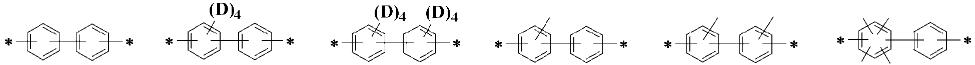

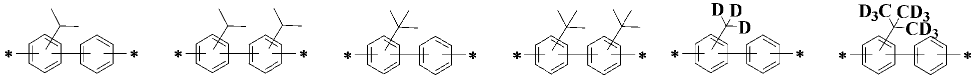

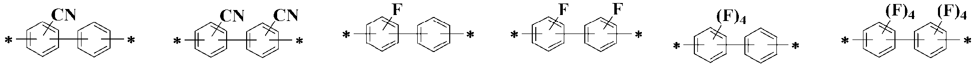

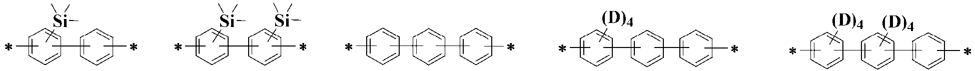

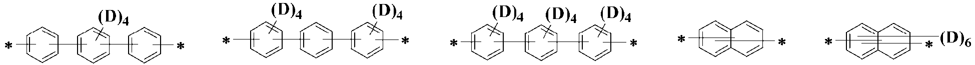

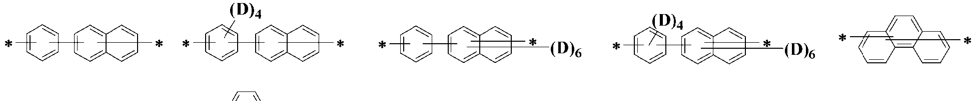

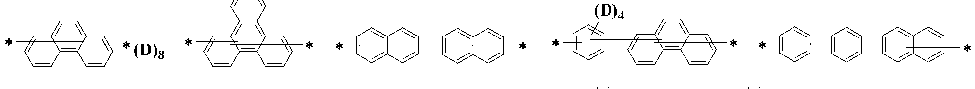

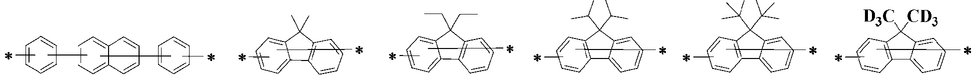

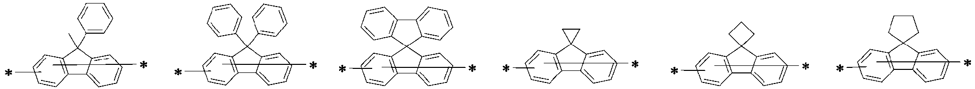

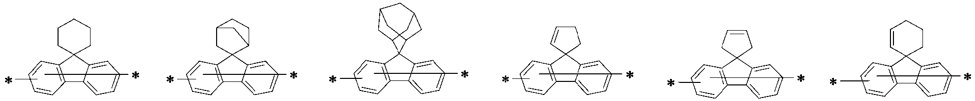

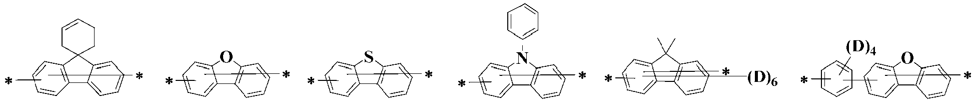

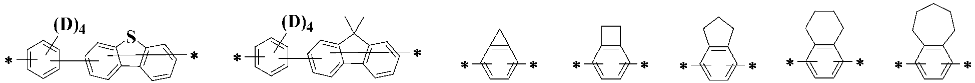

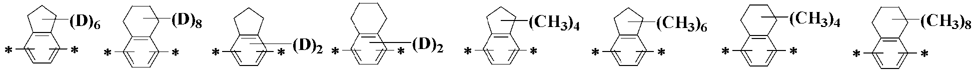

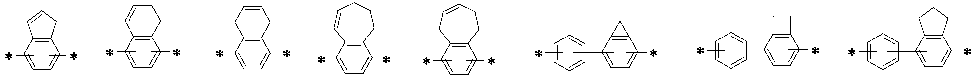

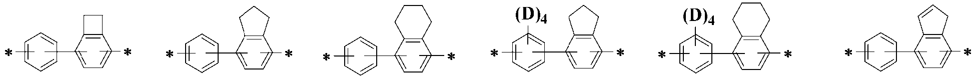

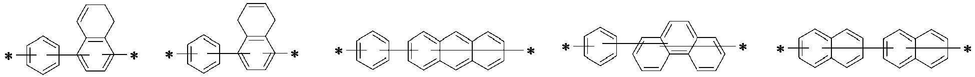

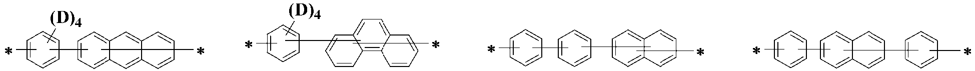

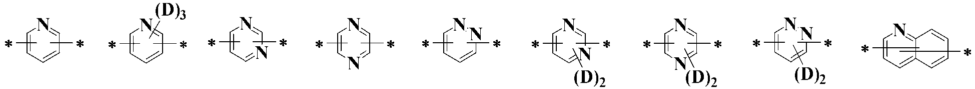

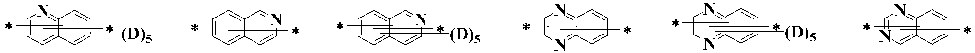

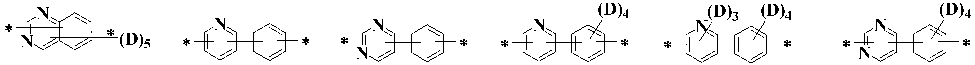

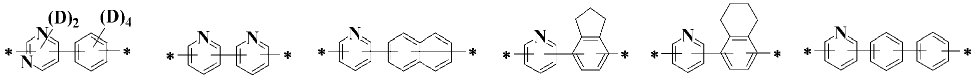

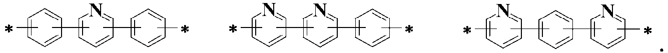

Preferably, L_{b} and L_{c} are independently selected from a single bond or any one of the following groups: 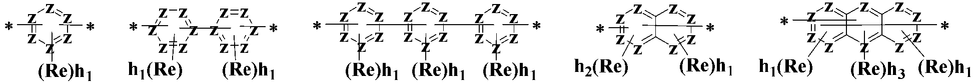 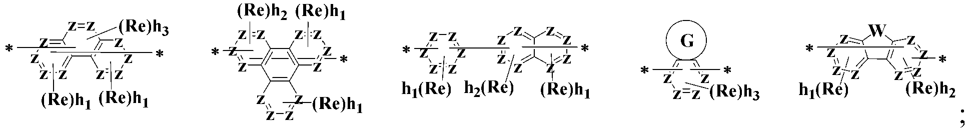
wherein z is identically or differently selected from N or CH; z at a linkage is selected from C;
Rₑ is identically or differently selected from any one of hydrogen, deuterium, tritium, cyano, halogen, nitro, substituted or unsubstituted C1 to C15 alkyl, a substituted or unsubstituted C3 to C15 alicyclic group, substituted or unsubstituted silyl, substituted or unsubstituted C6 to C30 aryl, substituted or unsubstituted fused cyclyl formed by fusing a C3 to C15 alicyclic ring to a C6 to C30 aromatic ring, substituted or unsubstituted C2 to C30 heteroaryl, substituted or unsubstituted fused cyclyl formed by fusing a C3 to C15 alicyclic ring to a C2 to C30 heteroaromatic ring, and combinations thereof; or two adjacent Rₑ can be linked to each other to form a substituted or unsubstituted ring;
the ring G is selected from a substituted or unsubstituted C3 to C10 alicyclic group;
W is selected from O, S, N(R_{g}) or C(RₕRᵢ);
R_{g} is selected from any one of substituted or unsubstituted C1 to C15 alkyl, a substituted or unsubstituted C3 to C15 alicyclic group, substituted or unsubstituted silyl, substituted or unsubstituted C6 to C30 aryl, substituted or unsubstituted C2 to C30 heteroaryl, substituted or unsubstituted fused cyclyl formed by fusing a C3 to C15 alicyclic ring to a C6 to C30 aromatic ring, substituted or unsubstituted fused cyclyl formed by fusing a C3 to C15 alicyclic ring to a C6 to C30 heteroaromatic ring, and combinations thereof;
Rₕ and Rᵢ are independently selected from any one of hydrogen, deuterium, tritium, cyano, halogen, nitro, substituted or unsubstituted C1 to C15 alkyl, a substituted or unsubstituted C3 to C15 alicyclic group, substituted or unsubstituted silyl, substituted or unsubstituted C6 to C30 aryl, substituted or unsubstituted C2 to C30 heteroaryl, substituted or unsubstituted fused cyclyl formed by fusing a C3 to C15 alicyclic ring to a C6 to C30 aromatic ring, and combinations thereof; or Rₕ and Rᵢ can be linked to each other to form a substituted or unsubstituted ring;
h₁ is selected from 0, 1, 2, 3 or 4; h₂ is selected from 0, 1, 2 or 3; h₃ is selected from 0, 1 or 2; when two or more Rₑ and R_{f} are present, the two or more Rₑ and R_{f} are identical to or different from each other.

Further, preferably, L_{b} and L_{c} are independently selected from a single bond or any one of the following groups: 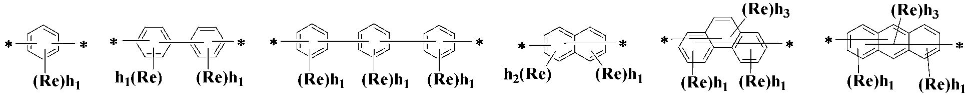 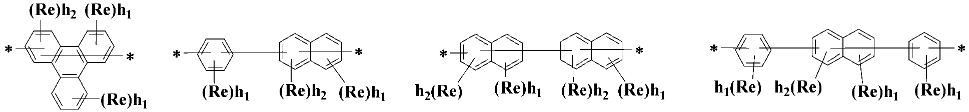 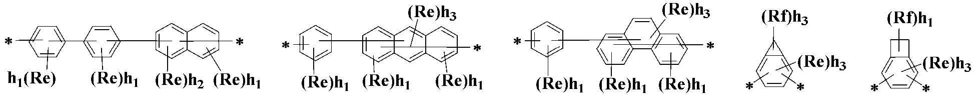 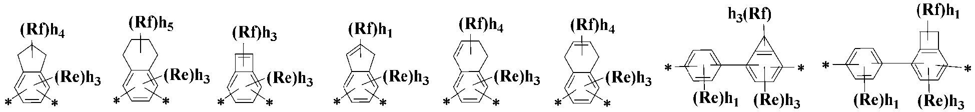 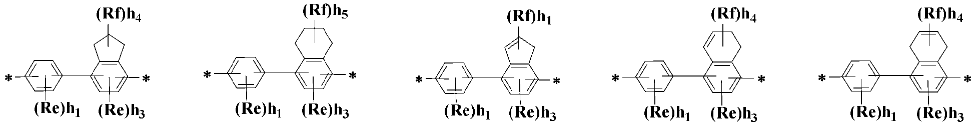 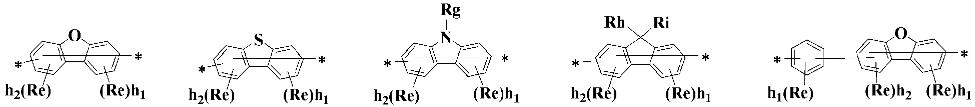 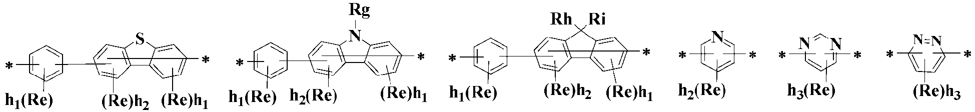 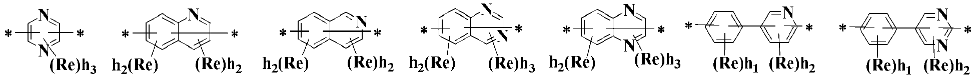 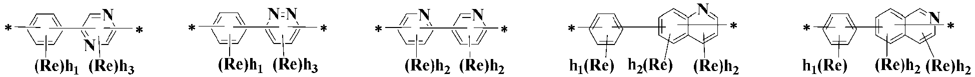 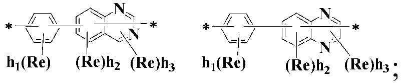
wherein Rₑ is identically or differently selected from any one of hydrogen, deuterium, tritium, cyano, halogen, trifluoromethyl, substituted or unsubstituted following groups: methyl, ethyl, *n*-propyl, isopropyl, *n*-butyl, isobutyl, *tert-butyl,* cyclopropyl, cyclobutyl, cyclopentyl, cyclohexyl, adamantyl, norbornyl, phenyl, biphenyl, terphenyl, naphthyl, anthryl, phenanthryl, triphenylenyl, fluorenyl, benzofuryl, dibenzofuryl, benzothienyl, dibenzothienyl, benzoxazolyl, benzothiazolyl, benzimidazolyl, indolyl, carbazolyl, pyridyl, pyrimidinyl, pyrazinyl, pyridazinyl, quinolyl, isoquinolyl, quinazolinyl, quinoxalinyl, trimethylsilyl, triethylsilyl, tripropylsilyl, tri-*tert*-butylsilyl, triphenylsilyl and combinations thereof, or two adjacent Rₑ can be linked to each other to form a substituted or unsubstituted ring;
R_{f} is identically or differently selected from any one of hydrogen, deuterium, tritium, cyano, halogen, trifluoromethyl, substituted or unsubstituted following groups: methyl, ethyl, *n*-propyl, isopropyl, *n*-butyl, isobutyl, *tert-butyl,* cyclopropyl, cyclobutyl, cyclopentyl, cyclohexyl, adamantyl, norbornyl, phenyl, biphenyl, terphenyl, naphthyl, anthryl, phenanthryl, triphenylenyl, fluorenyl, benzofuryl, dibenzofuryl, benzothienyl, dibenzothienyl, carbazolyl, trimethylsilyl, triethylsilyl, tripropylsilyl, tri-*tert*-butylsilyl, triphenylsilyl and combinations thereof;
R_{g} is selected from any one of substituted or unsubstituted following groups: methyl, ethyl, *n*-propyl, isopropyl, *n*-butyl, isobutyl, *tert-butyl,* cyclopropyl, cyclobutyl, cyclopentyl, cyclohexyl, adamantyl, norbornyl, phenyl, biphenyl, terphenyl, naphthyl, anthryl, phenanthryl, triphenylenyl, benzocyclobutyl, benzocyclopentyl, benzocyclohexyl, indenyl, fluorenyl, benzofuryl, dibenzofuryl, benzothienyl, dibenzothienyl, indolyl, carbazolyl, pyridyl, pyrimidinyl, quinolyl, isoquinolyl, quinazolinyl, quinoxalinyl, trimethylsilyl, triethylsilyl, tripropylsilyl, tri-*tert*-butylsilyl, triphenylsilyl and combinations thereof;
Rₕ and Rᵢ are independently selected from any one of hydrogen, deuterium, tritium, cyano, halogen, trifluoromethyl, substituted or unsubstituted following groups: methyl, ethyl, *n*-propyl, isopropyl, *n*-butyl, isobutyl, *tert-*butyl*,* cyclopropyl, cyclobutyl, cyclopentyl, cyclohexyl, adamantyl, norbornyl, phenyl, biphenyl, terphenyl, naphthyl, anthryl, phenanthryl, triphenylenyl, benzocyclobutyl, benzocyclopentyl, benzocyclohexyl, indenyl, fluorenyl, benzofuryl, dibenzofuryl, benzothienyl, dibenzothienyl, indolyl, carbazolyl, pyridyl, pyrimidinyl, quinolyl, isoquinolyl, quinazolinyl, quinoxalinyl, trimethylsilyl, triethylsilyl, tripropylsilyl, tri-*tert*-butylsilyl, triphenylsilyl and combinations thereof; or Rₕ and Rᵢ can be linked to each other to form a substituted or unsubstituted ring;
h₁ is selected from 0, 1, 2, 3 or 4; h₂ is selected from 0, 1, 2 or 3; h₃ is selected from 0, 1 or 2; h₄ is selected from 0, 1, 2, 3, 4, 5 or 6; h₅ is independently selected from 0, 1, 2, 3, 4, 5, 6, 7 or 8; when two or more Rₑ and R_{f} are present, the two or more Rₑ and R_{f} are identical to or different from each other.

The substituent in "substituted or unsubstituted" in Rₑ, R_{f}, R_{g}, Rₕ and Rᵢ is selected from one or more of deuterium, tritium, halogen, cyano, methyl, ethyl, *n*-propyl, isopropyl, *n*-butyl, isobutyl, *tert*-butyl, cyclopropyl, cyclobutyl, cyclopentyl, cyclohexyl, adamantyl, norbornyl, phenyl, biphenyl or naphthyl, and when two or more substituents are present, the two or more substituents are present are identical to or different from each other.

More preferably, L_{b} and L_{c} are independently selected from a single bond or any one of the following groups:

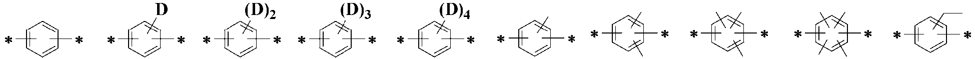

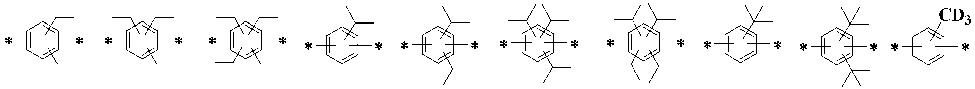

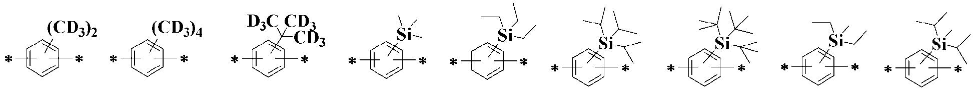

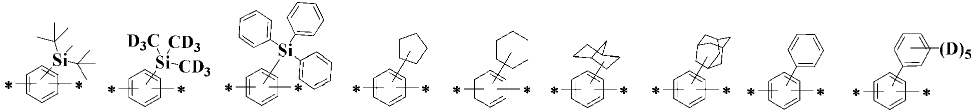

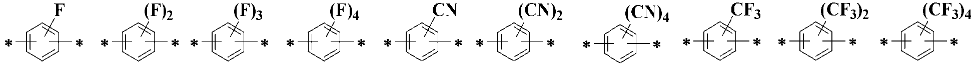

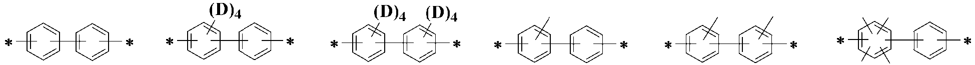

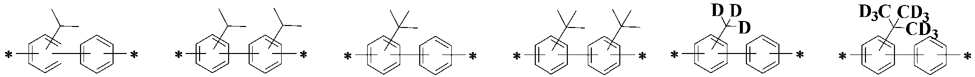

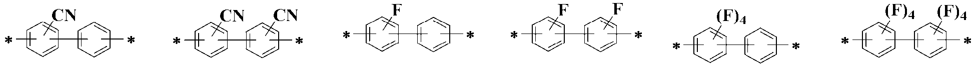

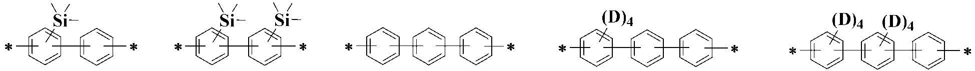

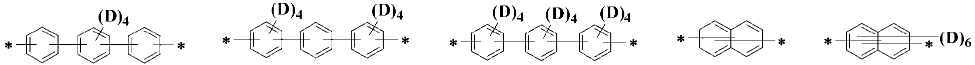

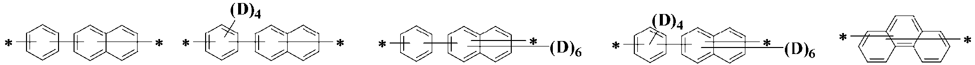

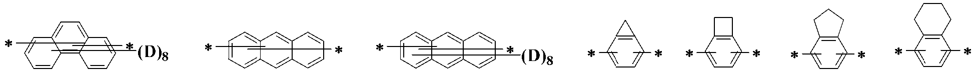

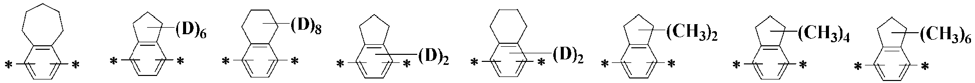

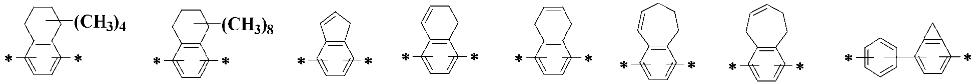

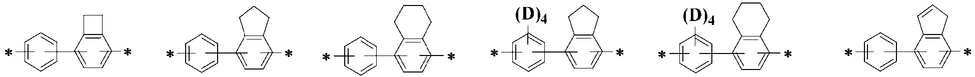

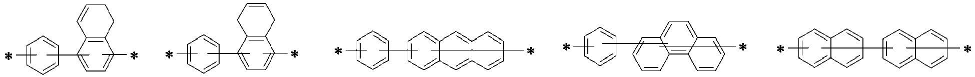

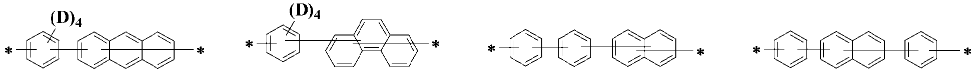

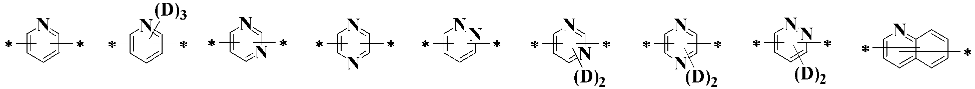

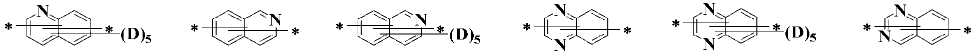

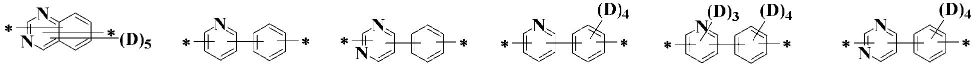

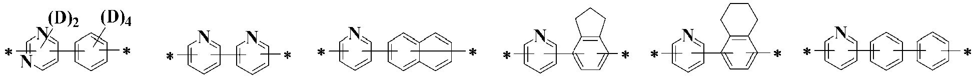

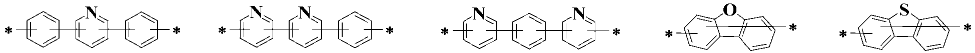

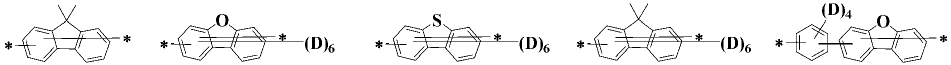

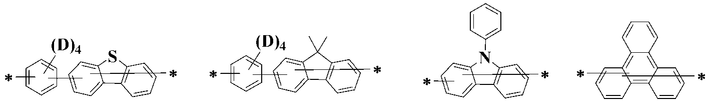

Most preferably, the triarylamine compound is selected from any one of the following structures:

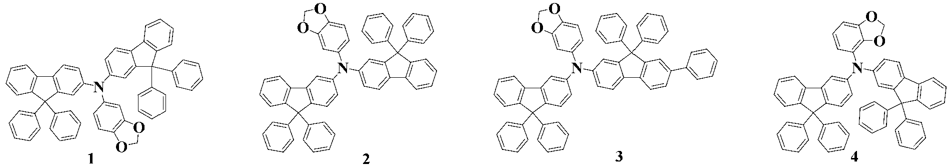

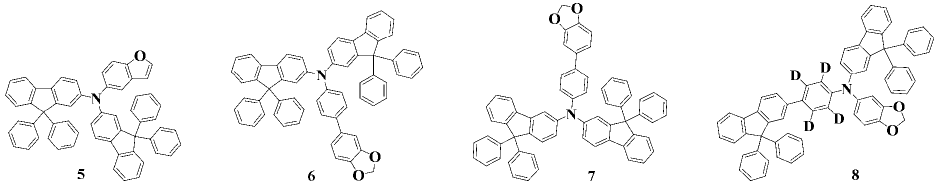

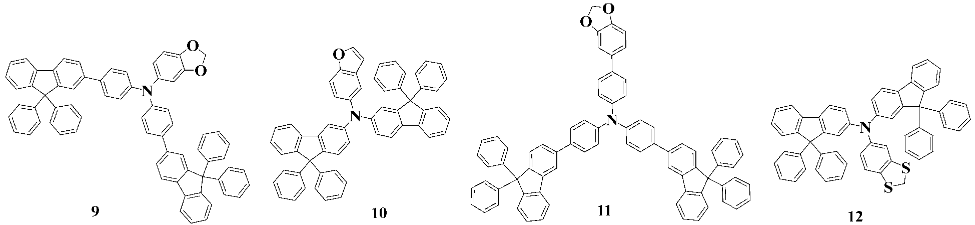

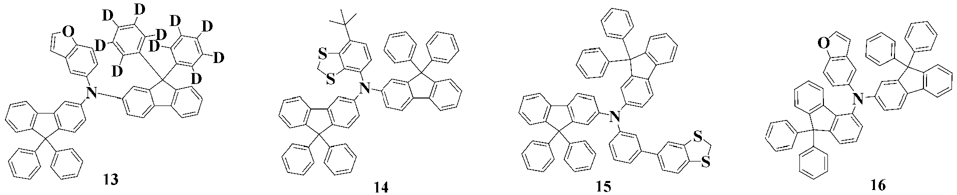

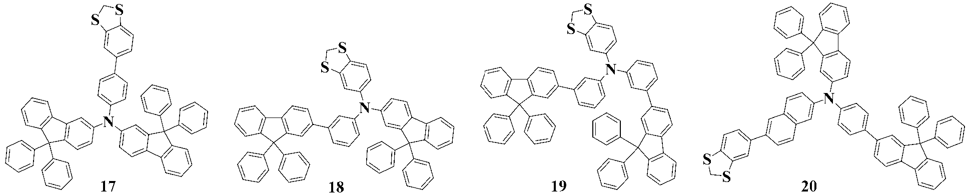

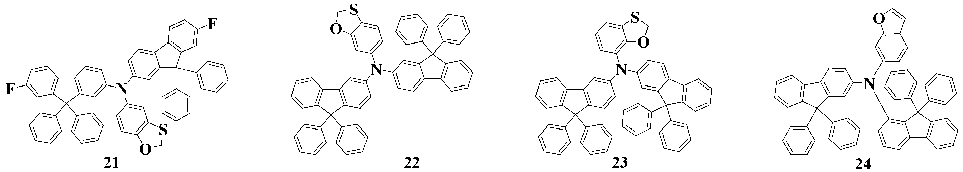

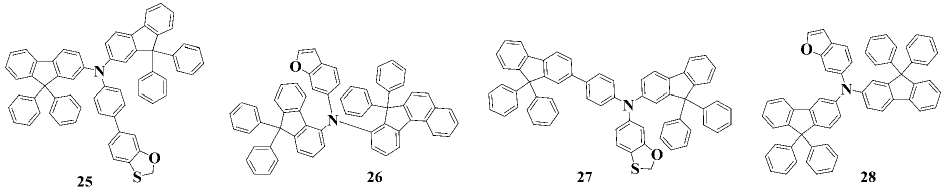

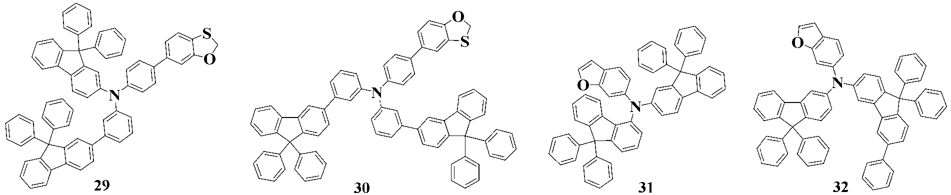

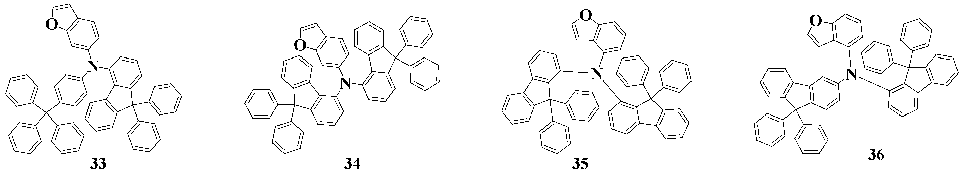

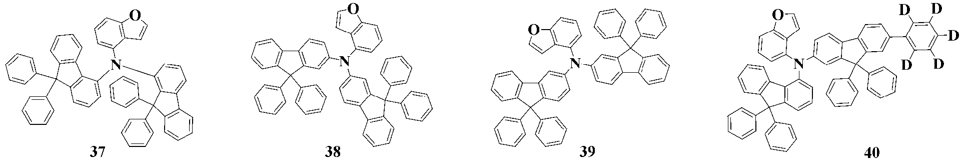

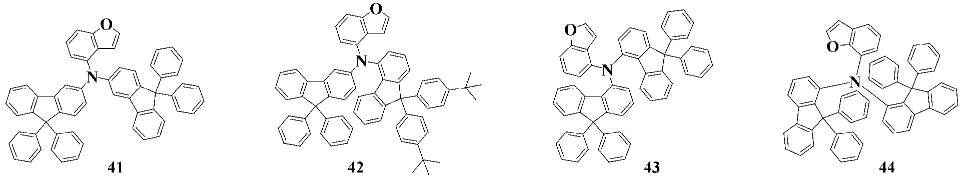

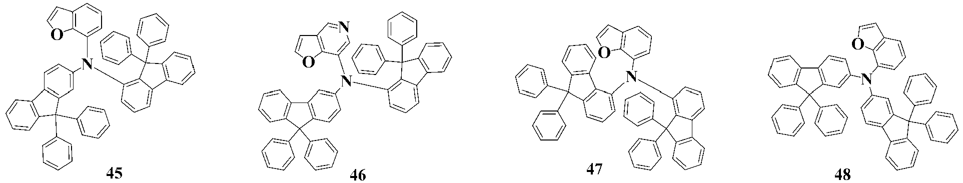

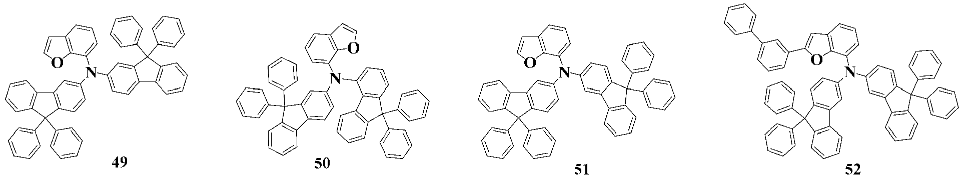

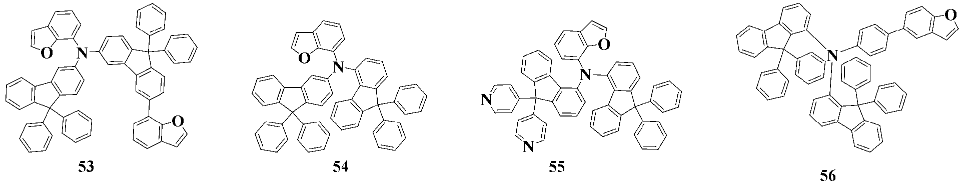

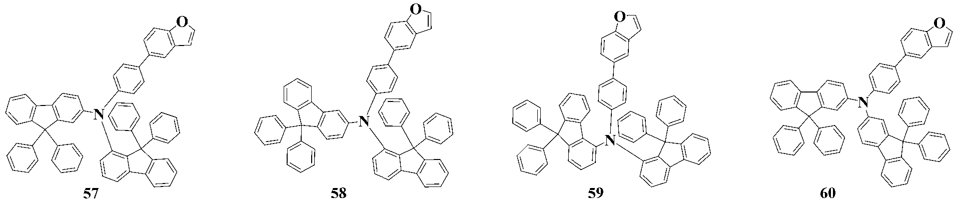

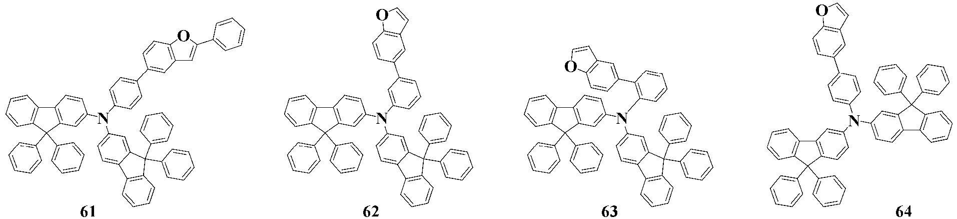

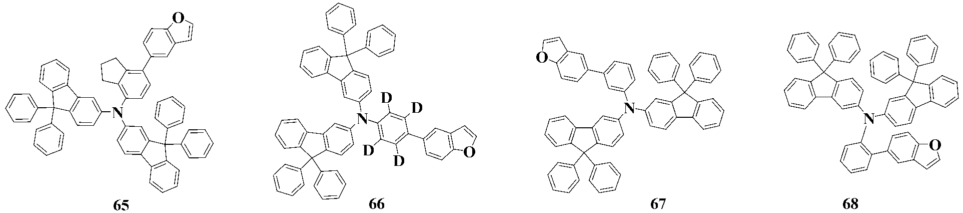

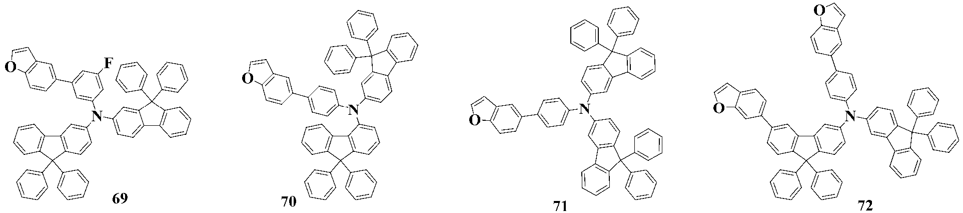

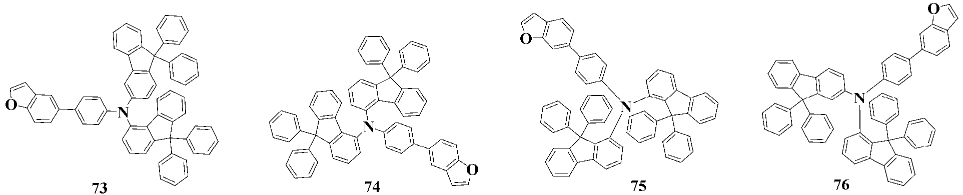

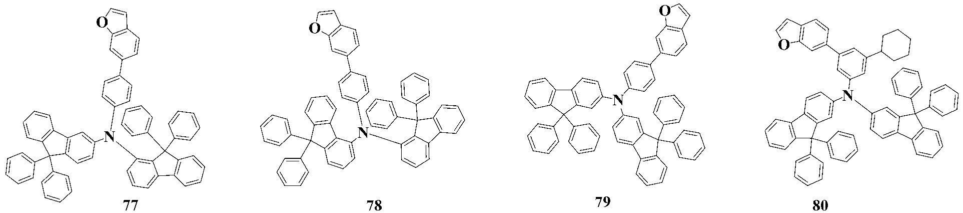

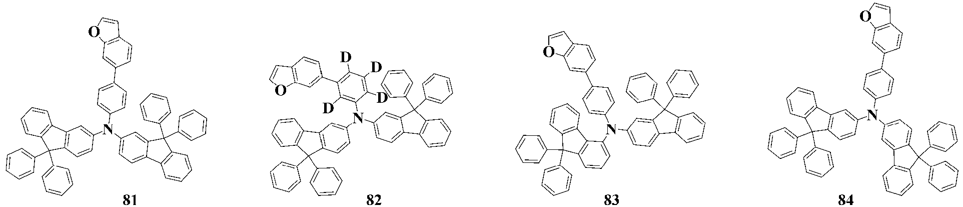

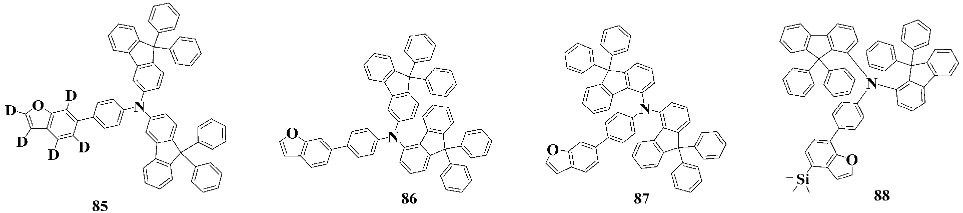

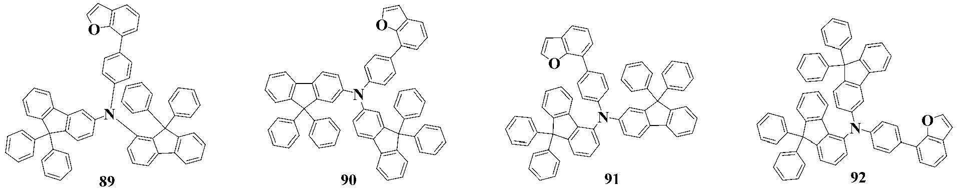

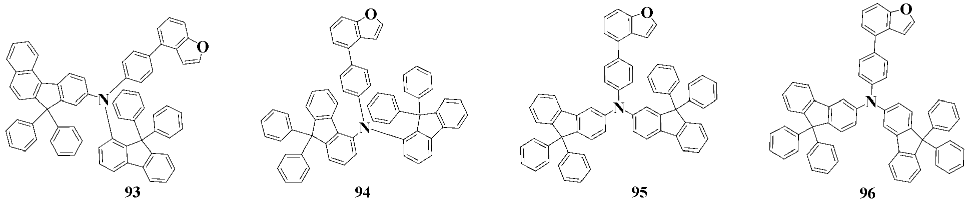

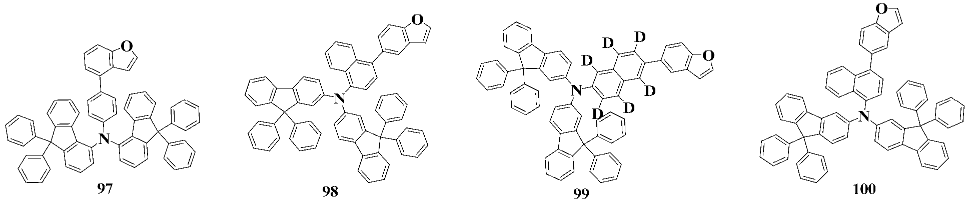

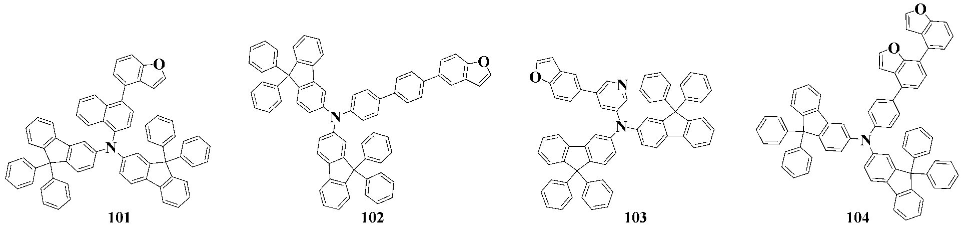

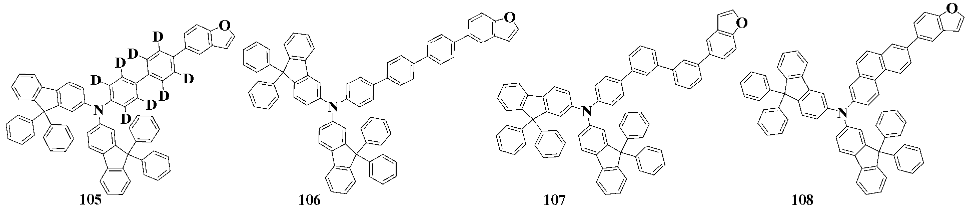

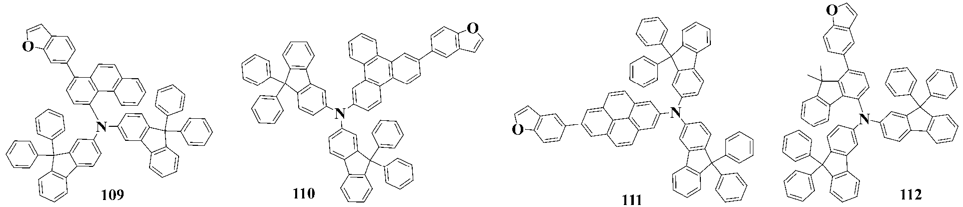

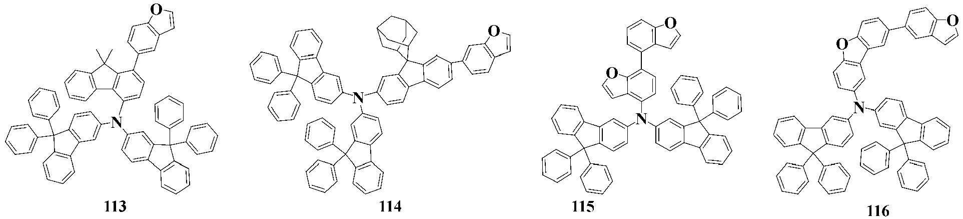

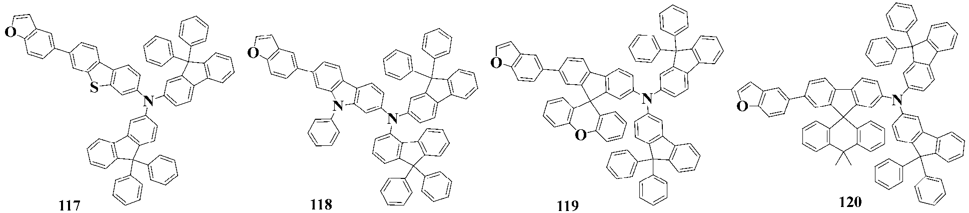

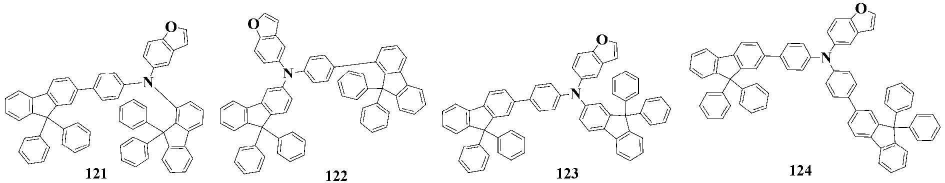

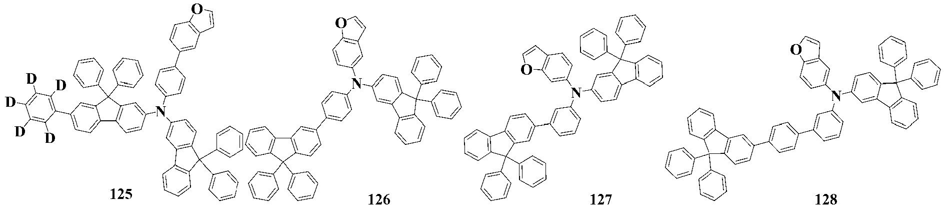

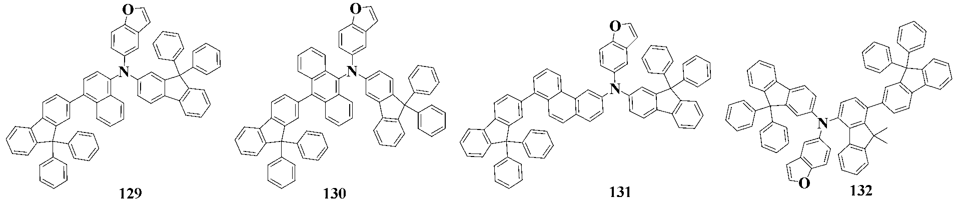

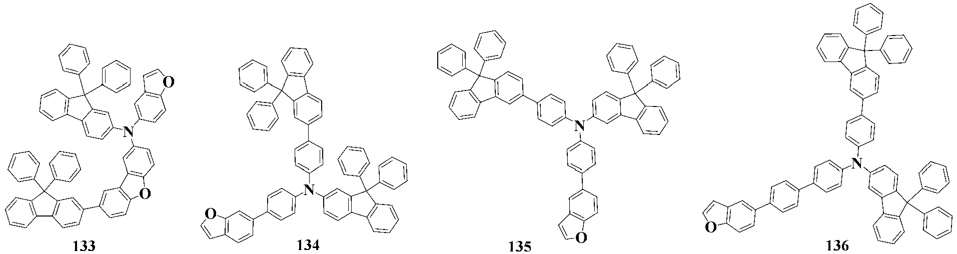

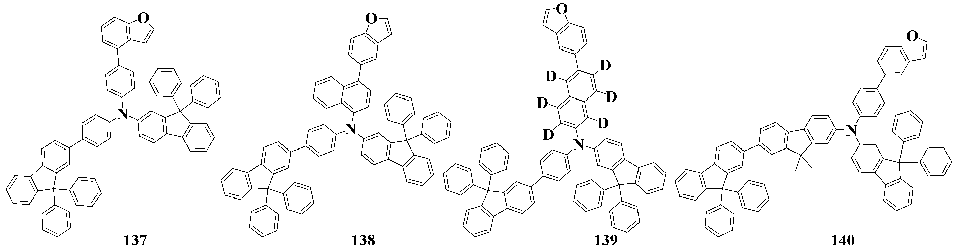

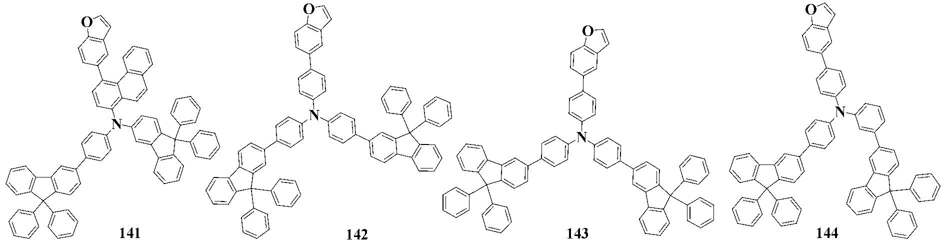

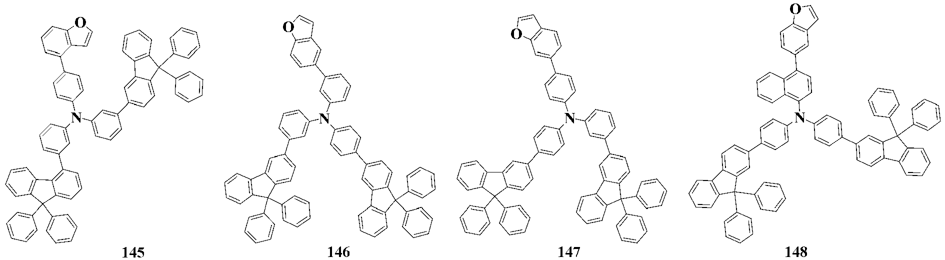

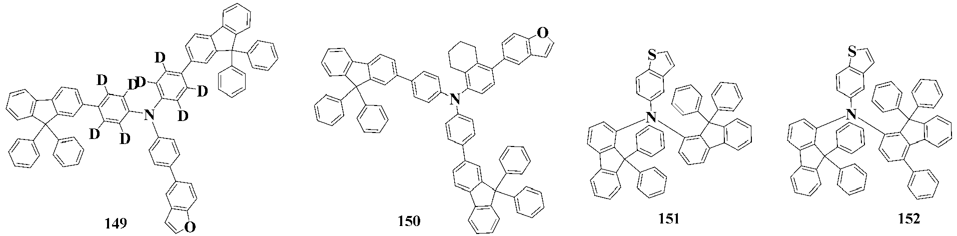

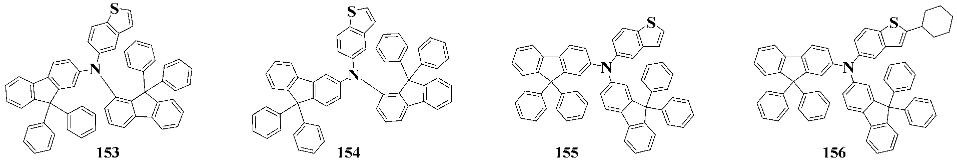

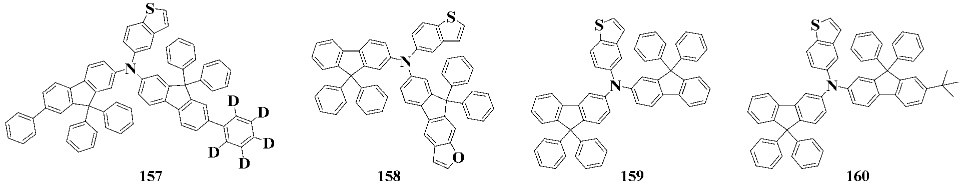

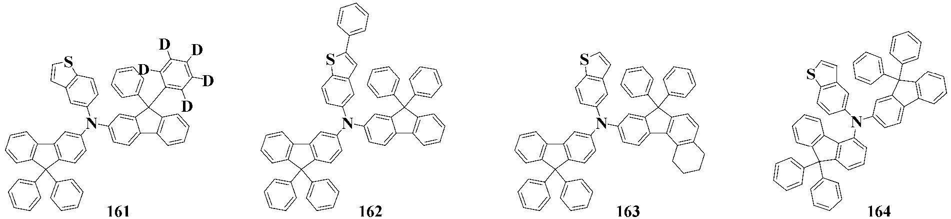

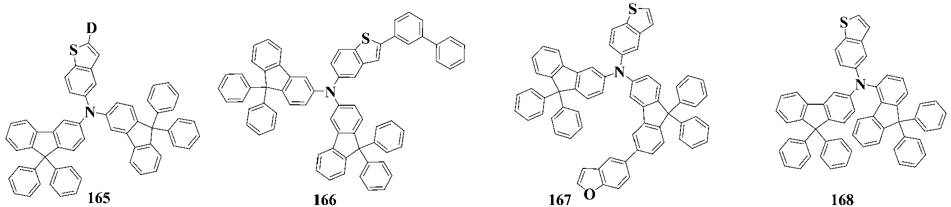

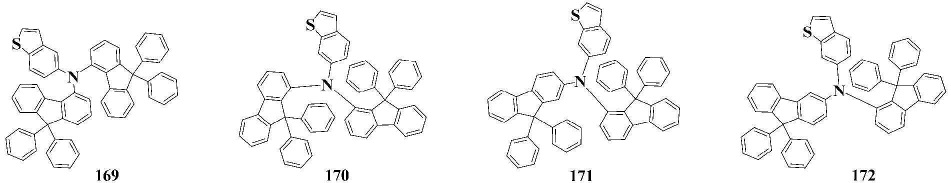

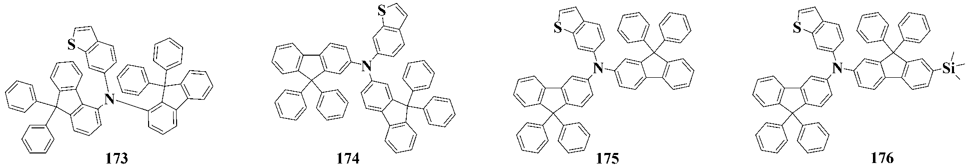

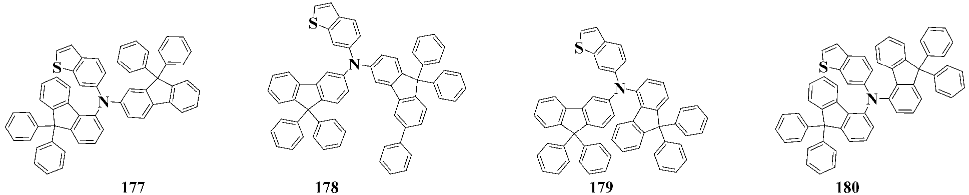

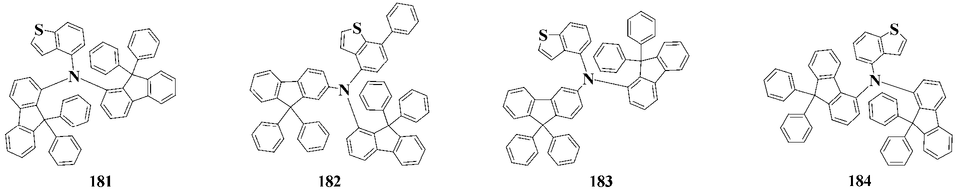

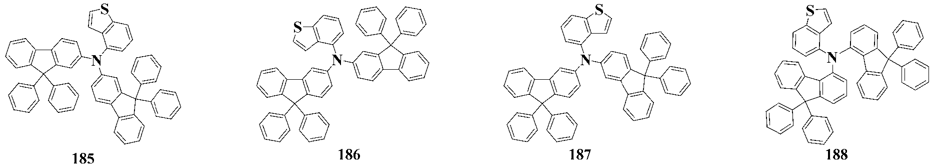

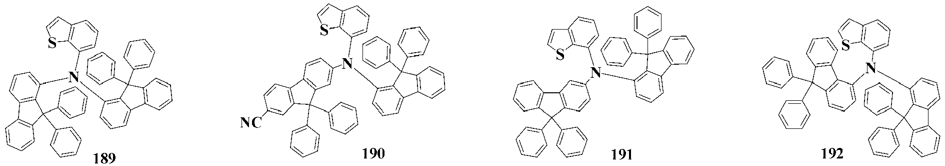

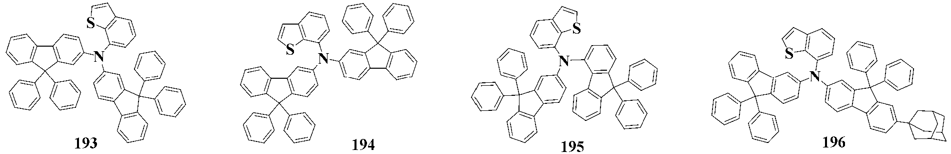

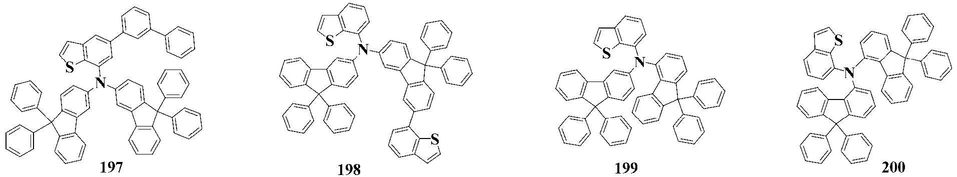

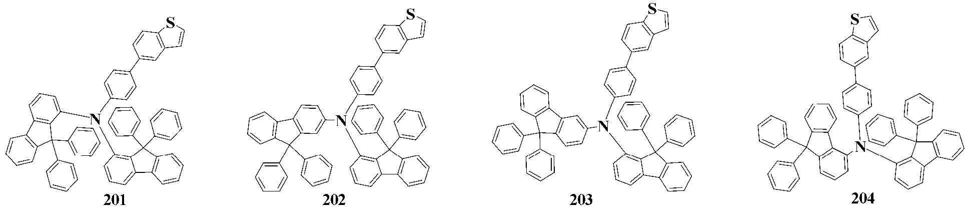

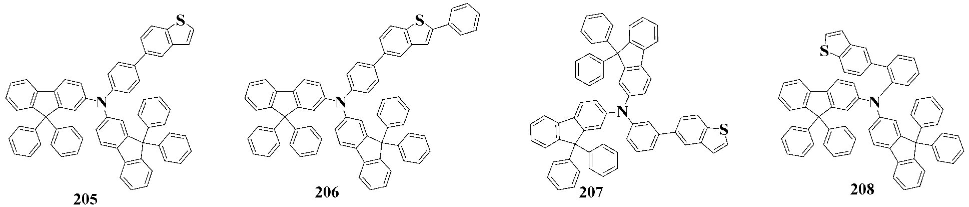

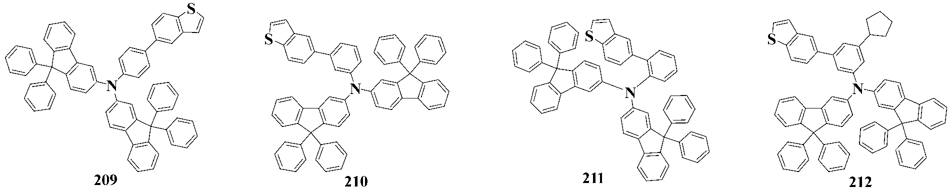

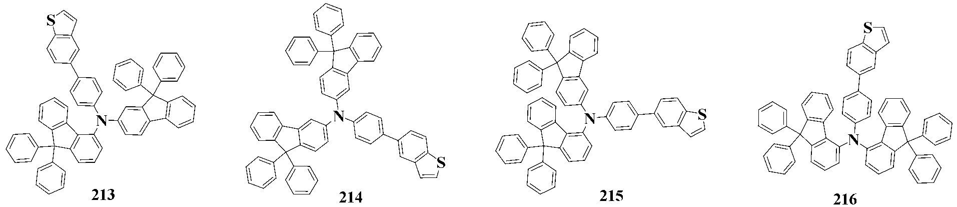

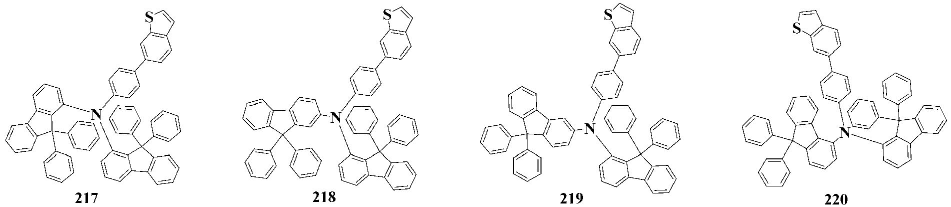

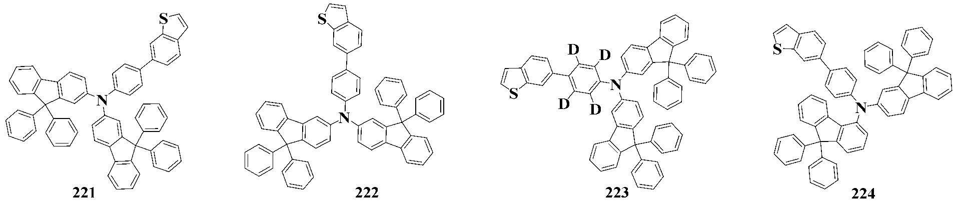

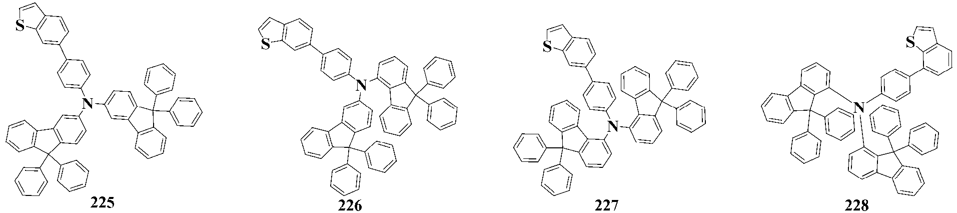

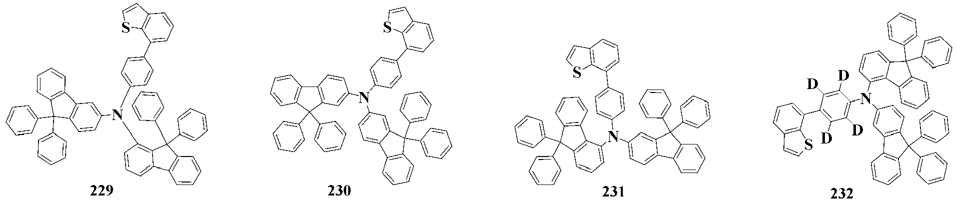

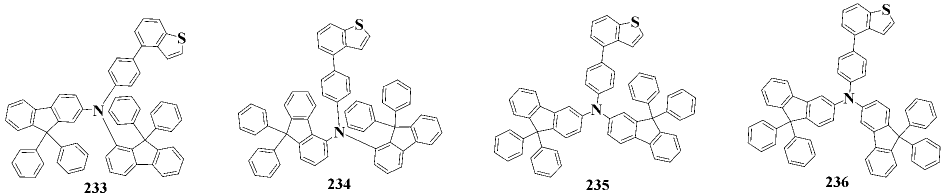

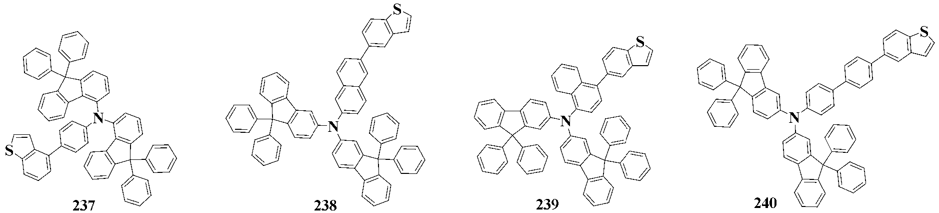

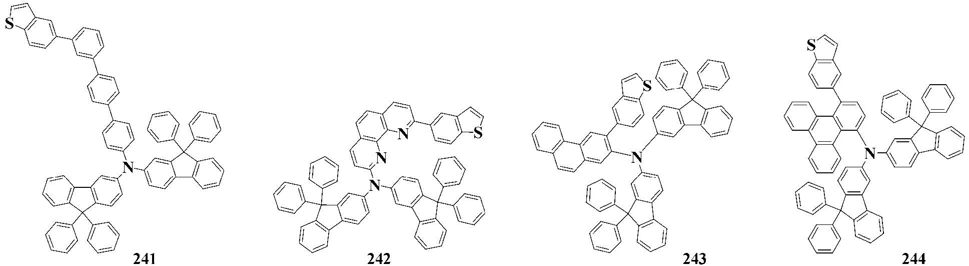

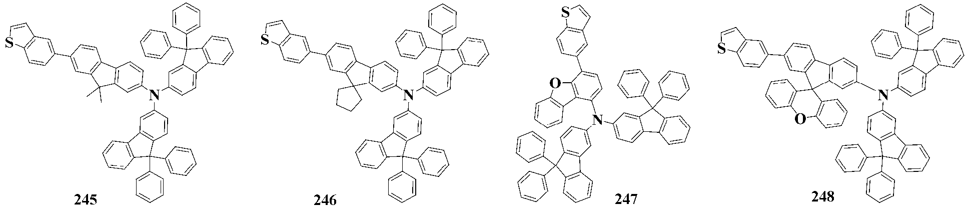

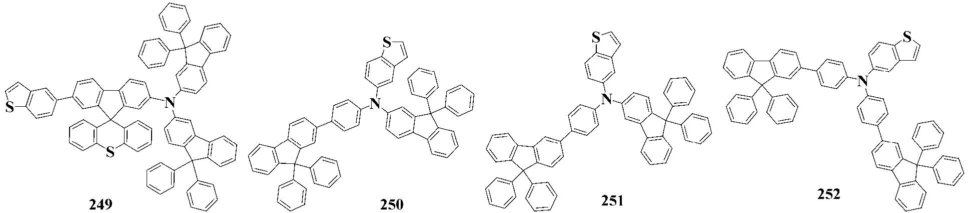

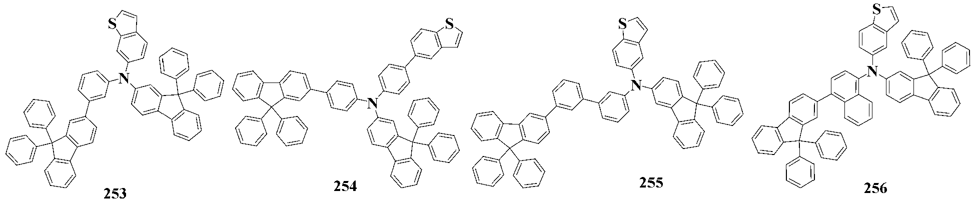

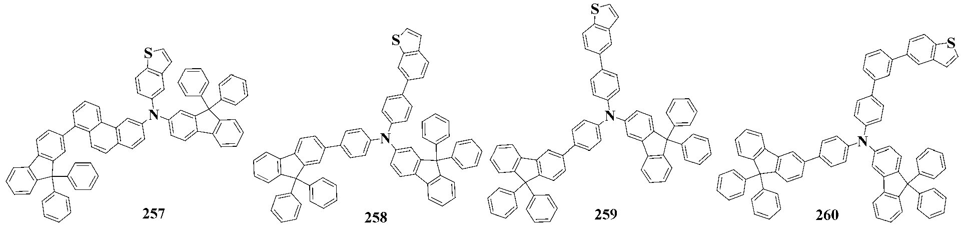

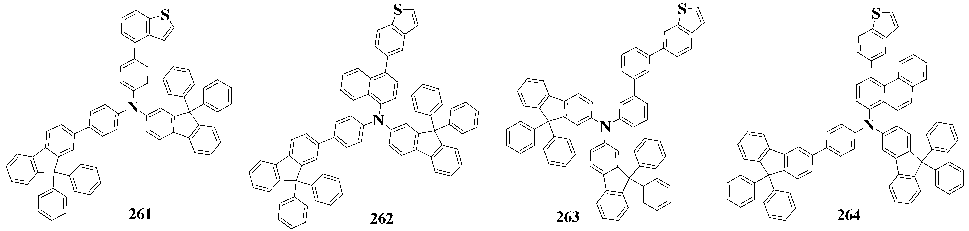

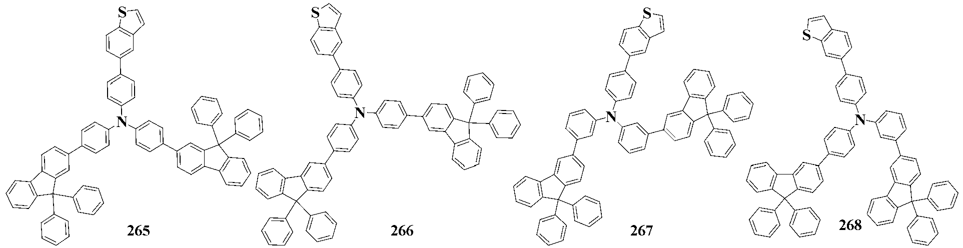

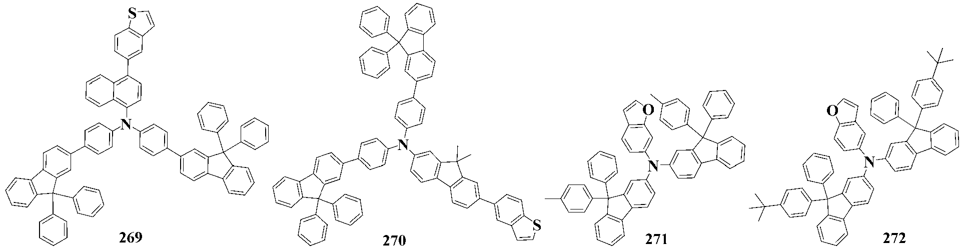

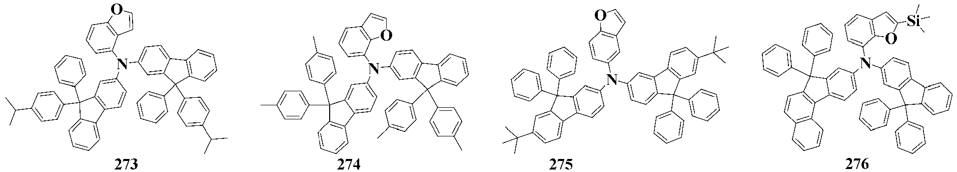

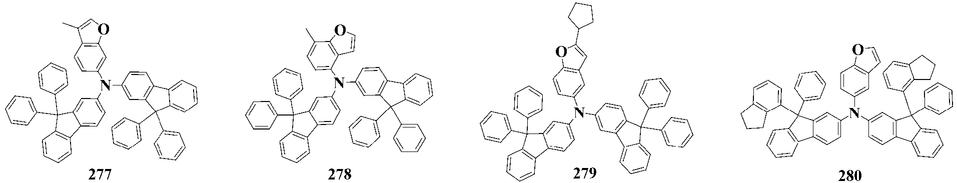

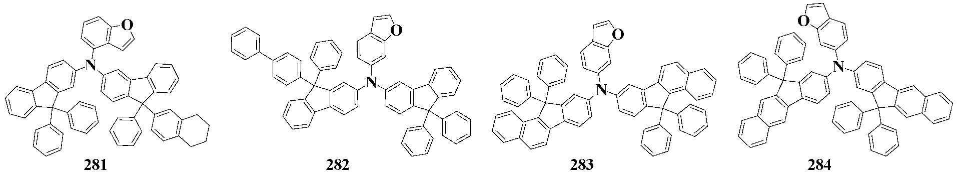

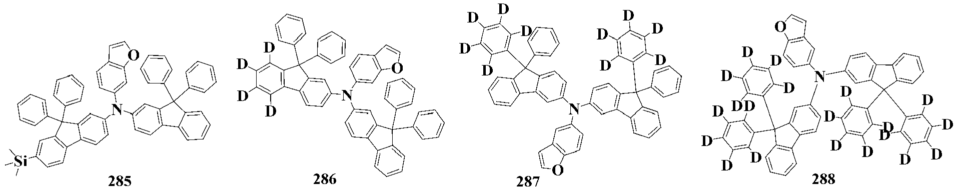

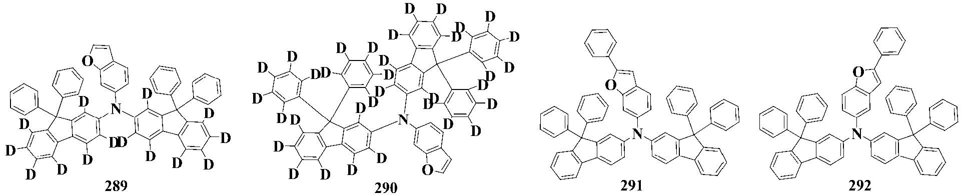

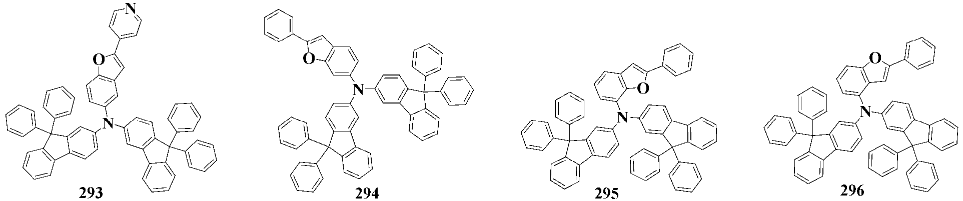

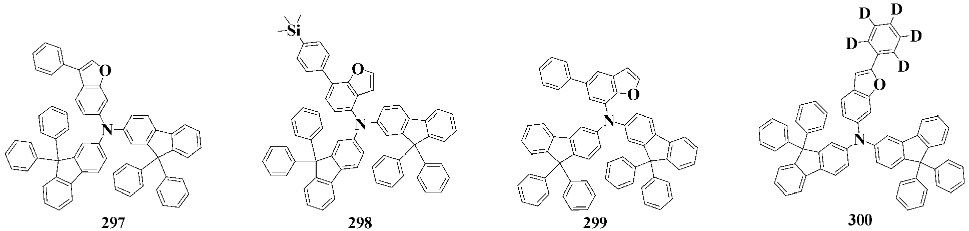

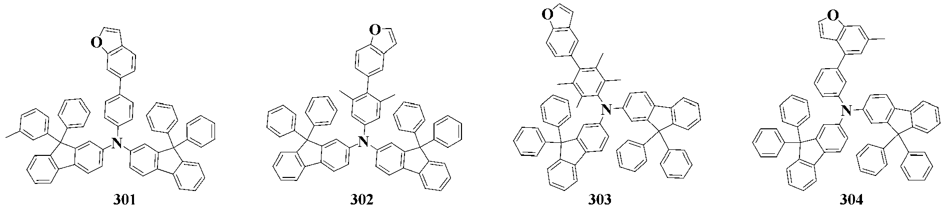

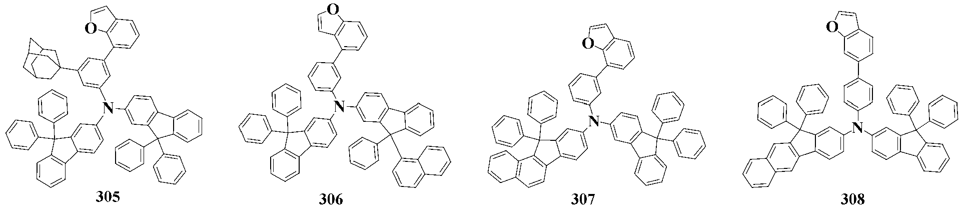

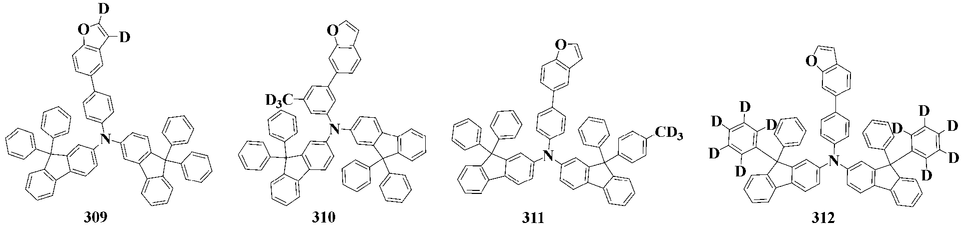

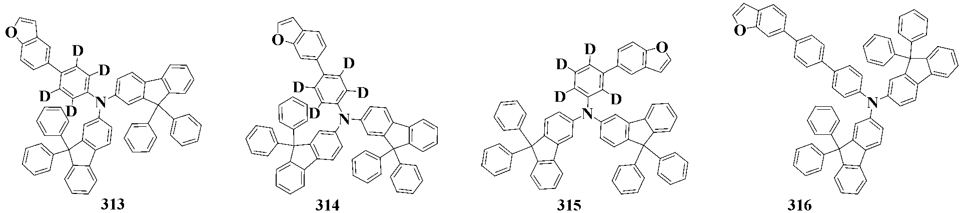

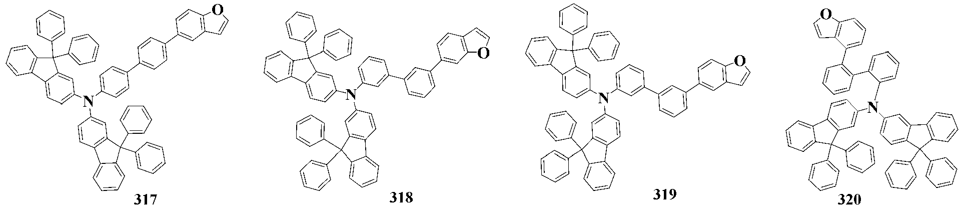

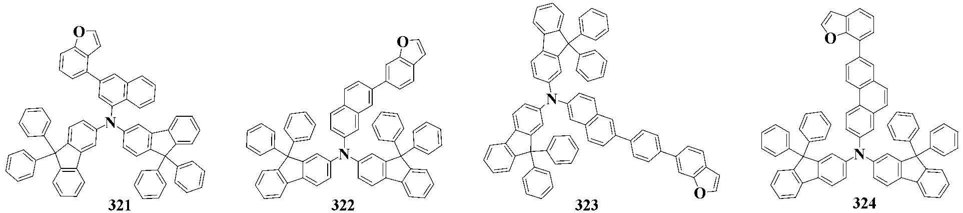

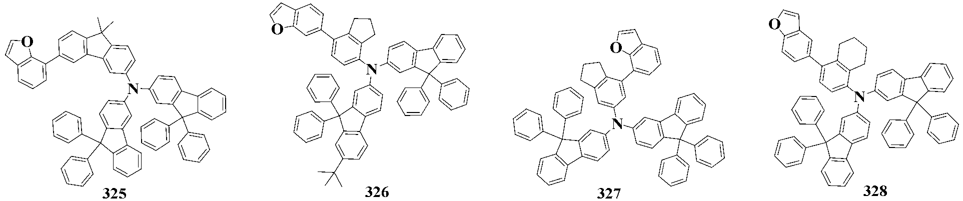

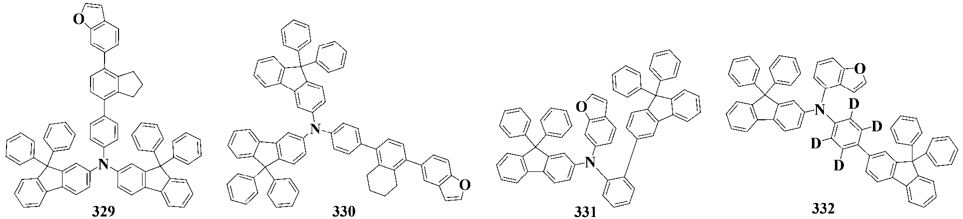

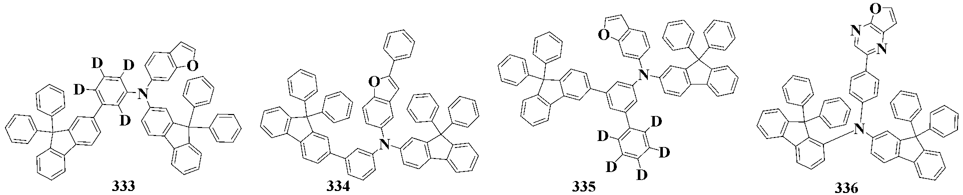

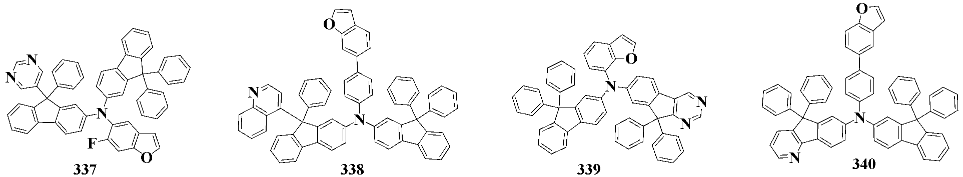

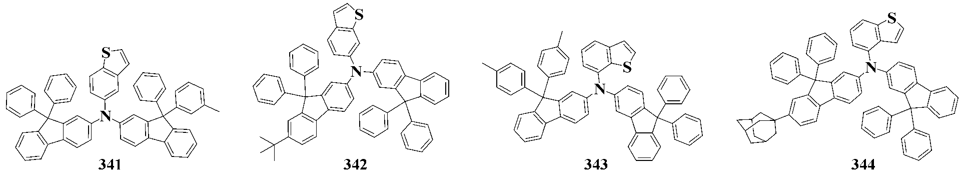

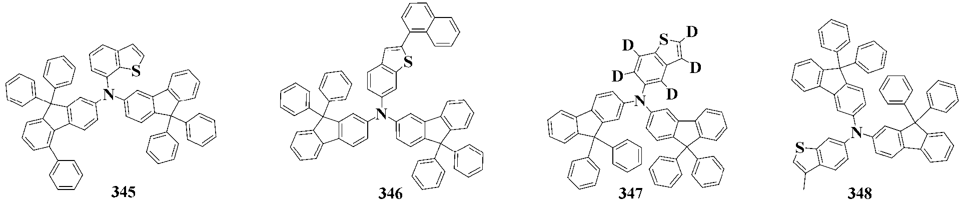

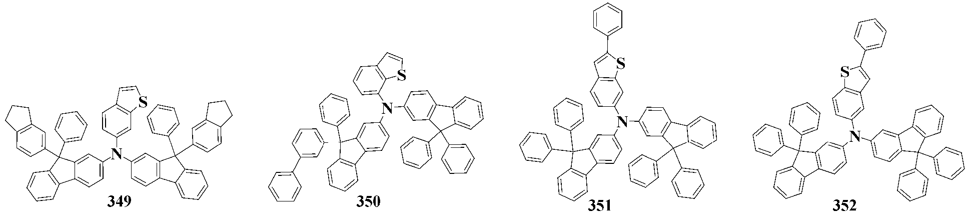

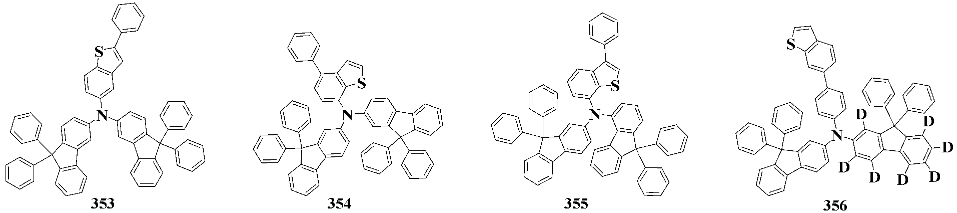

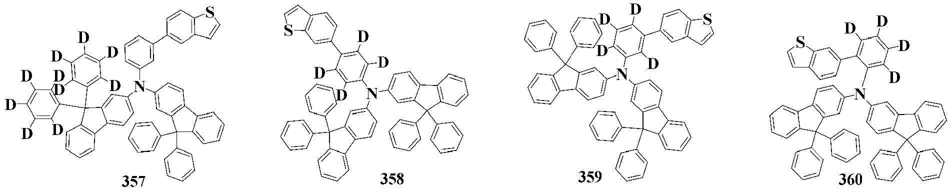

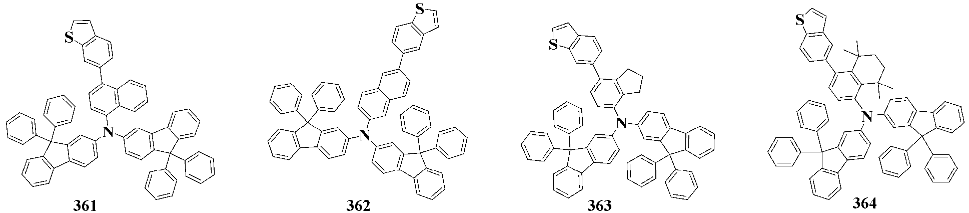

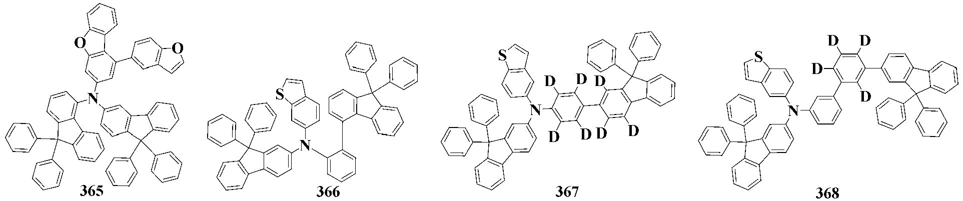

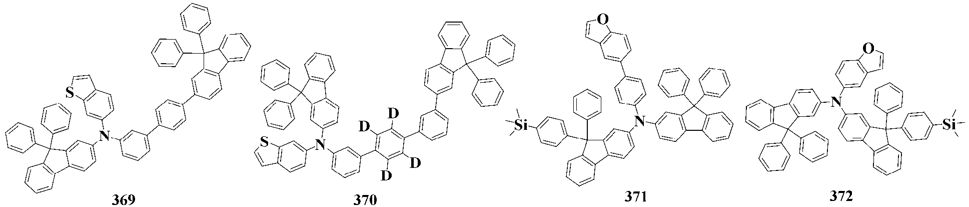

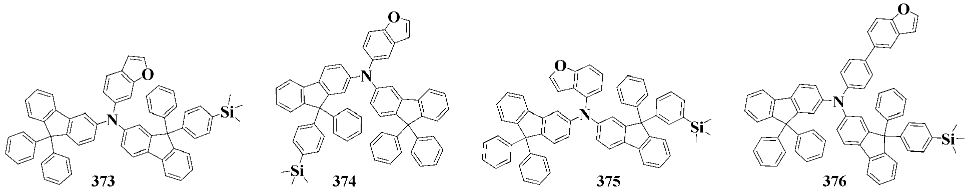

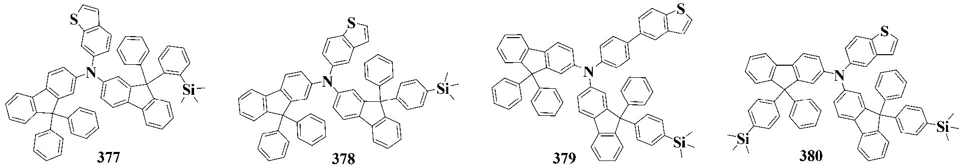

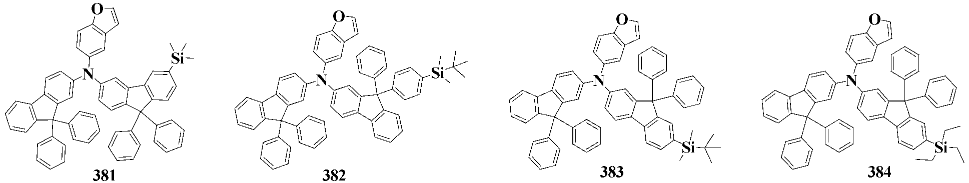

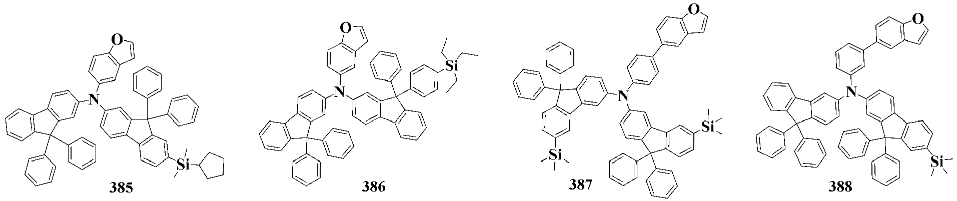

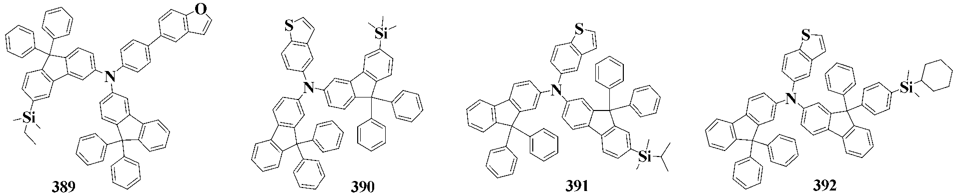

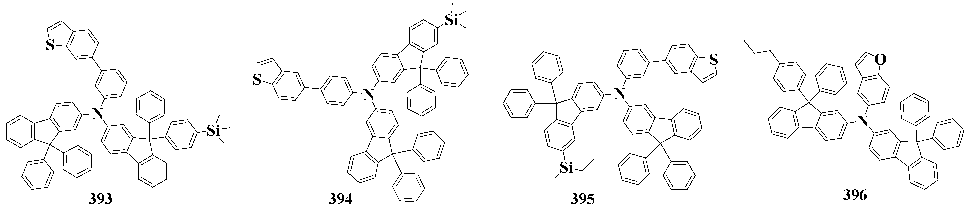

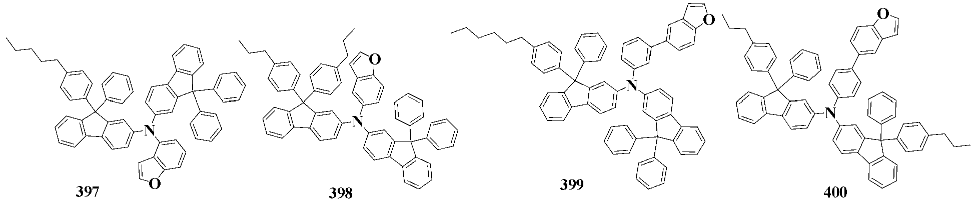

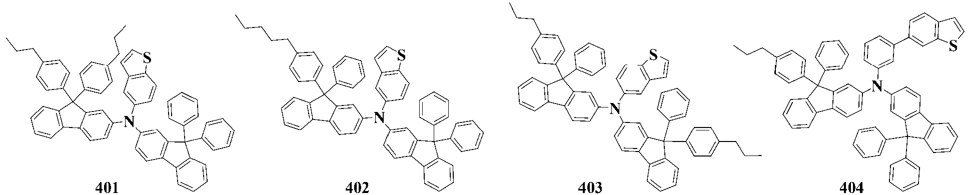

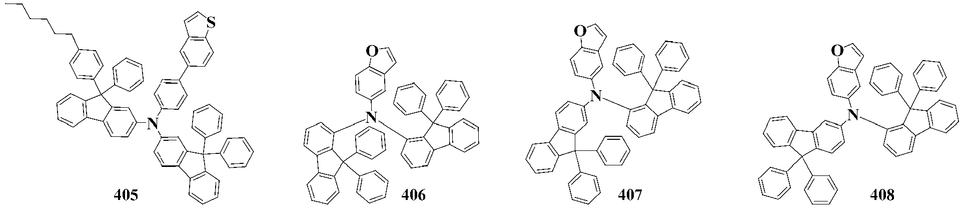

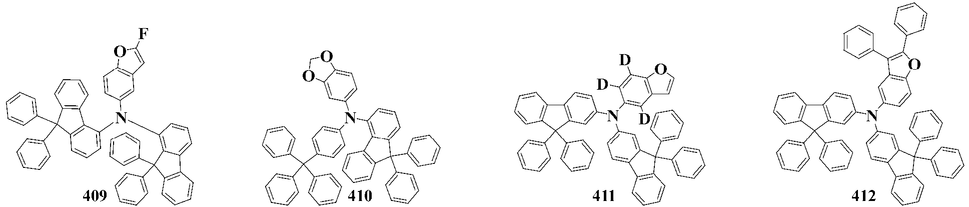

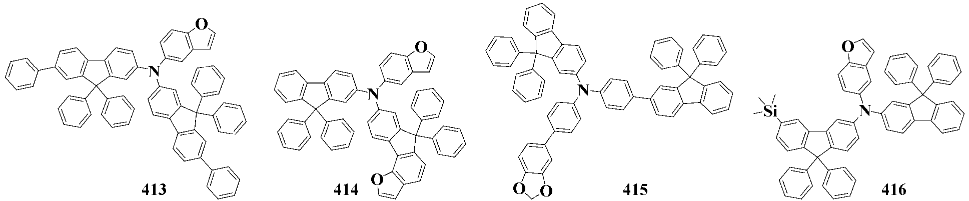

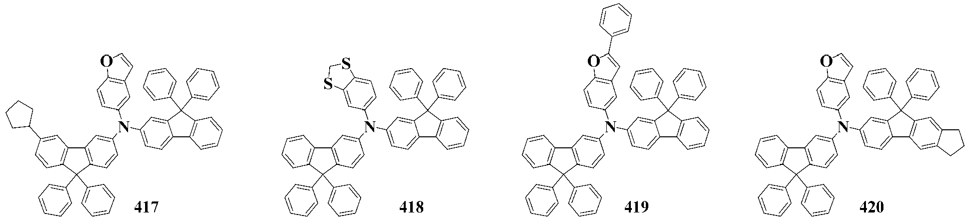

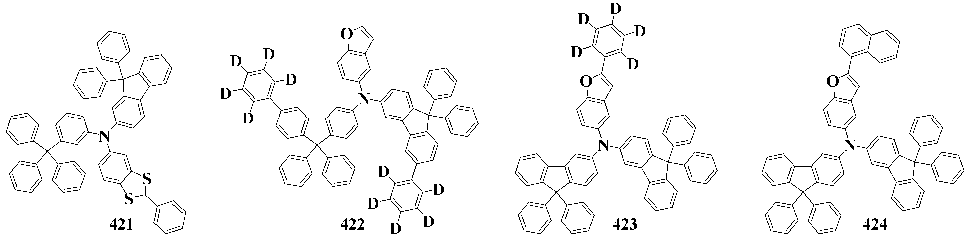

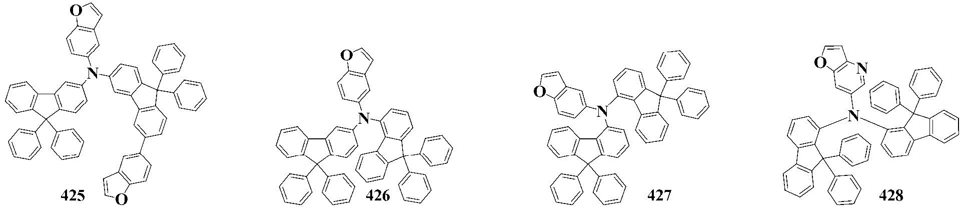

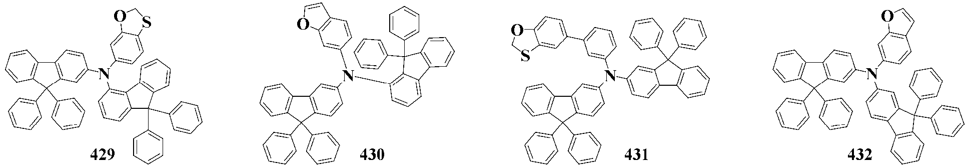

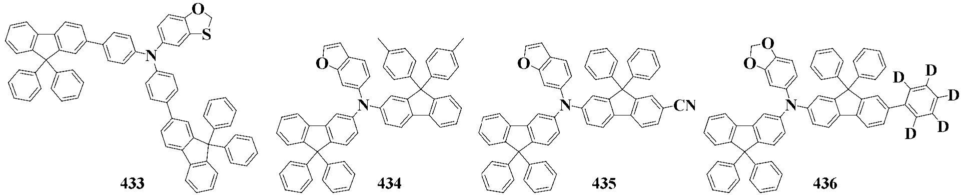

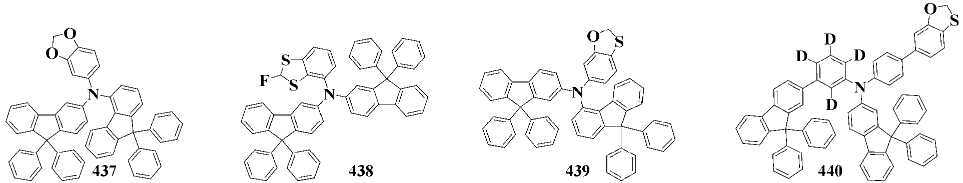

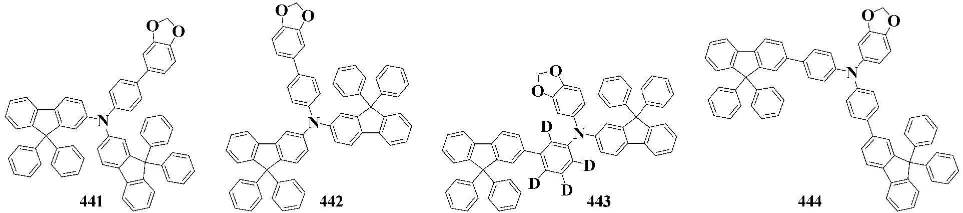

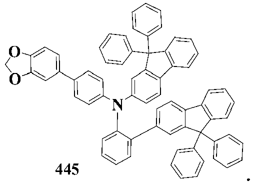

Some specific structures of the triarylamine compound represented by Formula 1 of the present disclosure are exemplified above. However, the present disclosure is not limited to these chemical structures, and any group based on the structure represented by Formula 1 and having the substituent defined above shall be included therein.

The present disclosure further provides an organic electroluminescent device. The organic electroluminescent device includes an anode, a cathode and an organic layer, wherein the organic layer is disposed between the anode and the cathode or is disposed on an outer side of at least one of the anode and the cathode, and the organic layer includes any one or a combination of at least two of the triarylamine compound described in the present disclosure.

The organic layer in the present disclosure includes a hole transport region, a light-emitting layer, an electron transport region and a capping layer. The hole transport region includes functional layers such as a hole injection layer, a hole transport layer, and an electron blocking layer. The electron transport region includes functional layers such as a hole blocking layer, an electron transport layer, and an electron injection layer. The organic functional layers may be correspondingly increased or decreased according to actual requirements.

The organic layer in the present disclosure may have a monolayer structure and a multi-layer structure. The monolayer structure includes a single layer including a single material or a single layer including multiple materials; the multi-layer structure includes multiple layers including multiple materials. For example, the electron transport layer may further have a first electron transport layer and a second electron transport layer.

Preferably, the organic layer is disposed between the anode and the cathode, the organic layer includes a hole transport region, and the hole transport region includes any one or a combination of at least two of the triarylamine compound described in the present disclosure.

Preferably, the hole transport region includes a hole transport layer, and the hole transport layer includes any one or a combination of at least two of the triarylamine compound described in the present disclosure.

Preferably, the hole transport layer includes a first hole transport layer, a second hole transport layer, and a third hole transport layer. The first hole transport layer is disposed between the anode and the light-emitting layer, the second hole transport layer is disposed between the first hole transport layer and the light-emitting layer, the third hole transport layer is disposed between the second hole transport layer and the light-emitting layer, and at least one layer of the first hole transport layer, the second hole transport layer or the third hole transport layer includes any one or a combination of at least two of the triarylamine compound described in the present disclosure.

Preferably, the hole transport layer includes a first hole transport layer and a second hole transport layer. The first hole transport layer is disposed between the anode and the light-emitting layer, the second hole transport layer is disposed between the first hole transport layer and the light-emitting layer, and the first hole transport layer includes any one or a combination of at least two of the triarylamine compound described in the present disclosure.

Preferably, the hole transport layer includes a first hole transport layer and a second hole transport layer. The first hole transport layer is disposed between the anode and the light-emitting layer, the second hole transport layer is disposed between the first hole transport layer and the light-emitting layer, and the second hole transport layer includes any one or a combination of at least two of the triarylamine compound described in the present disclosure.

Preferably, the hole transport layer includes a first hole transport layer, a second hole transport layer, and a third hole transport layer. The first hole transport layer is disposed between the anode and the light-emitting layer, the second hole transport layer is disposed between the first hole transport layer and the light-emitting layer, the third hole transport layer is disposed between the second hole transport layer and the light-emitting layer, and the third hole transport layer includes any one or a combination of at least two of the triarylamine compound described in the present disclosure.

Preferably, the hole transport layer includes a first hole transport layer and a second hole transport layer. The first hole transport layer is disposed between the anode and the light-emitting layer, the second hole transport layer is disposed between the first hole transport layer and the light-emitting layer, and both the first hole transport layer and the second hole transport layer include any one or a combination of at least two of the triarylamine compound described in the present disclosure.

The present disclosure does not particularly limit the material of each film in the organic electroluminescent device, and the substances known in the art can be used. The above-mentioned organic functional layers of the organic electroluminescent device and the electrodes on both sides of the device are introduced below.

The organic electroluminescent device of the present disclosure is usually formed on a substrate. The substrate, as the connection point between the organic electroluminescent device and an external circuit, is preferably a material with good stability, and commonly used substrate materials include, but are not limited to, glass, resin, silicon, metal foil, and the like.

The material of the anode in the present disclosure is preferably a material having good conductivity and a high work function. The material of the anode may include, but are not limited to, metals, such as vanadium, chromium, copper, zinc and gold, or alloys thereof; metal oxides, such as zinc oxide, indium oxide, indium tin oxide (ITO), and indium zinc oxide (IZO); combinations of metals and oxides, such as ITO-Ag-ITO; and conducting polymers, such as poly(3-methylthiophene), polypyrrole, polyaniline, poly[3,4-(ethylene-1,2-dioxy)thiophene] (PEDT), and the like.

The hole injection material in the present disclosure is preferably a material having a good capability to accept holes. The hole injection material includes, but is not limited to, metal oxides such as silver oxides, vanadium oxides, tungsten oxides, copper oxides and titanium oxides as well as materials such as phthalocyanine compounds, benzidine compounds, and phenazine compounds, for example, copper phthalocyanine (CuPc), titanyl phthalocyanine, N,N'-di-phenyl-N,N'-di-[4-(N,N-di-phenyl-amino)pheny]benzidine (NPNPB), N,N,N',N'-tetrakis(4-methoxy-phenyl)benzidine (MeO-TPD), diquinoxalino[2,3-a:2',3'-c]phenazine (HATNA), 4,4',4"-tris[2-naphthyl(phenyl)amino]triphenylamine (2T-NATA), 2,3,6,7,10,11-hexacyano-1,4,5,8,9,12-hexaazatriphenylene (HAT-CN), 4,4'4"-tris(N,N-diphenylamino)triphenylamine (TDATA), and the like.

The material of the hole transport layer material in the present disclosure is preferably a material having a high hole mobility. In addition to the triarylamine compound provided by the present disclosure, the material of the hole transport layer further includes, but is not limited to, diphenylamine compounds, triphenylamine compounds, fluorene compounds, and carbazole compounds, for example, N,N'-diphenyl-N,N'-(1-naphthalenyl)-1,1'-biphenyl-4,4'-diamine (NPB), N,N'-bis(naphthalen-1-yl)-N,N'-bis(phenyl)-2,2'-dimethylbenzidine (α-NPD), N,N'-diphenyl-N,N'-bis(3-methylphenyl)-1,1'-diphenyl-4,4'-diamine (TPD), 4-[1-[4-[bis(4-methylphenyl)amino]phenyl]cyclohexyl]-N-(3-methylphenyl)-N-(4-methylpheny l)benzenamine (TAPC), and the like.

The material of the light-emitting layer in the present disclosure includes a light-emitting layer host material and a light-emitting layer doped material. The host material may be selected from anthracene derivatives, pyrene derivatives, naphthalene derivatives, pentacene derivatives, phenanthrene derivatives, fluoranthene derivatives, carbazole derivatives, dibenzofuran derivatives, dibenzothiophene derivatives, pyrimidine derivatives, and the like. Specific examples of the host material include, but are not limited to, 4,4'-bis(9-carbazolyl)biphenyl (CBP), 9,10-di(2-naphthyl)anthracene (ADN), 4,4-bis(9-carbazolyl)biphenyl (CPB), 9,9'-(1,3-phenylene)bis-9H-carbazole (mCP), 4,4',4"-tris(carbazol-9-yl)-triphenylamine (TCTA), 9,10-di(1-naphthyl)anthracene (α-ADN), N,N'-di-(1-naphthalenyl)-N,N'-diphenyl-[1,1':4',1":4",1‴-quaterphenyl]-4,4‴-diamine (4PNPB), 1,3,5-tri(9-carbazolyl)benzene (TCP), and the like. The dope material may be selected from fused polycyclic aromatic derivatives, styrylamine derivatives, fused ring amine derivatives, boron-containing compounds, pyrrole derivatives, indole derivatives, carbazole derivatives, heavy metal complexes, rare-earth metal complexes with phosphorescent properties, and the like. Specific examples of the doped material include, but are not limited to, (6-(4-(diphenylamino(phenyl)-N,N-diphenylpyren-1-amine) DPAP-DPPA), 2,5,8,11-tetra-*tert*-butylperylene (TBPe), 4,4'-bis[4-(diphenylamino)styryl]biphenyl (BDAVBi), 4,4'-bis[4-(di-p-tolylamino)styryl]biphenyl (DPAVBi), bis[(2-hydroxyphenyl)-pyridine]beryllium (Bepp₂), bis(4,6-difluorophenylpyridinato-C2,N)picolinatoiridium (FIrpic), tris(2-phenylpyridine)iridium (Ir(ppy)₃), acetylacetonatobis(2-phenylpyridine)iridium (Ir(ppy)₂(acac)), 9,10-bis[N-(p-tolyl)anilino]anthracene (TPA), 4-(dicyanomethylene)-2-methyl-6-(4-dimethylaminostyryl)-4H-pyran (DCM), tris[1-phenylisoquinoline-C2,N]iridium(III) (Ir(piq)₃), bis(1-phenyl-isoquinoline)(acetylacetonato)iridium(III) (Ir(piq)₂(acac)), and the like.

The material of the electron transport layer in the present disclosure is preferably a material having high electron mobility and includes quinolines, imidazoles, o-diazophene derivatives, triazoles, metal complexes, triazines, pyridines, and the like. The quinolines include 8-hydroxyquinolinolato-lithium (LiQ) and the like; the imidazoles include 1,3,5-tris(1-phenyl-1H-benzimidazol-2-yl)benzene (TPBi) and the like; the o-diazophene derivatives include 2,9-(dimethyl)-4,7-biphenyl-1,10-phenanthroline (BCP), 2-(naphthalen-2-yl)-4,7-diphenyl-1,10-phenanthroline (HNBphen), and the like; the triazoles include 3-(biphen-4-yl)-5-(4-tert-butylphenyl)-4-phenyl-4H-1,2,4-triazole (TAZ) and the like; the metal complexes include 8-hydroxyquinoline aluminum (Alq₃) and the like; the triazines include 4,4'-bis(4,6-diphenyl-1,3,5-triazinyl)biphenyl (BTB) and the like; the pyridines include 1,3,5-tris[(3-pyridyl)-phenyl]benzene (TmPyPB) and the like, which is not limited thereto.

The electron injection material in the present disclosure is preferably a material having a good capability to inject electrons and includes metal salts, metal oxides, metals, and the like. The metal salts include lithium fluoride (LiF), 8-hydroxyquinolinolato-lithium (Liq), sodium fluoride (NaF), rubidium fluoride (RbF), cesium fluoride (CsF), magnesium phosphide (MgP), cesium carbonate (Cs₂CO₃), and the like; the metal oxides include lithium oxide (Li₂O), lithium boron oxide (LiBO₂), aluminum oxide (Al₂O₃), vanadium oxide (V₂O₅), and the like; the metals include lithium (Li), cesium (Cs), and the like, which is not limited thereto.

The material of the cathode in the present disclosure is preferably a material having a low work function and includes metals, metal alloys, and the like. The metals include aluminum (Al), silver (Ag), gold (Au), lead (Pb), lithium (Li), magnesium (Mg), and the like, and the metal alloys include magnesium-silver alloy (Mg/Al), lithium-aluminum alloy (Li/Al), calcium/silver (Ca/Ag), and the like, which is not limited thereto.

The material of the capping layer in the present disclosure is preferably a material having excellent light extraction performance and includes metal compounds, aromatic amine derivatives, carbazole derivatives, and the like. The metal complexes include tris(8-hydroxyquinolinato)aluminum (Alq₃) and the like, the aromatic amine derivatives include N,N'-bis(naphthalen-1-yl)-N,N'-bis(phenyl)-2,2'-dimethylbenzidine (NPD) and the like, and the carbazole derivatives include 4,4'-bis(9-carbazolyl)biphenyl (CBP) and the like, which is not limited thereto.

The thickness of each organic layer of the organic electroluminescent device is not particularly limited in the present disclosure, and the thickness commonly used in the art can be used.

The preparation method of each film of the organic electroluminescent device of the present disclosure is not particularly limited, which may be, but is not limited to, a vacuum evaporation method, a vapor deposition method, a sputtering method, a spin coating method, a spraying method, a silk-screen printing method, and a laser transfer printing method.

The organic light-emitting device of the present disclosure may be widely applied to the fields of panel display, lighting sources, flexible OLEDs, electronic paper, organic solar cells, organic photoreceptors or organic thin-film transistors, signs, traffic lights, and the like, which is not limited thereto.

### Synthesis Example

The method for preparing the triarylamine compound represented by Formula 1 of the present disclosure is not particularly limited, and conventional preparation methods well known to those skilled in the art can be used. For example, the triarylamine compound can be prepared using the synthetic route shown below, but the present disclosure is not limited thereto. 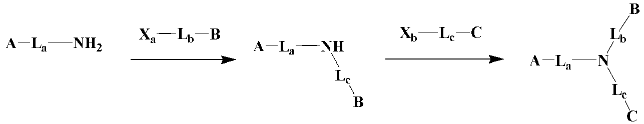 wherein Xₐ and X_{b} are independently selected from any one of I, Br or Cl.

Raw materials and reagents: the raw materials or reagents used in the following synthesis examples are not particularly limited in the present disclosure and may be commercially available products or those prepared by preparation methods well-known to those skilled in the art.

Instruments: the mass spectrometer used is G2-Si quadrupole-time-of-flight mass spectrometer produced by WATER CORPORATION, UK, and the element analyzer used is Vario EL cube elemental analyzer produced by ELEMENTAR CO., GERMANY.

### [Synthesis Example 1] Synthesis of Compound 5

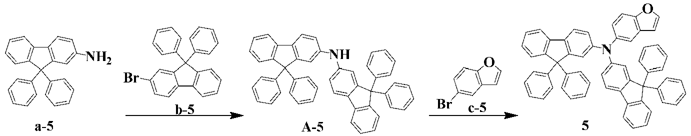

### Preparation of A-5:

Under nitrogen protection, a-5 (20.01 g, 60.00 mmol), b-5 (23.84 g, 60.00 mmol), palladium acetate (0.11 g, 0.48 mmol), tri-tert-butyl phosphine (0.19 g, 0.96 mmol), sodium tert-butoxide (6.92 g, 72.00 mmol) and toluene (600 ml) were added to a reaction flask, stirred under reflux for 5 hours, and cooled to room temperature, and water was added to the reaction mixture. The reaction mixture was extracted with ethyl acetate. The organic phases were combined and dried with anhydrous magnesium sulfate, the solvent was removed under reduced pressure, and the resulting product was recrystallized with ethyl acetate/petroleum ether (4:1) to give A-5 (32.36 g, 83%) with HPLC purity ≥ 99.83%. Mass spectrometry m/z: 649.2786 (theoretical value: 649.2770).

### Preparation of Compound 5:

Under nitrogen protection, A-5 (19.50 g, 30.00 mmol), c-5 (5.91 g, 30.00 mmol), sodium *tert*-butoxide (3.46 g, 36.00 mmol), tris(dibenzylideneacetone)dipalladium (0.27 g, 0.30 mmol), tri-*tert*-butyl phosphine (0.12 g, 0.60 mmol) and toluene (200 ml) reacted under reflux for 6 hours. After the reaction was completed, the reaction mixture was cooled to room temperature, and water was added to the reaction mixture. The organic phases were separated and dried with anhydrous magnesium sulfate. The solvent was removed under reduced pressure, and the crude product was recrystallized with toluene/ethanol (3:1) to give Compound 5 (16.54 g, 72%) with HPLC purity ≥ 99.96%. Mass spectrometry m/z: 765.3046 (theoretical value: 765.3032). Theoretical element content (%) of C₅₈H₃₉NO: C, 90.95; H, 5.13; N, 1.83. Measured element content (%): C, 90.92; H, 5.16; N, 1.88.

### Synthesis Example 2] Synthesis of Compound 10

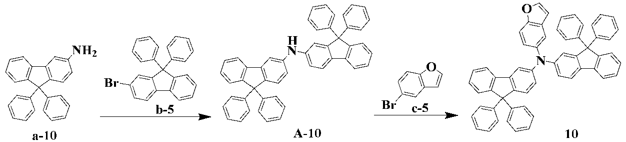

According to the preparation method in Synthetic Example 1, equimolar a-5 was replaced with equimolar a-10 to give Compound 10 (16.77 g, with a yield of 73%) with HPLC purity ≥ 99.97%. Mass spectrometry m/z: 765.3023 (theoretical value: 765.3032). Theoretical element content (%) of C₅₈H₃₉NO: C, 90.95; H, 5.13; N, 1.83. Measured element content (%): C, 90.92; H, 5.16; N, 1.85.

### [Synthesis Example 3] Synthesis of Compound 13

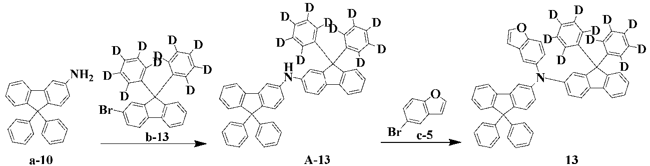

According to the preparation method in Synthetic Example 1, equimolar a-5 and b-5 were replaced with equimolar a-10 and b-13 respectively to give Compound 13 (16.53g, with a yield of 71%) with HPLC purity ≥ 99.95%. Mass spectrometry (m/z): 775.3648 (theoretical value: 775.3659). Theoretical element content (%) of C₅₈H₂₉D₁₀NO: C, 89.77; H, 6.36; N, 1.80. Measured element content (%): C, 89.72; H, 6.33; N, 1.85.

### [Synthesis Example 4] Synthesis of Compound 28

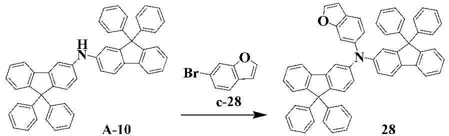

According to the preparation method in Synthetic Example 1, equimolar A-5 and c-5 were replaced with equimolar A-10 and c-28 respectively to give Compound 28 (16.54g, with a yield of 72%) with HPLC purity ≥ 99.96%. Mass spectrometry m/z: 765.3046 (theoretical value: 765.3032). Theoretical element content (%) of C₅₈H₃₉NO: C, 90.95; H, 5.13; N, 1.83. Measured element content (%): C, 90.91; H, 5.17; N, 1.86.

### [Synthesis Example 5] Synthesis of Compound 35

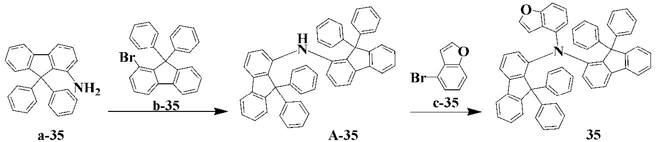

According to the preparation method in Synthetic Example 1, equimolar a-5, b-5 and c-5 were replaced with equimolar a-35, b-35 and c-35 respectively to give Compound 35 (16.77g, with a yield of 73%) with HPLC purity ≥ 99.97%. Mass spectrometry m/z: 765.3048 (theoretical value: 765.3032). Theoretical element content (%) of C₅₈H₃₉NO: C, 90.95; H, 5.13; N, 1.83. Measured element content (%): C, 90.97; H, 5.15; N, 1.80.

### [Synthesis Example 6] Synthesis of Compound 60

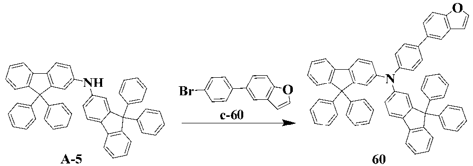

According to the preparation method in Synthetic Example 1, equimolar c-5 was replaced with equimolar c-60 to give Compound 60 (17.18g, with a yield of 68%) with HPLC purity ≥ 99.94%. Mass spectrometry m/z: 841.3356 (theoretical value: 841.3345). Theoretical element content (%) of C₆₄H₄₃NO: C, 91.29; H, 5.15; N, 1.66. Measured element content (%): C, 91.26; H, 5.13; N, 1.68.

### [Synthesis Example 7] Synthesis of Compound 64

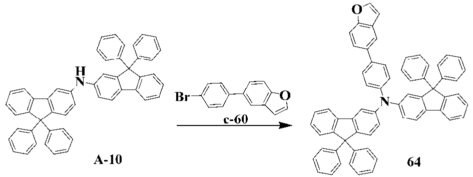

According to the preparation method in Synthetic Example 1, equimolar A-5 and c-5 were replaced with equimolar A-10 and c-60 respectively to give Compound 64 (17.43g, with a yield of 69%) with HPLC purity ≥ 99.95%. Mass spectrometry m/z: 841.3335 (theoretical value: 841.3345). Theoretical element content (%) of C₆₄H₄₃NO: C, 91.29; H, 5.15; N, 1.66. Measured element content (%): C, 91.32; H, 5.18; N, 1.61.

### [Synthesis Example 8] Synthesis of Compound 65

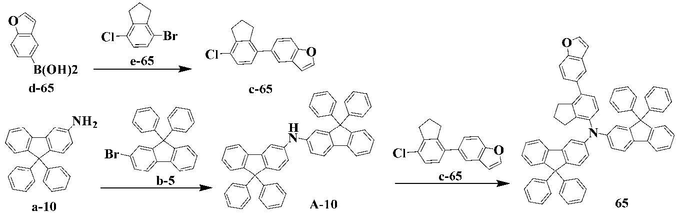

### Preparation of c-65:

Under nitrogen protection, d-65 (11.34 g, 70.00 mmol), e-65 (16.21 g, 70.00 mmol), tetrakis(triphenylphosphine)palladium (0.81 g, 0.70 mmol), potassium carbonate (14.51 g, 105.00 mmol), tetrahydrofuran (250 ml) and water (100 ml) were added to a reaction flask and stirred under reflux for 5 hours. After the reaction was completed, the reaction mixture was cooled to room temperature, the solvent was removed under reduced pressure, and water was added to the reaction mixture. The reaction mixture was extracted with dichloromethane. The organic phases were dried with anhydrous magnesium sulfate, the solvent was removed under reduced pressure, and the resulting product was recrystallized with toluene:n-hexane = 4:1 to give c-65 (15.99 g, 85%). The purity of the solid detected by HPLC was ≥ 99.75%. Mass spectrometry m/z: 268.0669 (theoretical value: 268.0655).

### Preparation ofA-10:

Under nitrogen protection, a-10 (20.01 g, 60.00 mmol), b-5 (23.84 g, 60.00 mmol), palladium acetate (0.11 g, 0.48 mmol), tri-*tert*-butyl phosphine (0.19 g, 0.96 mmol), sodium *tert*-butoxide (6.92 g, 72.00 mmol) and toluene (600 ml) were added to a reaction flask, stirred under reflux for 6 hours and cooled to room temperature, and water was added to the reaction mixture. The reaction mixture was extracted with ethyl acetate. The organic phases were combined and dried with anhydrous magnesium sulfate, the solvent was removed under reduced pressure, and the resulting product was recrystallized with ethyl acetate/petroleum ether (4:1) to give A-10 (31.97 g, 82%) with HPLC purity ≥ 99.86%. Mass spectrometry m/z: 649.2755 (theoretical value: 649.2770).

### Preparation of Compound 65:

Under nitrogen protection, A-10 (19.50 g, 30.00 mmol), c-65 (8.06 g, 30.00 mmol), sodium *tert*-butoxide (3.46 g, 36.00 mmol), tris(dibenzylideneacetone)dipalladium (0.27 g, 0.30 mmol), tri-*tert*-butyl phosphine (0.12 g, 0.60 mmol) and toluene (200 ml) were added to a reaction flask and reacted under reflux for 8 hours. After the reaction was completed, the reaction mixture was cooled to room temperature, and water was added to the reaction mixture. The organic phases were separated and dried with anhydrous magnesium sulfate, the solvent was removed under reduced pressure, and the crude product was recrystallized with toluene/ethanol (3:1) to give Compound 65 (17.73g, with a yield of 67%) with HPLC purity ≥ 99.93%. Mass spectrometry m/z: 881.3669 (theoretical value: 881.3658). Theoretical element content (%) of C₆₇H₄₇NO: C, 91.23; H, 5.37; N, 1.59. Measured element content (%): C, 91.27; H, 5.40; N, 1.56.

### [Synthesis Example 9] Synthesis of Compound 66

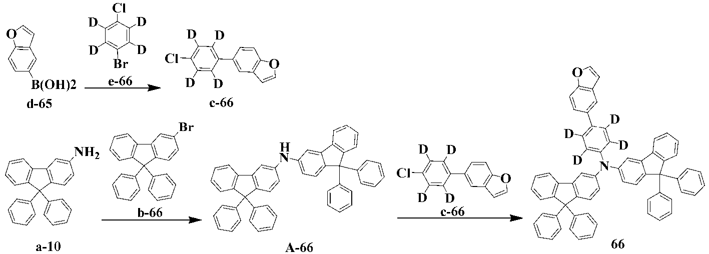

According to the preparation method in Synthetic Example 8, equimolar e-65, b-5 and c-65 were replaced with equimolar e-66, b-66 and c-66 respectively to give Compound 66 (17.26 g, with a yield of 68%) with HPLC purity ≥ 99.94%. Mass spectrometry m/z: 845.3583 (theoretical value: 845.3596). Theoretical element content (%) of C₆₄H₃₉D₄NO: C, 90.85; H, 5.60; N, 1.66. Measured element content (%): C, 90.82; H, 5.64; N, 1.61.

### [Synthesis Example 10] Synthesis of Compound 67

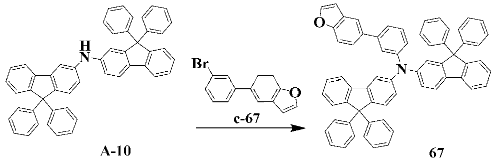

According to the preparation method in Synthetic Example 1, equimolar A-5 and c-5 were replaced with equimolar A-10 and c-67 respectively to give Compound 67 (17.43 g, with a yield of 69%) with HPLC purity ≥ 99.95%. Mass spectrometry m/z: 841.3354 (theoretical value: 841.3345). Theoretical element content (%) of C₆₄H₄₃NO: C, 91.29; H, 5.15; N, 1.66. Measured element content (%): C, 91.25; H, 5.12; N, 1.68.

### [Synthesis Example 11] Synthesis of Compound 98

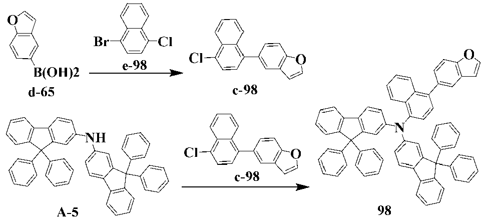

According to the preparation method in Synthetic Example 8, equimolar e-65, A-10 and c-65 were replaced with equimolar e-98, A-5 and c-98 respectively to give Compound 98 (17.93 g, with a yield of 67%) with HPLC purity ≥ 99.93%. Mass spectrometry m/z: 891.3516 (theoretical value: 891.3501). Theoretical element content (%) of C₆₈H₄₅NO: C, 91.55; H, 5.08; N, 1.57. Measured element content (%): C, 91.53; H, 5.11; N, 1.52.

### [Synthesis Example 12] Synthesis of Compound 103

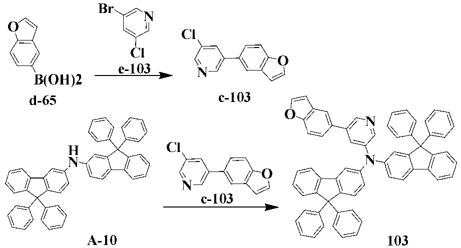

According to the preparation method in Synthetic Example 8, equimolar e-65 and c-65 were replaced with equimolar e-103 and c-103 respectively to give Compound 103 (17.20 g, with a yield of 68%) with HPLC purity ≥ 99.94%. Mass spectrometry m/z: 842.3281 (theoretical value: 842.3297). Theoretical element content (%) of C₆₃H₄₂N₂O: C, 89.76; H, 5.02; N, 3.32. Measured element content (%): C, 89.74; H, 5.06; N, 3.35.

### [Synthesis Example 13] Synthesis of Compound 108

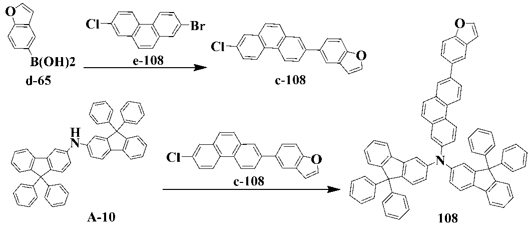

According to the preparation method in Synthetic Example 8, equimolar e-65 and c-65 were replaced with equimolar e-108 and c-108 respectively to give Compound 108 (18.37 g, with a yield of 65%) with HPLC purity ≥ 99.92%. Mass spectrometry m/z: 941.3669 (theoretical value: 941.3658). Theoretical element content (%) of C₇₂H₄₇NO: C, 91.79; H, 5.03; N, 1.49. Measured element content (%): C, 91.75; H, 5.05; N, 1.46.

### [Synthesis Example 14] Synthesis of Compound 112

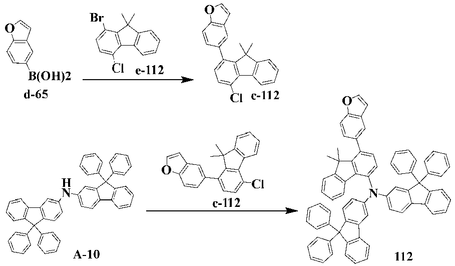

According to the preparation method in Synthetic Example 8, equimolar e-65 and c-65 were replaced with equimolar e-112 and c-112 respectively to give Compound 112 (18.40 g, with a yield of 64%) with HPLC purity ≥ 99.91%. Mass spectrometry m/z: 957.3983 (theoretical value: 957.3971). Theoretical element content (%) of C₇₃H₅₁NO: C, 91.50; H, 5.36; N, 1.46. Measured element content (%): C, 91.54; H, 5.34; N, 1.48.

### [Synthesis Example 15] Synthesis of Compound 116

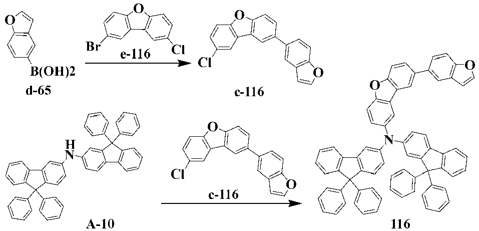

According to the preparation method in Synthetic Example 8, equimolar e-65 and c-65 were replaced with equimolar e-116 and c-116 respectively to give Compound 116 (18.46 g, with a yield of 66%) with HPLC purity ≥ 99.93%. Mass spectrometry m/z: 931.3442 (theoretical value: 931.3450). Theoretical element content (%) of C₇₀H₄₅NO₂: C, 90.20; H, 4.87; N, 1.50. Measured element content (%): C, 90.25; H, 4.85; N, 1.48.

### [Synthesis Example 16] Synthesis of Compound 123

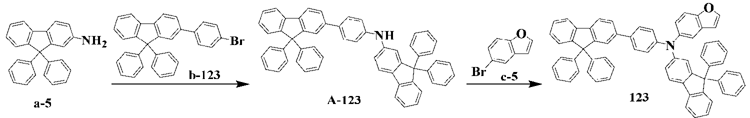

According to the preparation method in Synthetic Example 1, equimolar b-5 was replaced with equimolar b-123 to give Compound 123 (17.18 g, with a yield of 68%) with HPLC purity ≥ 99.95%. Mass spectrometry m/z: 841.3359 (theoretical value: 841.3345). Theoretical element content (%) of C₆₄H₄₃NO: C, 91.29; H, 5.15; N, 1.66. Measured element content (%): C, 91.32; H, 5.17; N, 1.64.

### [Synthesis Example 17] Synthesis of Compound 125

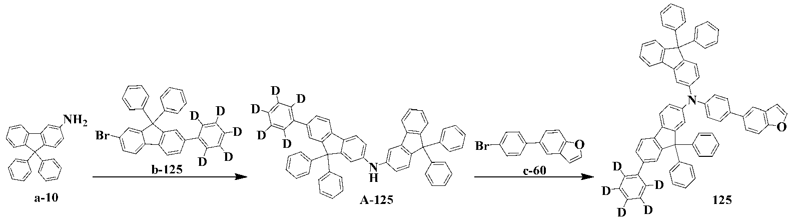

According to the preparation method in Synthetic Example 1, equimolar a-5, b-5 and c-5 were replaced with equimolar a-10, b-125 and c-60 respectively to give Compound 125 (18.28 g, with a yield of 66%) with HPLC purity ≥ 99.93%. Mass spectrometry m/z: 922.3984 (theoretical value: 922.3971). Theoretical element content (%) of C₇₀H₄₂D₅NO: C, 91.07; H, 5.68; N, 1.52. Measured element content (%): C, 91.05; H, 5.64; N, 1.55.

### [Synthesis Example 18] Synthesis of Compound 142

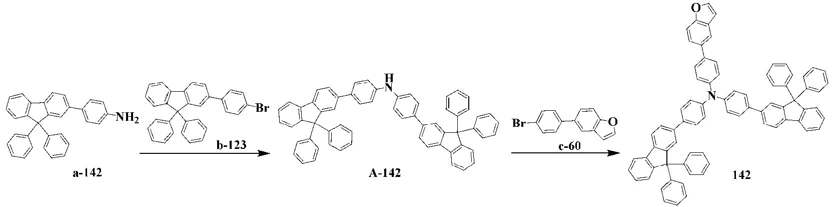

According to the preparation method in Synthetic Example 1, equimolar a-5, b-5 and c-5 were replaced with equimolar a-142, b-123 and c-60 respectively to give Compound 142 (19.09 g, with a yield of 64%) with HPLC purity ≥ 99.91%. Mass spectrometry m/z: 993.3960 (theoretical value: 993.3971). Theoretical element content (%) of C₇₆H₅₁NO: C, 91.81; H, 5.17; N, 1.41. Measured element content (%): C, 91.85; H, 5.12; N, 1.43.

### [Synthesis Example 19] Synthesis of Compound 160

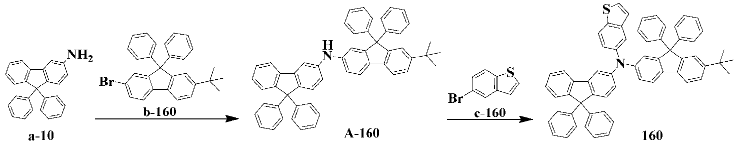

According to the preparation method in Synthetic Example 1, equimolar a-5, b-5 and c-5 were replaced with equimolar a-10, b-160 and c-160 respectively to give Compound 160 (17.35 g, with a yield of 69%) with HPLC purity ≥ 99.95%. Mass spectrometry m/z: 837.3413 (theoretical value: 837.3429). Theoretical element content (%) of C₆₂H₄₇NS: C, 88.85; H, 5.65; N, 1.67. Measured element content (%): C, 88.81; H, 5.67; N, 1.64.

### [Synthesis Example 20] Synthesis of Compound 176

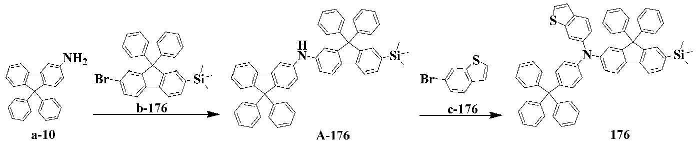

According to the preparation method in Synthetic Example 1, equimolar a-5, b-5 and c-5 were replaced with equimolar a-10, b-176 and c-176 respectively to give Compound 176 (17.43 g, with a yield of 68%) with HPLC purity ≥ 99.94%. Mass spectrometry m/z: 853.3185 (theoretical value: 853.3198). Theoretical element content (%) of C₆₁H₄₇NSSi: C, 85.77; H, 5.55; N, 1.64. Measured element content (%): C, 85.75; H, 5.58; N, 1.67.

### [Synthesis Example 21] Synthesis of Compound 196

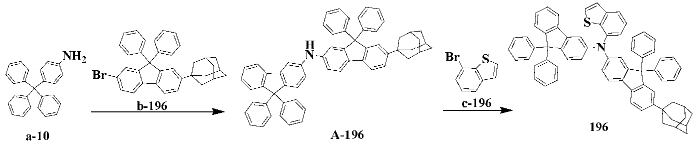

According to the preparation method in Synthetic Example 1, equimolar a-5, b-5 and c-5 were replaced with equimolar a-10, b-196 and c-196 respectively to give Compound 196 (18.14 g, with a yield of 66%) with HPLC purity ≥ 99.93%. Mass spectrometry m/z: 915.3885 (theoretical value: 915.3899). Theoretical element content (%) of C₆₈H₅₃NS: C, 89.14; H, 5.83; N, 1.53. Measured element content (%): C, 89.12; H, 5.86; N, 1.57.

### [Synthesis Example 22] Synthesis of Compound 205

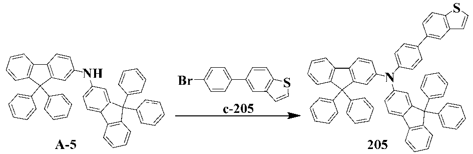

According to the preparation method in Synthetic Example 1, equimolar c-5 was replaced with equimolar c-205 to give Compound 205 (17.76 g, with a yield of 69%) with HPLC purity ≥ 99.95%. Mass spectrometry m/z: 857.3131 (theoretical value: 857.3116). Theoretical element content (%) of C₆₄H₄₃NS: C, 89.58; H, 5.05; N, 1.63. Measured element content (%): C, 89.54; H, 5.07; N, 1.66.

### [Synthesis Example 23] Synthesis of Compound 251

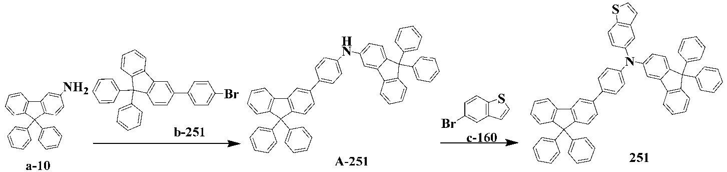

According to the preparation method in Synthetic Example 1, equimolar a-5, b-5 and c-5 were replaced with equimolar a-10, b-251 and c-160 respectively to give Compound 251 (17.51 g, with a yield of 68%) with HPLC purity ≥ 99.94%. Mass spectrometry m/z: 857.3105 (theoretical value: 857.3116). Theoretical element content (%) of C₆₄H₄₃NS: C, 89.58; H, 5.05; N, 1.63. Measured element content (%): C, 89.56; H, 5.09; N, 1.60.

### [Synthesis Example 24] Synthesis of Compound 263

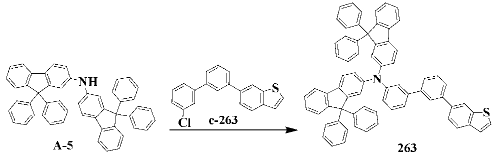

According to the preparation method in Synthetic Example 1, equimolar c-5 was replaced with equimolar c-263 to give Compound 263 (18.50 g, with a yield of 66%) with HPLC purity ≥ 99.92%. Mass spectrometry m/z: 933.3415 (theoretical value: 933.3429). Theoretical element content (%) of C₇₀H₄₇NS: C, 90.00; H, 5.07; N, 1.50. Measured element content (%): C, 90.03; H, 5.05; N, 1.53.

### [Synthesis Example 25] Synthesis of Compound 421

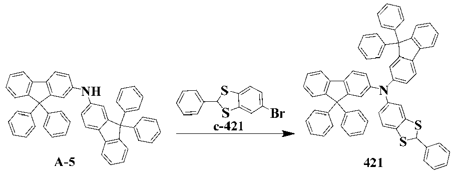

According to the preparation method in Synthetic Example 1, equimolar c-5 was replaced with equimolar c-421 to give Compound 421 (17.91 g, with a yield of 68%) with HPLC purity ≥ 99.94%. Mass spectrometry m/z: 877.2821 (theoretical value: 877.2837). Theoretical element content (%) of C₆₃H₄₃NS₂: C, 86.17; H, 4.94; N, 1.60. Measured element content (%): C, 86.13; H, 4.97; N, 1.62.

### [Synthesis Example 26] Synthesis of Compound 429

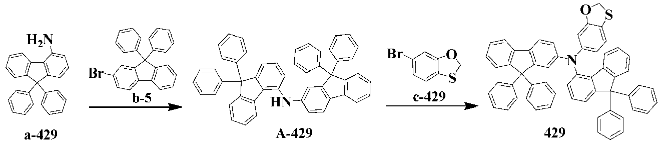

According to the preparation method in Synthetic Example 1, equimolar a-5 and c-5 were replaced with equimolar a-429 and c-429 respectively to give Compound 429 (16.74 g, with a yield of 71%) with HPLC purity ≥ 99.96%. Mass spectrometry m/z: 785.2764 (theoretical value: 785.2752). Theoretical element content (%) of C₅₇H₃₉NOS: C, 87.10; H, 5.00; N, 1.78. Measured element content (%): C, 87.14; H, 5.02; N, 1.74.

### [Synthesis Example 27] Synthesis of Compound 442

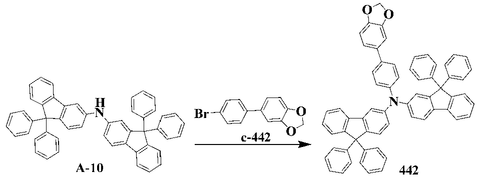

According to the preparation method in Synthetic Example 1, equimolar A-5 and c-5 were replaced with equimolar A-10 and c-442 respectively to give Compound 442 (18.53 g, with a yield of 73%) with HPLC purity ≥ 99.97%. Mass spectrometry m/z: 845.3283 (theoretical value: 845.3294). Theoretical element content (%) of C₆₃H₄₃NO₂: C, 89.44; H, 5.12; N, 1.66. Measured element content (%): C, 89.45; H, 5.15; N, 1.65.

### [Comparative Example 1] Device Preparation Example: (first hole transport layer)

Comparative Example 1: Firstly, a glass substrate with ITO/Ag/ITO evaporated thereon was washed twice in distilled water, washed with ultrasonic waves for 30 minutes, washed twice in distilled water, and washed with ultrasonic waves for 10 minutes. After washing in distilled water and washing with ultrasonic waves in isopropanol, acetone and methanol solvents in sequence were completed, the glass substrate was dried on a hot plate heated to 120 °C. The dried substrate was transferred to a plasma cleaner, washed for 5 minutes, and then transferred to an evaporator.

HI-1 was evaporated on the washed ITO/Ag/ITO substrate to form a hole injection layer with an evaporation thickness of 15 nm; Ref-1 was evaporated on the hole injection layer to form a first hole transport layer with an evaporation thickness of 25 nm; HT2 was evaporated on the first hole transport layer to form a second hole transport layer with an evaporation thickness of 30 nm; a mixture of BH-1 and BD-1 (at a mass ratio of 95:5) was vacuum-evaporated on the second hole transport layer to form a light-emitting layer with an evaporation thickness of 35 nm; ET-1 and Liq (at a mass ratio of 1:1) were evaporated on the light-emitting layer to form an electron transport layer with an evaporation thickness of 30 nm; LiF was evaporated on the electron transport layer to form an electron injection layer with an evaporation thickness of 1 nm; Mg:Ag (at a mass ratio of 1:9) were evaporated on the electron injection layer to form a cathode with an evaporation thickness of 15 nm; CP-1 was vacuum-evaporated on the cathode to form a capping layer with an evaporation thickness of 55 nm to prepare an organic electroluminescent device (the material structures of the functional layers in the preparation process of the organic electroluminescent device are as follows):

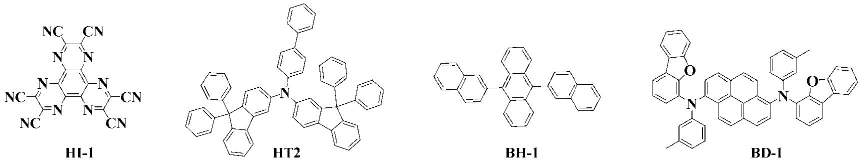

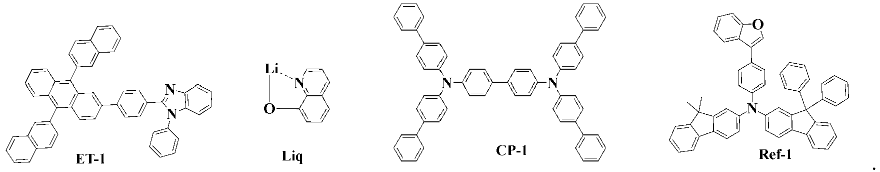

### [Examples 1 to 20]

The material of the first hole transport layer of the organic electroluminescent device was replaced with Compound 5, Compound 10, Compound 13, Compound 28, Compound 60, Compound 64, Compound 65, Compound 66, Compound 98, Compound 108, Compound 112, Compound 123, Compound 125, Compound 142, Compound 160, Compound 176, Compound 205, Compound 251, Compound 421 and Compound 442 of the present disclosure sequentially, and other steps are the same as in Comparative Example 1.

The luminescence efficiency of each organic electroluminescent device was tested using a combined IVL test system composed of test software, a computer, a K2400 digital source meter produced by KEITHLEY, USA, and a PR788 spectral scanning photometer produced by PHOTO RESEARCH, USA. The lifetime was tested using an M6000 OLED lifetime test system produced by MCSCIENCE. The test was conducted in an atmospheric environment and at room temperature. The test results of the luminescence property of the organic electroluminescent devices obtained in Device Comparative Example 1 and Device Examples 1 to 20 in the present disclosure are shown in Table 1.

**Table 1 Luminescence property test data of organic electroluminescent devices**

| Example | First hole transport layer | Efficiency [cd/A] (@ 10 mA/cm²) | Lifetime [T95, h] (@ 10 mA/cm²) |
|---|---|---|---|
| Comparative Example 1 | Ref-1 | 6.76 | 126.3 |
| Example 1 | Compound 5 | 8.06 | 157.7 |
| Example 2 | Compound 10 | 8.02 | 155.1 |
| Example 3 | Compound 13 | 8.35 | 171.9 |
| Example 4 | Compound 28 | 8.07 | 158.2 |
| Example 5 | Compound 60 | 8.31 | 169.8 |
| Example 6 | Compound 64 | 8.24 | 166.3 |
| Example 7 | Compound 65 | 8.14 | 163.9 |
| Example 8 | Compound 66 | 8.47 | 172.4 |
| Example 9 | Compound 98 | 8.12 | 162.6 |
| Example 10 | Compound 108 | 7.69 | 149.5 |
| Example 11 | Compound 112 | 7.71 | 148.9 |
| Example 12 | Compound 123 | 7.94 | 152.4 |
| Example 13 | Compound 125 | 7.87 | 151.8 |
| Example 14 | Compound 142 | 7.83 | 150.4 |
| Example 15 | Compound 160 | 8.03 | 154.6 |
| Example 16 | Compound 176 | 8.19 | 165.5 |
| Example 17 | Compound 205 | 8.27 | 167.4 |
| Example 18 | Compound 251 | 7.75 | 151.1 |
| Example 19 | Compound 421 | 7.55 | 145.3 |
| Example 20 | Compound 442 | 7.64 | 147.2 |

As can be known from the results in Table 1, with the comparison with the device in Comparative Example 1, when the triarylamine compound provided by the present disclosure is used as the material of the first hole transport layer of the organic electroluminescent device, the luminescence efficiency and the lifetime of the device both showed better performance.

### [Comparative Example 2] Device Preparation Example: (second hole transport layer)

Comparative Example 2: Firstly, a glass substrate with ITO/Ag/ITO evaporated thereon was washed twice in distilled water, washed with ultrasonic waves for 30 minutes, washed twice in distilled water, and washed with ultrasonic waves for 10 minutes. After washing in distilled water and washing with ultrasonic waves in isopropanol, acetone and methanol solvents in sequence were completed, the glass substrate was dried on a hot plate heated to 120 °C. The dried substrate was transferred to a plasma cleaner, washed for 5 minutes, and then transferred to an evaporator.

HI-1 was evaporated on the washed ITO/Ag/ITO substrate to form a hole injection layer with an evaporation thickness of 10 nm; HT1 was evaporated on the hole injection layer to form a first hole transport layer with an evaporation thickness of 30 nm; Ref-2 was evaporated on the first hole transport layer to form a second hole transport layer with an evaporation thickness of 35 nm; a mixture of RH-1, RH-2 and RD-1 (at a mass ratio of 49:49:2) was vacuum-evaporated on the second hole transport layer to form a light-emitting layer with an evaporation thickness of 30 nm; ET-1 and Liq (at a mass ratio of 1:1) were evaporated on the light-emitting layer to form an electron transport layer with an evaporation thickness of 35 nm; LiF was evaporated on the electron transport layer to form an electron injection layer with an evaporation thickness of 1 nm; Mg:Ag (at a mass ratio of 1:9) were evaporated on the electron injection layer to form a cathode with an evaporation thickness of 12 nm; CP-1 was vacuum-evaporated on the cathode to form a capping layer with an evaporation thickness of 65 nm to prepare an organic electroluminescent device (the material structures of the functional layers in the preparation process of the organic electroluminescent device are as follows):

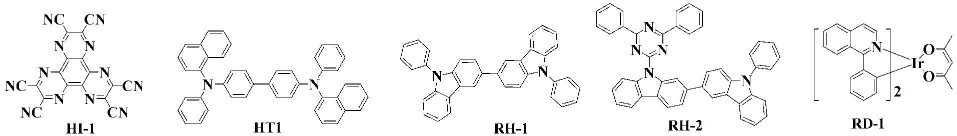

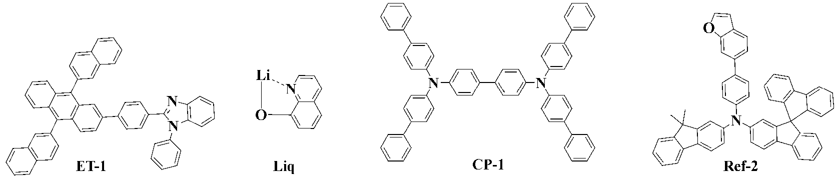

### [Examples 21 to 40]

The material of the second hole transport layer of the organic electroluminescent device was replaced with Compound 10, Compound 13, Compound 28, Compound 35, Compound 64, Compound 65, Compound 66, Compound 67, Compound 98, Compound 103, Compound 112, Compound 116, Compound 125, Compound 160, Compound 176, Compound 196, Compound 205, Compound 251, Compound 263 and Compound 429 of the present disclosure sequentially, and other steps are the same as in Comparative Example 2. The test results of the luminescence property of the organic electroluminescent devices obtained in Device Comparative Example 2 and Device Examples 21 to 40 in the present disclosure are shown in Table 2.

**Table 2 Luminescence property test data of organic electroluminescent devices**

| Example | Second hole transport layer | Efficiency [cd/A] (@ 10 mA/cm²) | Lifetime [T95, h] (@ 10 mA/cm²) |
|---|---|---|---|
| Comparative Example 2 | Ref-2 | 22.25 | 179.7 |
| Example 21 | Compound 10 | 26.47 | 202.6 |
| Example 22 | Compound 13 | 29.14 | 213.5 |
| Example 23 | Compound 28 | 26.63 | 203.2 |
| Example 24 | Compound 35 | 29.92 | 215.4 |
| Example 25 | Compound 64 | 28.17 | 210.7 |
| Example 26 | Compound 65 | 29.95 | 209.6 |
| Example 27 | Compound 66 | 28.63 | 211.2 |
| Example 28 | Compound 67 | 30.24 | 218.3 |
| Example 29 | Compound 98 | 26.16 | 201.3 |
| Example 30 | Compound 103 | 26.89 | 204.8 |
| Example 31 | Compound 112 | 25.78 | 197.4 |
| Example 32 | Compound 116 | 25.94 | 199.2 |
| Example 33 | Compound 125 | 27.32 | 206.5 |
| Example 34 | Compound 160 | 27.18 | 205.1 |
| Example 35 | Compound 176 | 28.86 | 212.7 |
| Example 36 | Compound 196 | 29.35 | 214.8 |
| Example 37 | Compound 205 | 27.44 | 207.6 |
| Example 38 | Compound 251 | 27.79 | 208.5 |
| Example 39 | Compound 263 | 30.16 | 217.6 |
| Example 40 | Compound 429 | 25.43 | 195.9 |

As can be known from the results in Table 2, with the comparison with the device in Comparative Example 2, when the triarylamine compound provided by the present disclosure is used as the material of the second hole transport layer of the organic electroluminescent device, the luminescence efficiency of the device can be improved, and the lifetime of the device can be extended.

### [Comparative Example 3] Device Preparation Example: (third hole transport layer)

Comparative Example 3: Firstly, a glass substrate with ITO/Ag/ITO evaporated thereon was washed twice in distilled water, washed with ultrasonic waves for 30 minutes, washed twice in distilled water, and washed with ultrasonic waves for 10 minutes. After washing in distilled water and washing with ultrasonic waves in isopropanol, acetone and methanol solvents in sequence were completed, the glass substrate was dried on a hot plate heated to 120 °C. The dried substrate was transferred to a plasma cleaner, washed for 5 minutes, and then transferred to an evaporator.

HI-1 was evaporated on the washed ITO/Ag/ITO substrate to form a hole injection layer with an evaporation thickness of 10 nm; HT1 was evaporated on the hole injection layer to form a first hole transport layer with an evaporation thickness of 25 nm; HT2 was evaporated on the first hole transport layer to form a second hole transport layer with an evaporation thickness of 25 nm; Ref-3 was evaporated on the second hole transport layer to form a third hole transport layer with an evaporation thickness of 20 nm; a mixture of GH-1, GH-2 and GD-1 (at a mass ratio of 47:47:6) was vacuum-evaporated on the third hole transport layer to form a light-emitting layer with an evaporation thickness of 40 nm; ET-1 and Liq (at a mass ratio of 1:1) were evaporated on the light-emitting layer to form an electron transport layer with an evaporation thickness of 30 nm; LiF was evaporated on the electron transport layer to form an electron injection layer with an evaporation thickness of 1 nm; Mg:Ag (at a mass ratio of 1:9) were evaporated on the electron injection layer to form a cathode with an evaporation thickness of 15 nm; CP-1 was vacuum-evaporated on the cathode to form a capping layer with an evaporation thickness of 60 nm to prepare an organic electroluminescent device (the material structures of the functional layers in the preparation process of the organic electroluminescent device are as follows):

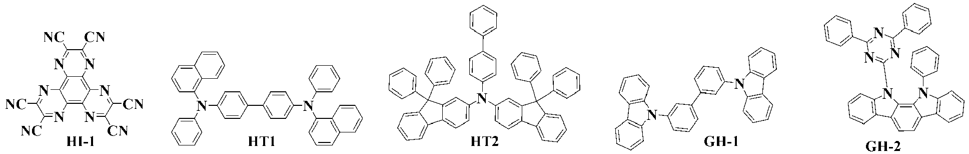

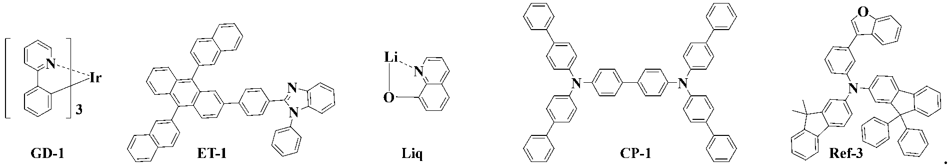

### [Examples 41 to 50]

The material of the third hole transport layer of the organic electroluminescent device was replaced with Compound 13, Compound 35, Compound 66, Compound 67, Compound 103, Compound 116, Compound 125, Compound 196, Compound 263 and Compound 429 of the present disclosure sequentially, and other steps are the same as in Comparative Example 3. The test results of the luminescence property of the organic electroluminescent devices obtained in Device Comparative Example 3 and Device Examples 41 to 50 in the present disclosure are shown in Table 3.

**Table 3 Luminescence property test data of organic electroluminescent devices**

| Example | Third hole transport layer | Efficiency [cd/A] (@ 10 mA/cm²) | Lifetime [T95, h] (@ 10 mA/cm²) |
|---|---|---|---|
| Comparative Example 3 | Ref-3 | 46.36 | 192.6 |
| Example 41 | Compound 13 | 56.24 | 239.5 |
| Example 42 | Compound 35 | 58.39 | 248.8 |
| Example 43 | Compound 66 | 55.83 | 236.8 |
| Example 44 | Compound 67 | 57.92 | 245.6 |
| Example 45 | Compound 103 | 53.74 | 231.3 |
| Example 46 | Compound 116 | 54.56 | 233.4 |
| Example 47 | Compound 125 | 56.62 | 241.9 |
| Example 48 | Compound 196 | 57.39 | 243.7 |
| Example 49 | Compound 263 | 58.21 | 247.2 |
| Example 50 | Compound 429 | 52.73 | 229.6 |

As can be known from the results in Table 3, with the comparison with the device in Comparative Example 3, when the triarylamine compound provided by the present disclosure is used as the material of the third hole transport layer of the organic electroluminescent device, the efficiency and lifetime of the organic electroluminescent device can be significantly improved.

It is to be noted that though the present disclosure has been specifically described through particular embodiments, those skilled in the art can make various improvements and modifications without departing from the principle of the present disclosure, and such improvements and modifications also fall within the scope of the present disclosure.

## Claims

1. A triarylamine compound, represented by a structure shown in Formula 1: 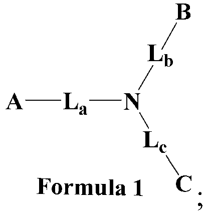 wherein A is selected from Formula 2, B is selected from Formula 3, and C is selected from Formula 3: 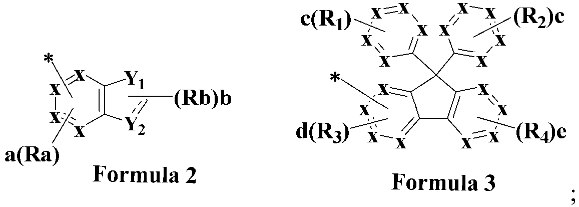
Y₁ is S; Y₂ is CH;
when Y₂ is selected from CH, "--" is selected from a single bond;
Rₐ, R_{b}, R₁, R₂, R₃ and R₄ are independently selected from any one of hydrogen, deuterium, tritium, cyano, halogen, nitro, substituted or unsubstituted C1 to C15 alkyl, a substituted or unsubstituted C3 to C15 alicyclic group, substituted or unsubstituted silyl, substituted or unsubstituted C6 to C30 aryl, substituted or unsubstituted fused cyclyl formed by fusing a C3 to C15 alicyclic ring to a C6 to C30 aromatic ring, substituted or unsubstituted C2 to C30 heteroaryl, substituted or unsubstituted fused cyclyl formed by fusing a C3 to C15 alicyclic ring to a C2 to C30 heteroaromatic ring, and combinations thereof; or two adjacent R₁, two adjacent R₂, two adjacent R₃ or two adjacent R₄ can be linked to each other to form a substituted or unsubstituted ring;
x is identically or differently selected from N or CH; x at a linkage is selected from C;
a is selected from 0, 1, 2 or 3; b is selected from 0, 1 or 2; c is selected from 0, 1, 2, 3, 4 or 5; d is selected from 0, 1, 2 or 3; e is selected from 0, 1, 2, 3 or 4; when two or more Rₐ, R_{b}, R₁, R₂, R₃ and R₄ are present, the two or more Rₐ, R_{b}, R₁, R₂, R₃ and R₄ are identical to or different from each other;
Lₐ is selected from any one of a single bond, substituted or unsubstituted phenylene, substituted or unsubstituted biphenylylene, substituted or unsubstituted terphenylene, substituted or unsubstituted naphthylene, substituted or unsubstituted phenanthrylene, substituted or unsubstituted triphenylenylene, substituted or unsubstituted fluorenylene, substituted or unsubstituted dibenzofurylene, substituted or unsubstituted dibenzothienylene, substituted or unsubstituted carbazolylene, substituted or unsubstituted benzocyclopropylene, substituted or unsubstituted benzocyclobutylene, substituted or unsubstituted benzocyclopentylene, substituted or unsubstituted benzocyclohexylene, substituted or unsubstituted benzocycloheptylene, substituted or unsubstituted benzocyclobutenylene, substituted or unsubstituted benzocyclopentenylene, substituted or unsubstituted benzocyclohexynylene, substituted or unsubstituted benzocycloheptenylene, substituted or unsubstituted pyridylene, substituted or unsubstituted pyrimidinylene, substituted or unsubstituted pyridazinylene, substituted or unsubstituted pyrazinylene, substituted or unsubstituted quinolylene, substituted or unsubstituted isoquinolylene, substituted or unsubstituted quinazolinylene, substituted or unsubstituted quinoxalinylene, and combinations thereof;
L_{b} and L_{c} are independently selected from any one of a single bond, substituted or unsubstituted C6 to C30 arylene, substituted or unsubstituted C2 to C30 heteroarylene, substituted or unsubstituted fused cyclylene formed by fusing a C3 to C15 alicyclic ring to a C6 to C30 aromatic ring, substituted or unsubstituted fused cyclylene formed by fusing a C3 to C15 alicyclic ring to a C2 to C30 heteroaromatic ring, and combinations thereof.

2. The triarylamine compound according to claim 1, wherein Formula 2 is selected from any one of the following groups: 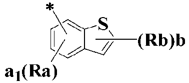 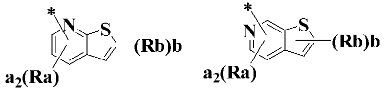 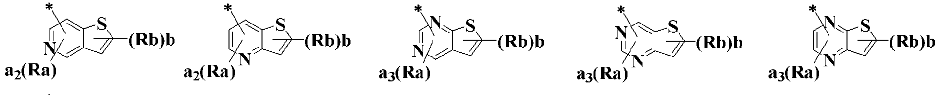 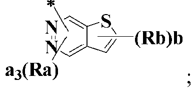 wherein a₁ is selected from 0, 1, 2 or 3; a₂ is selected from 0, 1 or 2; a₃ is selected from 0 or 1; b is selected from 0, 1 or 2; when two or more Rₐ and R_{b} are present, the two or more Rₐ and R_{b} are identical to or different from each other.

3. The triarylamine compound according to claim 1, wherein Rₐ and R_{b} are independently selected from any one of hydrogen, deuterium, tritium, cyano, halogen, nitro, or substituted or unsubstituted following groups: methyl, ethyl, *n*-propyl, isopropyl, *n*-butyl, isobutyl, *tert*-butyl, cyclopropyl, cyclobutyl, cyclopentyl, cyclohexyl, adamantyl, norbornyl, phenyl, biphenyl, terphenyl, naphthyl, anthryl, phenanthryl, triphenylenyl, benzocyclobutyl, benzocyclopentyl, benzocyclohexyl, benzocyclopentenyl, benzocyclohexenyl, fluorenyl, benzofuryl, dibenzofuryl, benzothienyl, dibenzothienyl, benzoxazolyl, benzothiazolyl, benzimidazolyl, indolyl, carbazolyl, pyridyl, pyrimidinyl, pyrazinyl, pyridazinyl, quinolyl, isoquinolyl, quinazolinyl, quinoxalinyl, trimethylsilyl, triethylsilyl, tripropylsilyl, tri-*tert*-butylsilyl, triphenylsilyl and combinations thereof.

4. The triarylamine compound according to claim 1, wherein Formula 3 is selected from any one of the following groups:

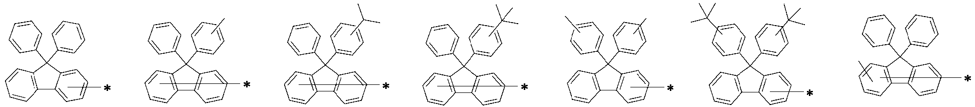

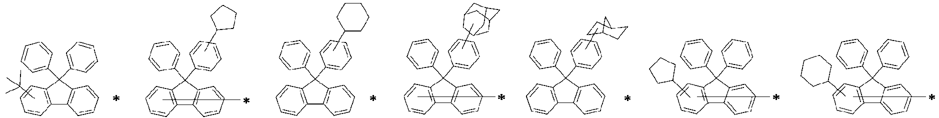

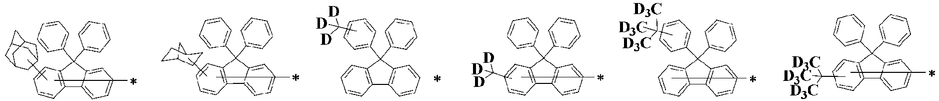

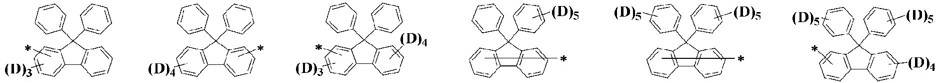

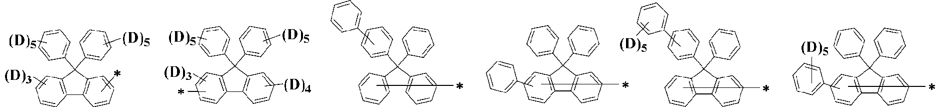

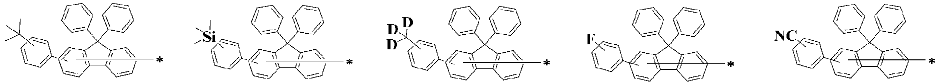

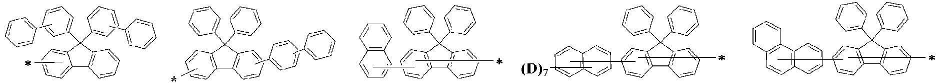

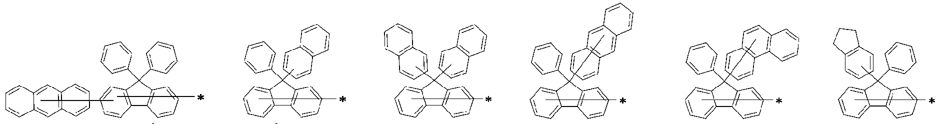

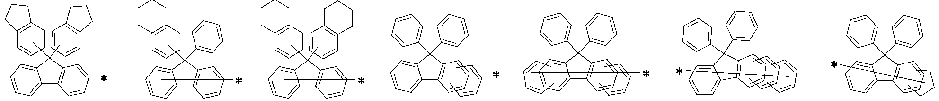

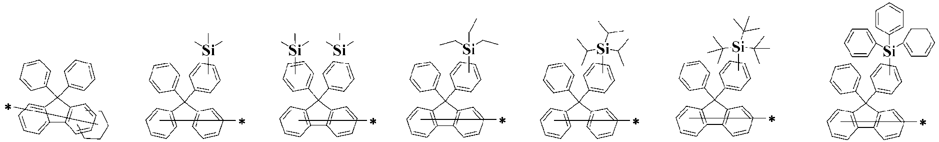

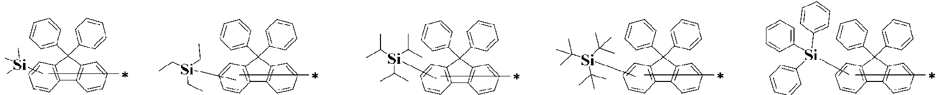

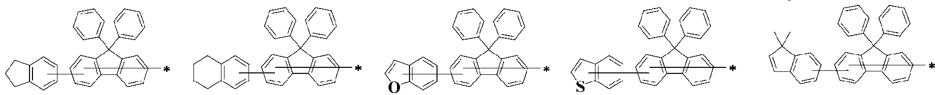

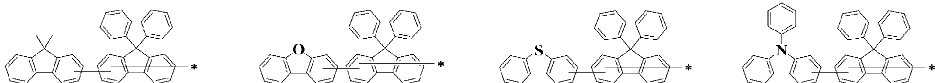

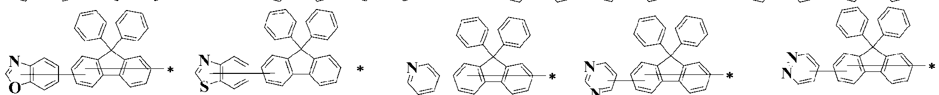

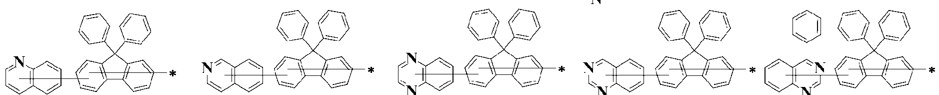

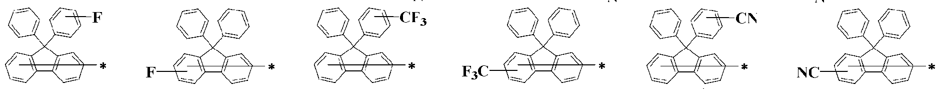

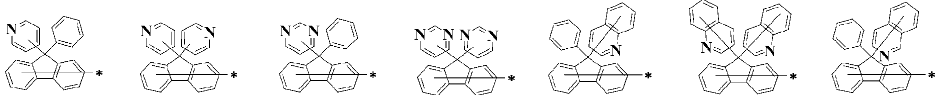

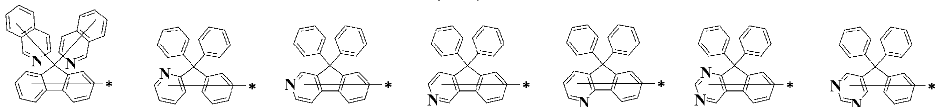

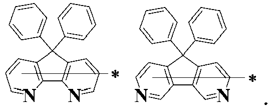

5. The triarylamine compound according to claim 1, wherein Lₐ is selected from a single bond or any one of the following groups: 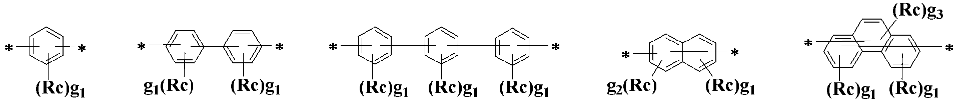 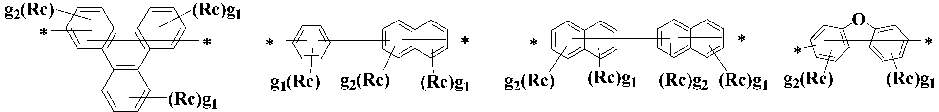 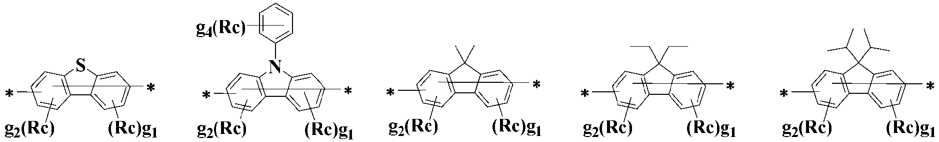 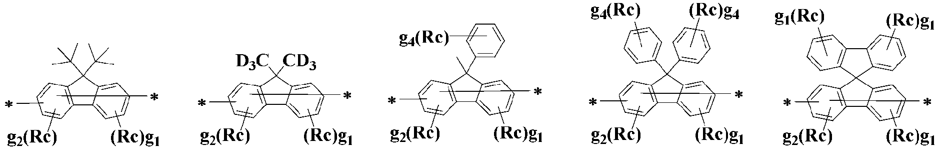 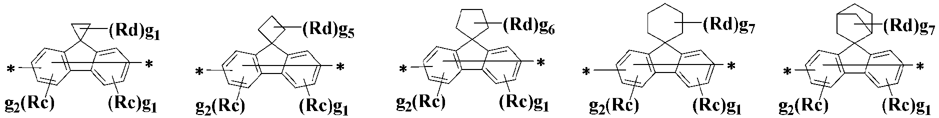 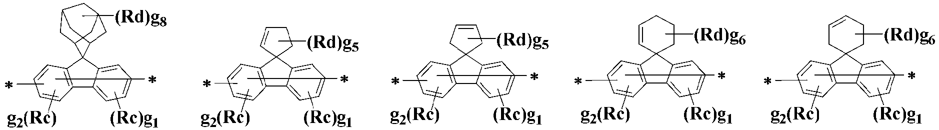 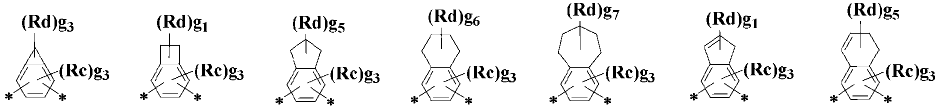 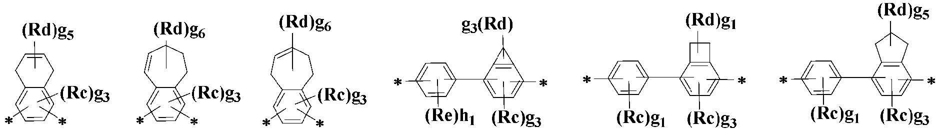 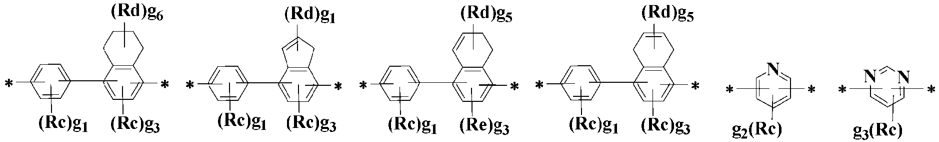 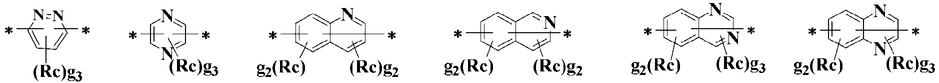 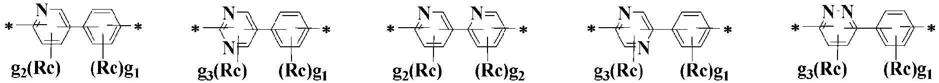 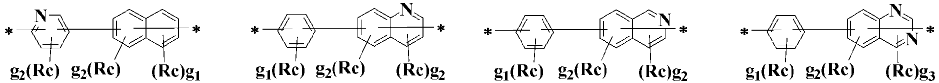 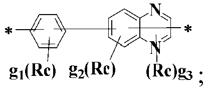
wherein R_{c} is identically or differently selected from any one of hydrogen, deuterium, tritium, cyano, halogen, substituted or unsubstituted following groups: methyl, ethyl, n-propyl, isopropyl, n-butyl, isobutyl, *tert-butyl,* cyclopropyl, cyclobutyl, cyclopentyl, cyclohexyl, adamantyl, norbornyl, phenyl, biphenyl, terphenyl, naphthyl, anthryl, phenanthryl, triphenylenyl, fluorenyl, benzofuryl, dibenzofuryl, benzothienyl, dibenzothienyl, benzoxazolyl, benzothiazolyl, benzimidazolyl, indolyl, carbazolyl, pyridyl, pyrimidinyl, pyrazinyl, pyridazinyl, quinolyl, isoquinolyl, quinazolinyl, quinoxalinyl, trimethylsilyl, triethylsilyl, tripropylsilyl, tri-tert-butylsilyl, triphenylsilyl and combinations thereof;
R_{d} is identically or differently selected from any one of hydrogen, deuterium, tritium, cyano, halogen, substituted or unsubstituted following groups: methyl, ethyl, n-propyl, isopropyl, n-butyl, isobutyl, tert-butyl, cyclopropyl, cyclobutyl, cyclopentyl, cyclohexyl, adamantyl, norbornyl, phenyl, biphenyl, terphenyl, naphthyl, anthryl, phenanthryl, triphenylenyl, fluorenyl, benzofuryl, dibenzofuryl, benzothienyl, dibenzothienyl, carbazolyl, trimethylsilyl, triethylsilyl, tripropylsilyl, tri-tert-butylsilyl, triphenylsilyl and combinations thereof;
g₁ is selected from 0, 1, 2, 3 or 4; g₂ is selected from 0, 1, 2 or 3; g₃ is selected from 0, 1 or 2; g₄ is selected from 0, 1, 2, 3, 4 or 5; g₅ is selected from 0, 1, 2, 3, 4, 5 or 6; g₆ is independently selected from 0, 1, 2, 3, 4, 5, 6, 7 or 8; g₇ is selected from 0, 1, 2, 3, 4, 5, 6, 7, 8, 9 or 10; g₈ is selected from 0, 1, 2, 3, 4, 5, 6, 7, 8, 9, 10, 11, 12, 13 or 14; when two or more R_{c} and R_{d} are present, the two or more R_{c} and R_{d} are identical to or different from each other.

6. The triarylamine compound according to claim 1, wherein L_{b} and L_{c} are independently selected from a single bond or any one of the following groups: 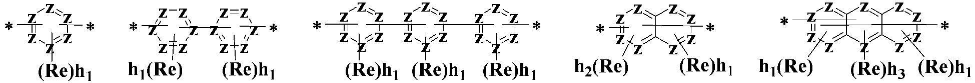 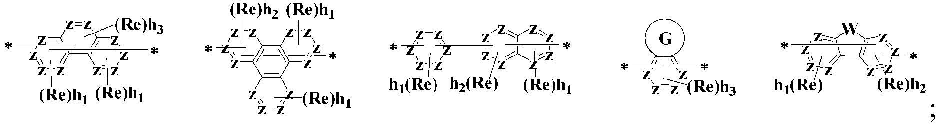
wherein z is identically or differently selected from N or CH; z at a linkage is selected from C;
Rₑ is identically or differently selected from any one of hydrogen, deuterium, tritium, cyano, halogen, nitro, substituted or unsubstituted C1 to C15 alkyl, a substituted or unsubstituted C3 to C15 alicyclic group, substituted or unsubstituted silyl, substituted or unsubstituted C6 to C30 aryl, substituted or unsubstituted fused cyclyl formed by fusing a C3 to C15 alicyclic ring to a C6 to C30 aromatic ring, substituted or unsubstituted C2 to C30 heteroaryl, substituted or unsubstituted fused cyclyl formed by fusing a C3 to C15 alicyclic ring to a C2 to C30 heteroaromatic ring, and combinations thereof; or two adjacent Rₑ can be linked to each other to form a substituted or unsubstituted ring;
the ring G is selected from a substituted or unsubstituted C3 to C10 alicyclic group;
W is selected from O, S, N(R_{g}) or C(RₕRᵢ);
R_{g} is selected from any one of substituted or unsubstituted C1 to C15 alkyl, a substituted or unsubstituted C3 to C15 alicyclic group, substituted or unsubstituted silyl, substituted or unsubstituted C6 to C30 aryl, substituted or unsubstituted C2 to C30 heteroaryl, substituted or unsubstituted fused cyclyl formed by fusing a C3 to C15 alicyclic ring to a C6 to C30 aromatic ring, substituted or unsubstituted fused cyclyl formed by fusing a C3 to C15 alicyclic ring to a C6 to C30 heteroaromatic ring, and combinations thereof;
Rₕ and Rᵢ are independently selected from any one of hydrogen, deuterium, tritium, cyano, halogen, nitro, substituted or unsubstituted C1 to C15 alkyl, a substituted or unsubstituted C3 to C15 alicyclic group, substituted or unsubstituted silyl, substituted or unsubstituted C6 to C30 aryl, substituted or unsubstituted C2 to C30 heteroaryl, substituted or unsubstituted fused cyclyl formed by fusing a C3 to C15 alicyclic ring to a C6 to C30 aromatic ring, and combinations thereof; or Rₕ and Rᵢ can be linked to each other to form a substituted or unsubstituted ring;
h₁ is selected from 0, 1, 2, 3 or 4; h₂ is selected from 0, 1, 2 or 3; h₃ is selected from 0, 1 or 2; when two or more Rₑ and R_{f} are present, the two or more Rₑ and R_{f} are identical to or different from each other.

7. The triarylamine compound according to claim 1, wherein the triarylamine compound is selected from one of the following structures:

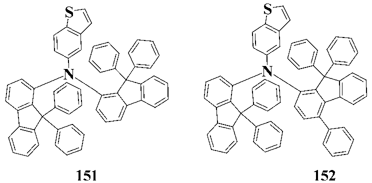

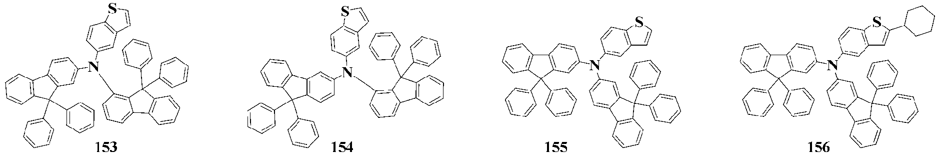

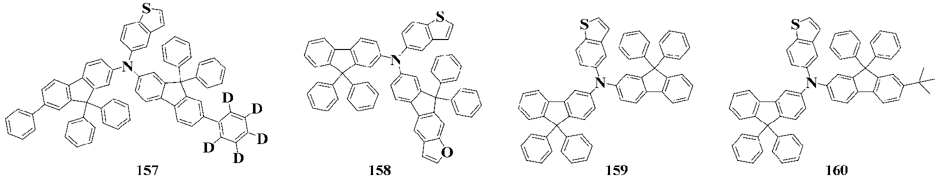

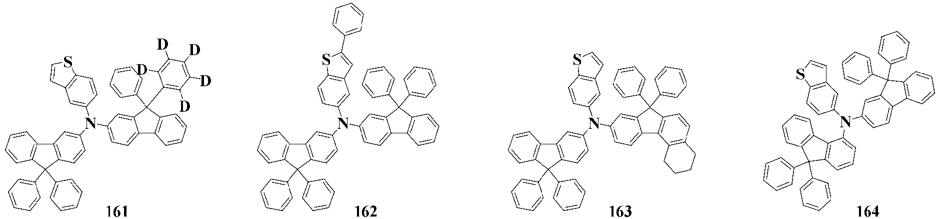

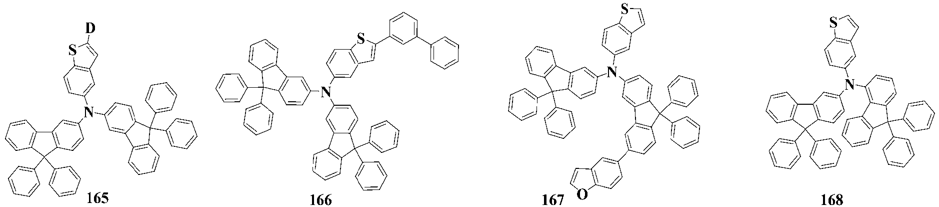

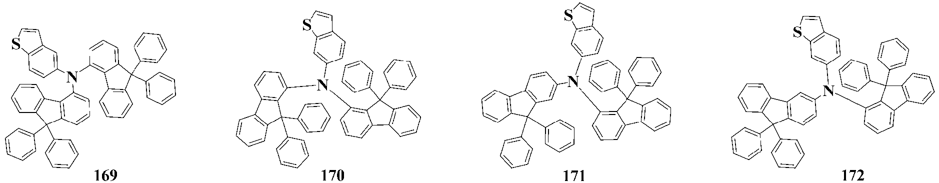

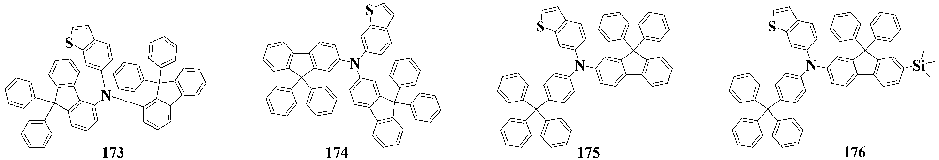

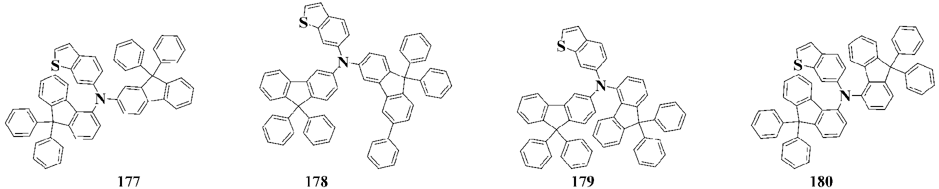

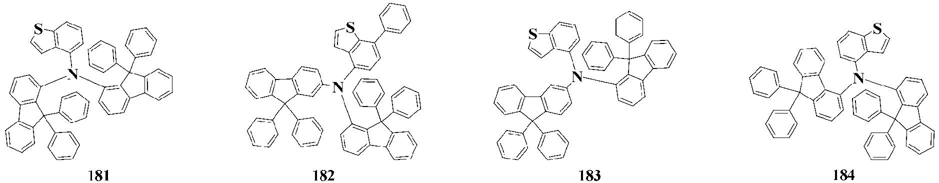

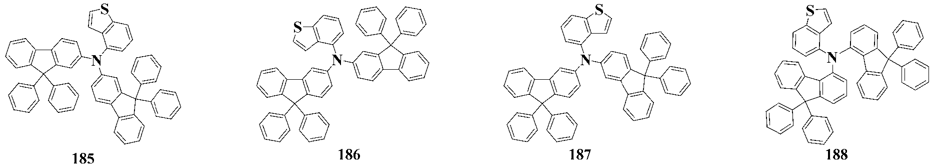

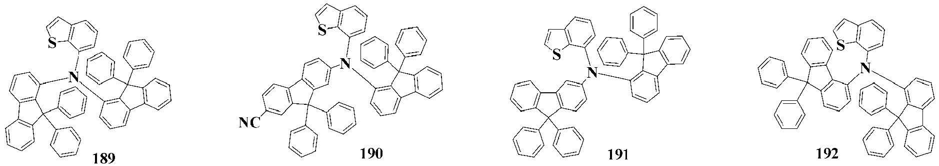

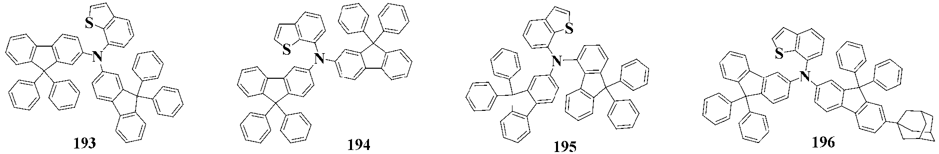

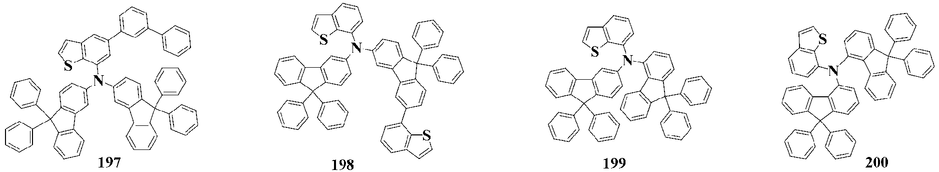

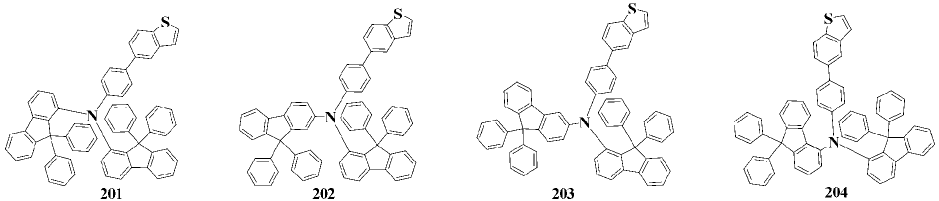

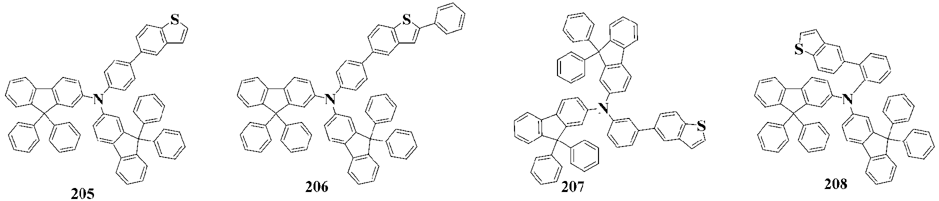

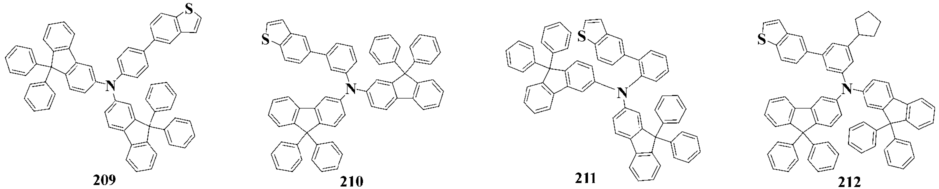

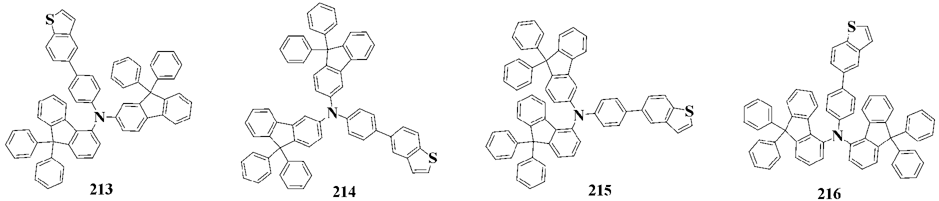

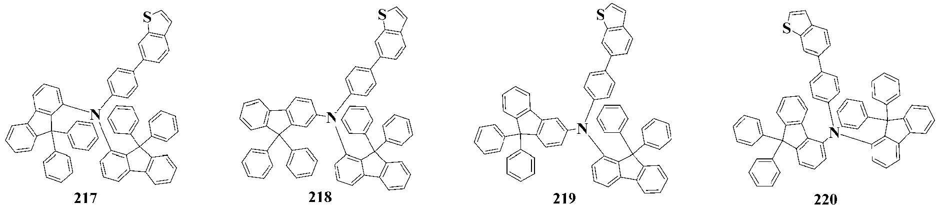

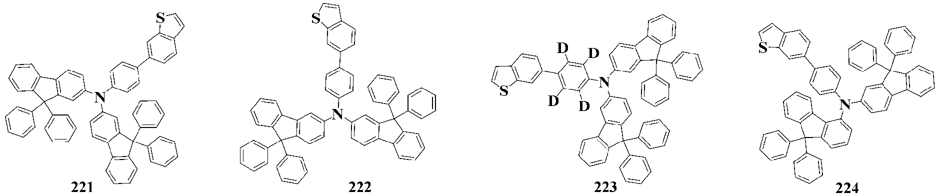

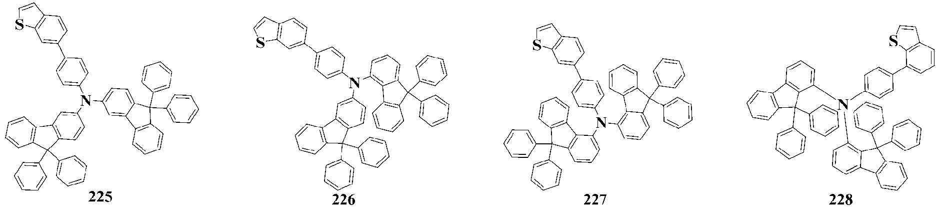

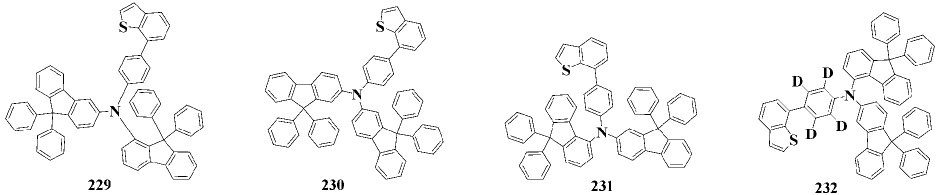

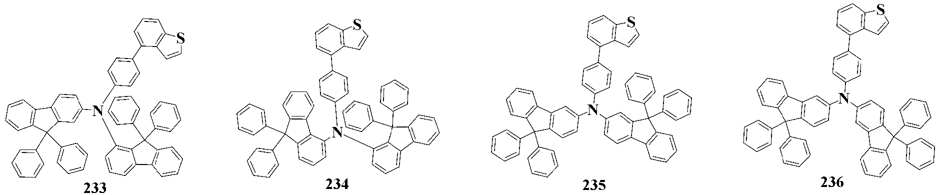

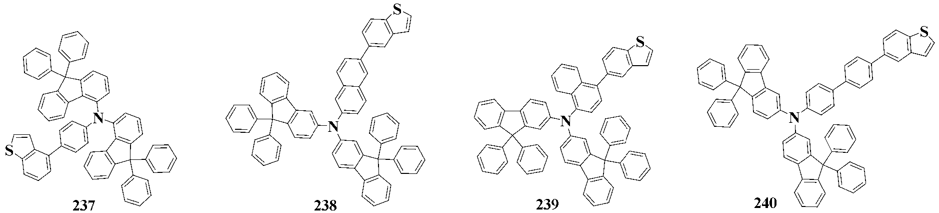

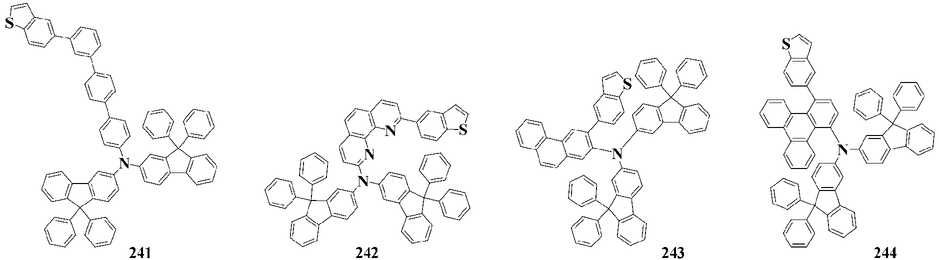

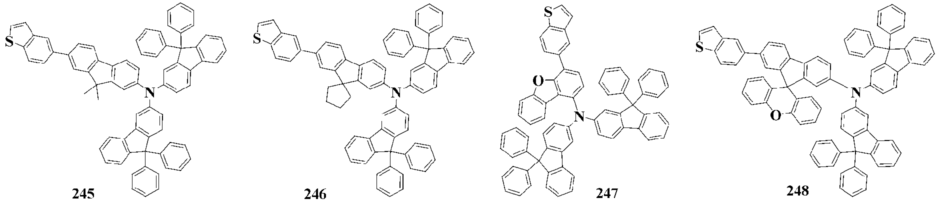

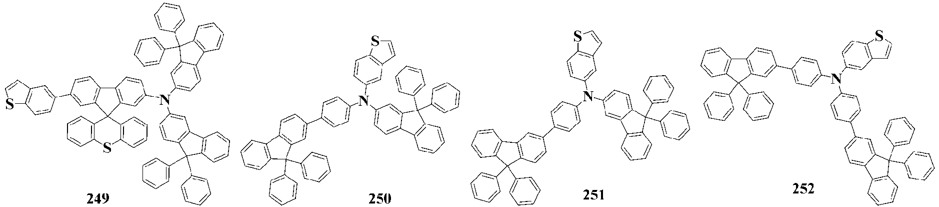

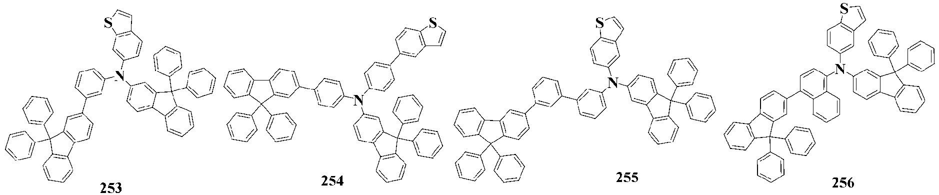

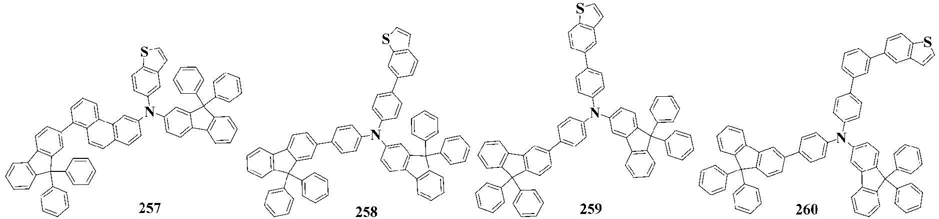

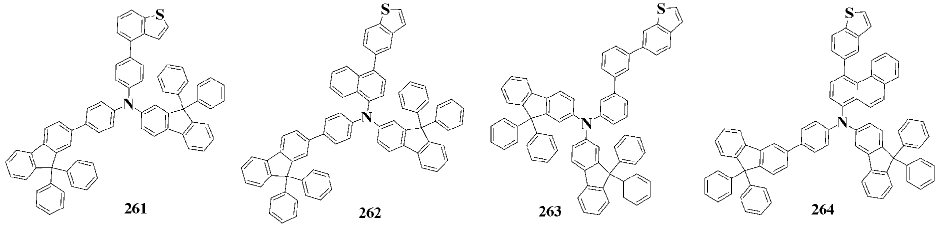

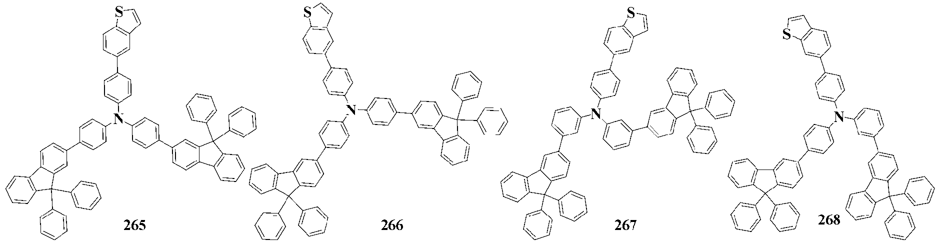

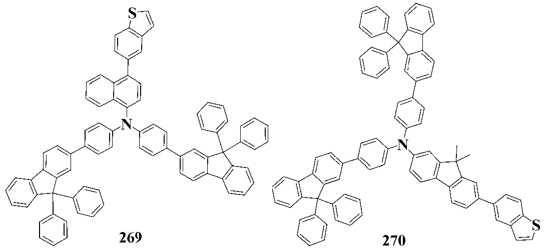

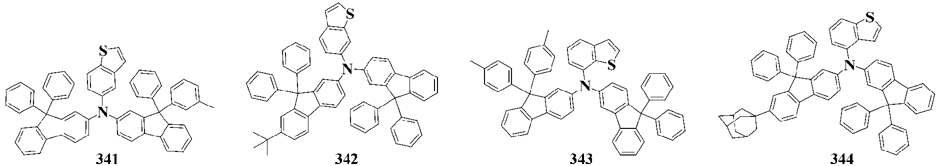

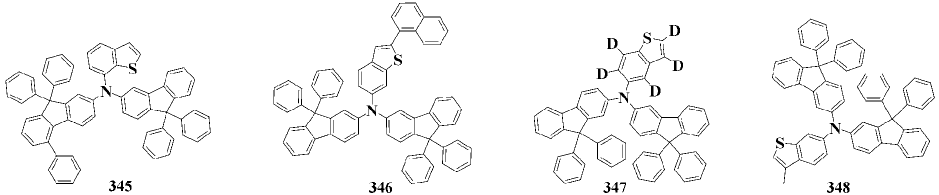

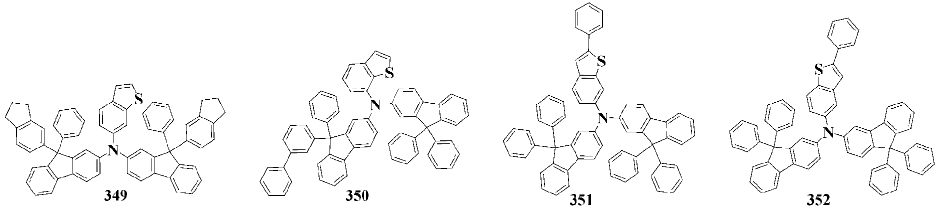

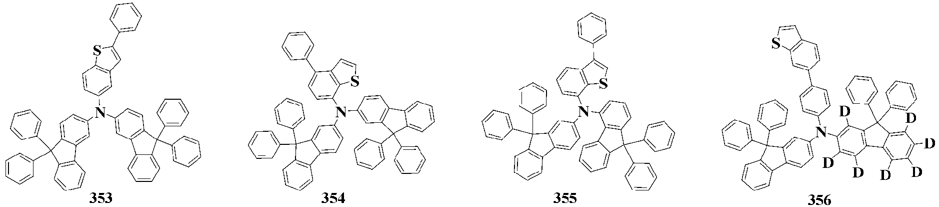

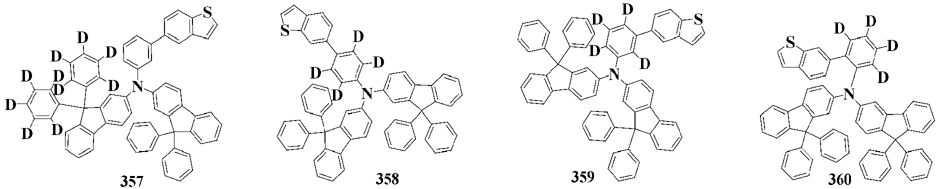

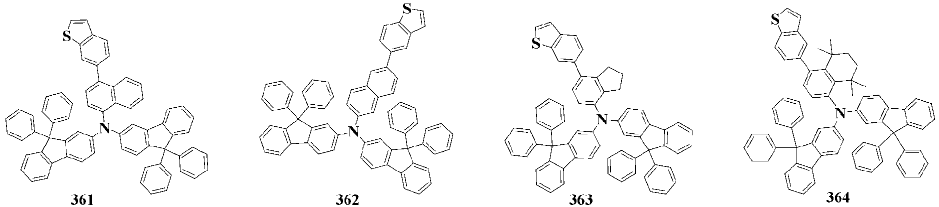

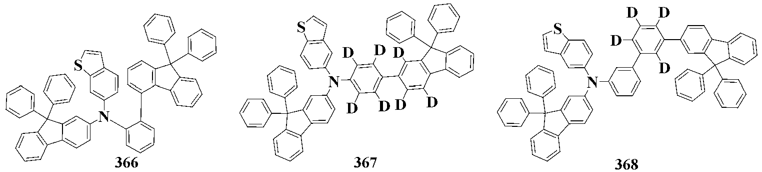

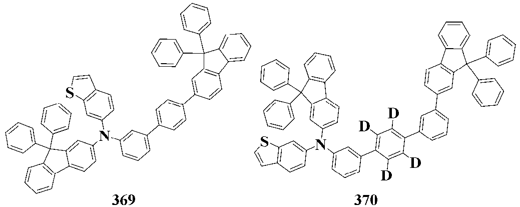

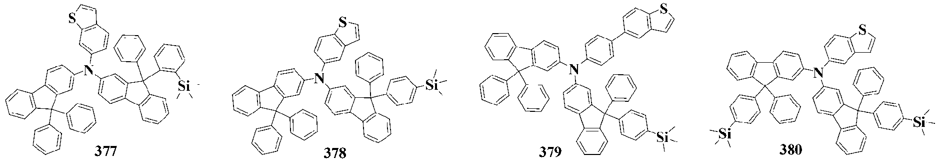

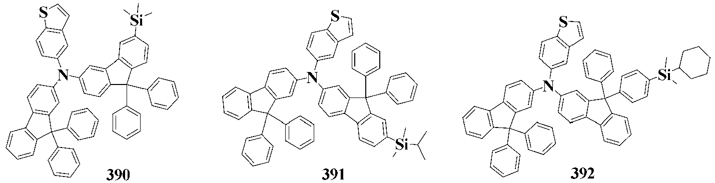

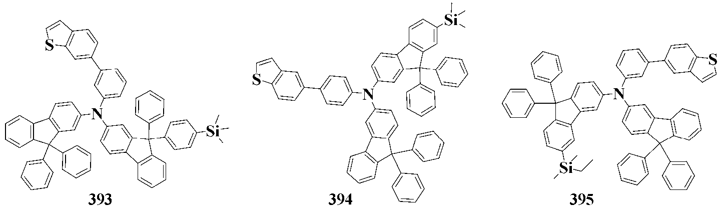

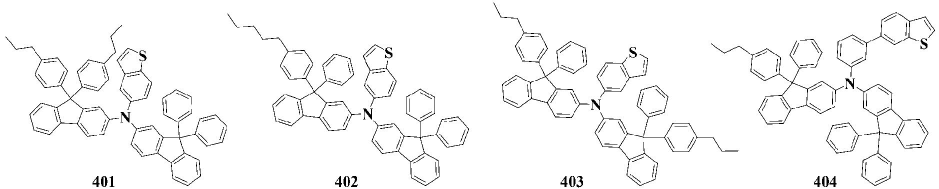

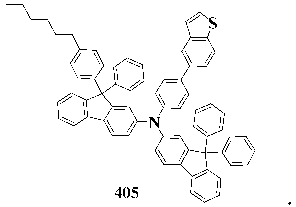

8. An organic electroluminescent device, comprising an anode, a cathode and an organic layer, wherein the organic layer is disposed between the anode and the cathode or is disposed on an outer side of at least one of the anode and the cathode, and the organic layer comprises any one or a combination of at least two of the triarylamine compound according to any one of claims 1 to 7.

9. The organic electroluminescent device according to claim 8, wherein the organic layer is disposed between the anode and the cathode, the organic layer comprises a hole transport layer, and the hole transport layer comprises any one or a combination of at least two of the triarylamine compound according to any one of claims 1 to 7.
